(19) Europäisches Patentamt
European Patent Office
Office européen des brevets

(11) Publication number: 0 168 135 B1

(12) # EUROPEAN PATENT SPECIFICATION

(45) Date of publication of patent specification: 06.12.89

(51) Int. Cl.$^4$: C 07 D 409/12, C 07 D 417/12, C 07 D 405/12, C 07 D 411/12, A 01 N 47/36

(21) Application number: 85303230.8

(22) Date of filing: 07.05.85

(54) **Herbicidal sulfonamides.**

(30) Priority: 07.05.84 US 607990
01.03.85 US 705833
21.05.84 US 612659
01.03.85 US 705832
05.06.84 US 617608
06.03.85 US 706961

(43) Date of publication of application:
15.01.86 Bulletin 86/03

(45) Publication of the grant of the patent:
06.12.89 Bulletin 89/49

(84) Designated Contracting States:
BE CH DE FR GB IT LI LU NL SE

(56) References cited:
EP-A-0 079 683
EP-A-0 099 339
EP-A-0 107 979
EP-A-0 108 708

The file contains technical information submitted after the application was filed and not included in this specification

(73) Proprietor: E.I. DU PONT DE NEMOURS AND COMPANY (a Delaware corporation)
1007 Market Street
Wilmington Delaware 19898 (US)

(72) Inventor: Pasteris, Robert James
305 Plymouth Road
Wilmington Delaware 19803 (US)
Inventor: Rorer, Morris Padgett
64 Lower Valley Lane
Newark Delaware 19711 (US)

(74) Representative: Hildyard, Edward Martin et al
Frank B. Dehn & Co. Imperial House 15-19
Kingsway
London WC2B 6UZ (GB)

(56) References cited:
CHEMICAL ABSTRACTS, vol. 99, 1983, page 647, no. 212540v, Columbus, Ohio, US;

EP 0 168 135 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to novel sulfonylurea derivatives, methods for their production, agriculturally suitable compositions containing them, and their method-of-use as pre-emergent or post-emergent herbicides or plant growth regulants.

U.S. 4,127,405 and U.S. 4,169,719 disclose herbicidal benzenesulfonylureas.

EP—A—79,683, published May 25, 1983, discloses herbicidal sulfonamides of formula

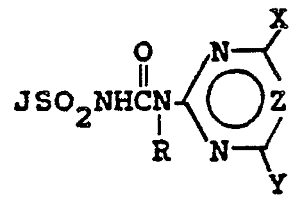

where

J is various benzofuran, benzothiophene, 1-benzopyran, 1-benzothiopyran, 1-benzoxepin and 1-benzothiepin moieties;

X is H, $CH_3$, $OCH_3$ or Cl;

Y is $CH_3$, $OCH_3$, $OCH_2CH_3$, $CH_2OCH_3$, $CH_2CH_3$, $NH_2$, $NHCH_3$ or $N(CH_3)_2$; and

Z is CH or N.

EP—A—107,979, published May 9, 1984, discloses herbicidal sulfonamides of formula

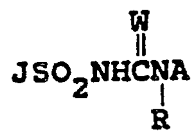

where J is, among other values,

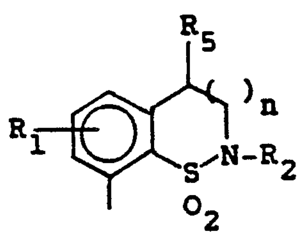
,
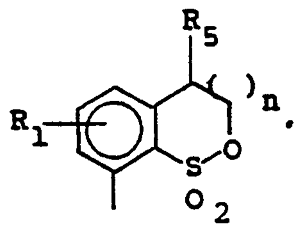
,
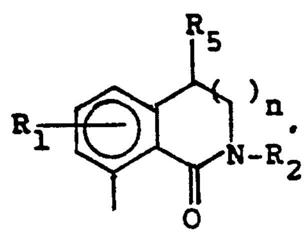
.

J1 J2 J3

EP—A—99,339 filed by Ciba-Geigy (Swiss Priority 7/16/82) discloses herbicidal sulfonylureas of the general structure shown below:

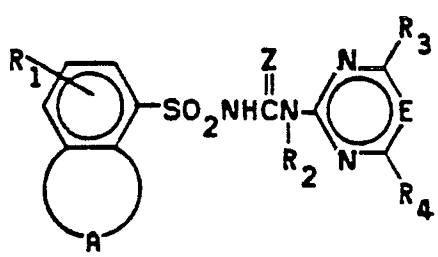

wherein

A is an unsubstituted or substituted bridge of 3 or 4 atoms which contains 1 or 2 oxygen, sulfur or nitrogen atoms and, together with the linking carbon atom, forms a non-aromatic 5- or 6-membered heterocyclic ring system, with the proviso that two oxygen atoms are separated by at least one carbon atom and that oxygen and sulfur atoms are only linked to each other if the sulfur atom takes the form of the —SO— or $SO_2$-group.

EP—A—108,708 discloses herbicidal sulfonamides of formula

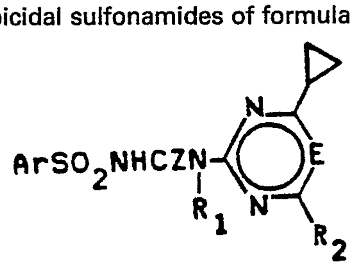

where

Ar is 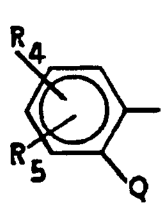 or 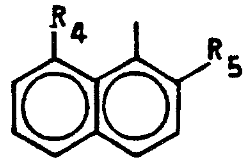 ;

and

$R_2$ is halogen, $C_1$—$C_3$ alkyl, $C_1$—$C_3$ haloalkyl, $C_1$—$C_3$ alkoxy, $C_1$—$C_3$ haloalkoxy, amino, $C_1$—$C_3$ alkylamino, di($C_1$—$C_3$ alkyl)amino, $C_3$—$C_3$ cycloalkyl or $C_2$—$C_3$ alkoxyalkyl.

This invention results from efforts to obtain novel and improved herbicides and growth regulants to help serve the ever-increasing need for food supplies in the world.

## Summary of the Invention

This invention relates to novel compounds of Formula I, agriculturally suitable compositions containing them, and their method-of-use as pre-emergent or post-emergent herbicides or plant growth regulants.

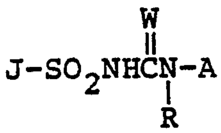

I

wherein

J is 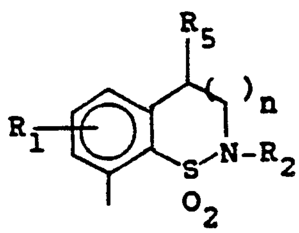 , 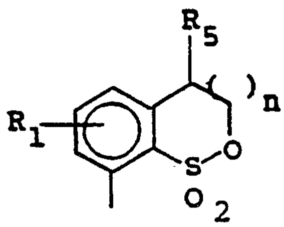 , 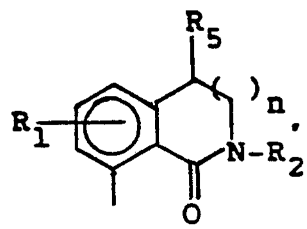 ,

J1 J2 J3

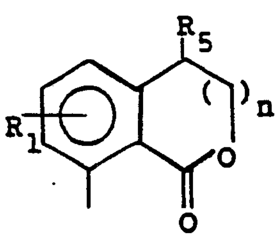 , 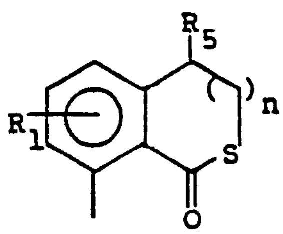 , 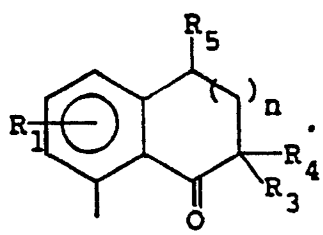 ,

J4 J5 J6

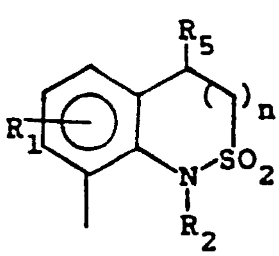 , 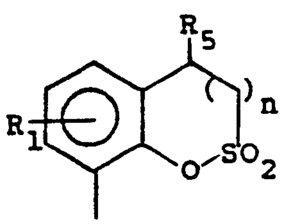 , 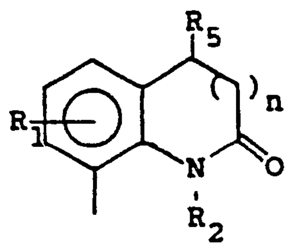 ,

J7 J8 J9

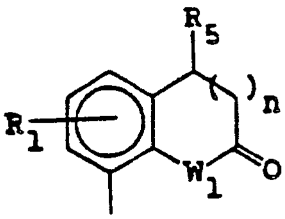

$J_{10}$ ,

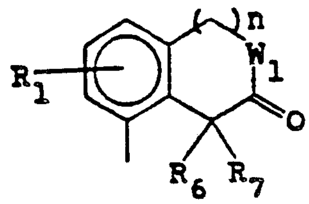

$J_{11}$ ,

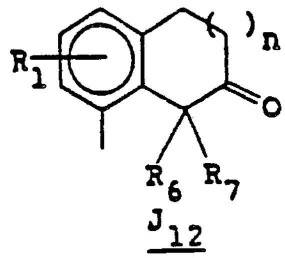

$J_{12}$ ,

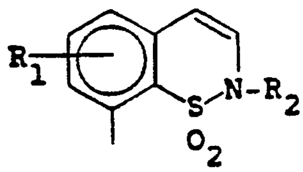

$J_{13}$ ,

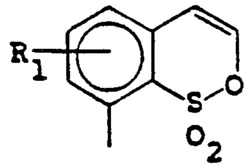

$J_{14}$ ,

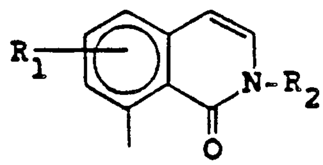

$J_{15}$ ,

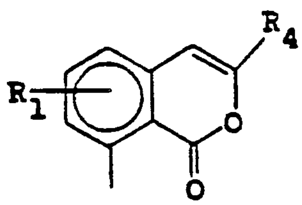

$J_{16}$ ,

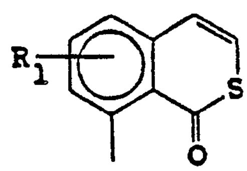

$J_{17}$ ,

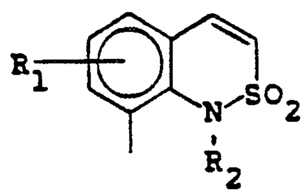

$J_{18}$ ,

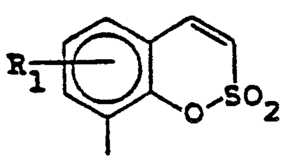

$J_{19}$ ,

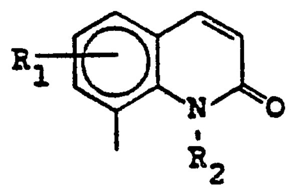

$J_{20}$ ,

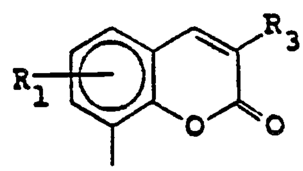

$J_{21}$ ,

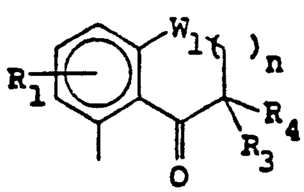

$J_{22}$ ,

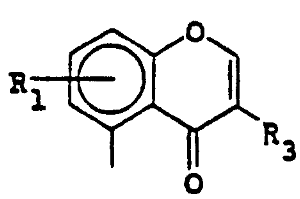

$J_{23}$ ,

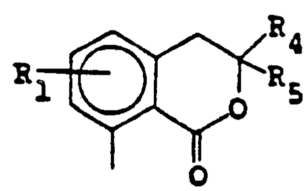

$J_{24}$ ,

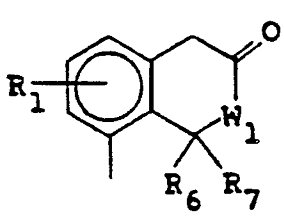

$J_{25}$

or

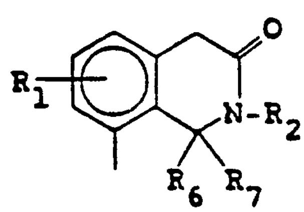

$J_{26}$ ;

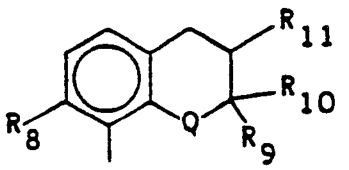

$J_{27}$ ,

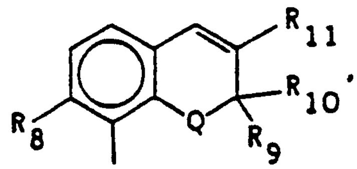

$J_{28}$ ,

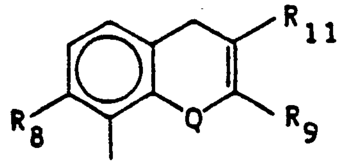

$J_{29}$ ,

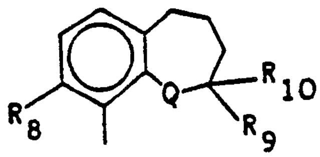

$J_{30}$ ,

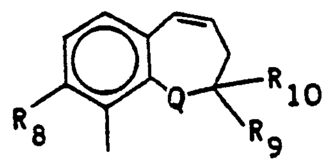

$J_{31}$ ,

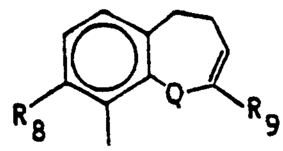

$J_{32}$ ,

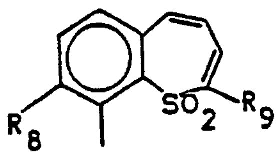

$J_{33}$ ,

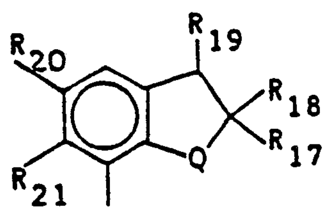

$J_{34}$ ,

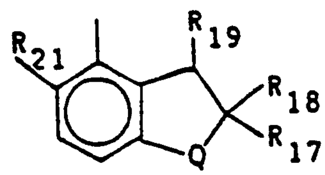

$J_{35}$ ,

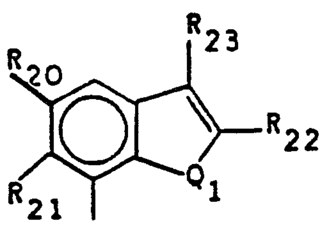

$J_{36}$ ,

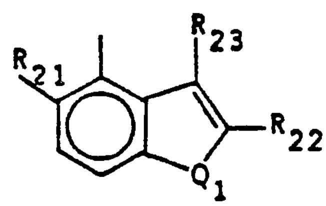

$J_{37}$ ;

n is 0, 1 or 2;
W is O or S;
$W_1$ is O or S;
Q is O, S, SO or $SO_2$;
$Q_1$ is O, S or $SO_2$;
R is H or $CH_3$;
$R_1$ is H, F, Cl, Br, $CH_3$, $OCH_3$, $CF_3$, $SCH_3$ or $OCF_2H$;
$R_2$ is H or $C_1$—$C_4$ alkyl;
$R_3$ and $R_4$ are independently H, $C_1$—$C_4$ alkyl, Cl or Br;
$R_5$ is H or $CH_3$;
$R_6$ is H or $CH_3$;
$R_7$ is H or $CH_3$;
$R_8$ is H, $CH_3$, $OCH_3$, Cl, Br, $CO_2R_{12}$, $SO_2R_{13}$, $OSO_2R_{14}$ or $SO_2NR_{15}R_{16}$;
$R_9$ and $R_{10}$ are independently H or $C_1$—$C_3$ alkyl;
$R_{11}$ is H or $CH_3$;
$R_{12}$ is $C_1$—$C_3$ alkyl, $CH_2CH{=}CH_2$, $CH_2CH_2OCH_3$ or $CH_2CH_2Cl$;
$R_{13}$ is $C_1$—$C_3$ alkyl;
$R_{14}$ is $C_1$—$C_3$ alkyl or $CF_3$;
$R_{15}$ and $R_{16}$ are independently $C_1$—$C_2$ alkyl;
$R_{17}$ is H or $C_1$—$C_4$ alkyl;
$R_{18}$ is H or $C_1$—$C_4$ alkyl;
$R_{19}$ is H or $CH_3$;

$R_{20}$ is H, Cl, $CH_3$, $CF_3$, $OCH_3$, Br, F, $SCH_3$ or $OCF_2H$;

$R_{21}$ is H, $CH_3$, $OCH_3$, Cl, Br, $NO_2$, $CO_2R_{24}$, $SO_2R_{25}$, $OSO_2R_{26}$, $SO_2NR_{27}R_{28}$, F, $CF_3$, $SCH_3$, $OCF_2H$ or $SO_2N(OCH_3)CH_3$;

$R_{22}$ is H, Cl, Br or $C_1$—$C_4$ alkyl;

$R_{22}$ is H, $CH_3$, Cl or Br;

$R_{24}$ is $C_1$—$C_3$ alkyl, $CH_2CH{=}CH_2$, $CH_2CH_2OCH_3$ or $CH_2CH_2Cl$;

$R_{25}$ is $C_1$—$C_3$ alkyl;

$R_{26}$ is $C_1$—$C_3$ alkyl or $CF_3$;

$R_{27}$ and $R_{28}$ are independently $C_1$—$C_2$ alkyl;

A is 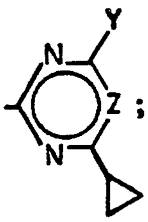 ;

Y is $C_1$—$C_2$ alkyl, $C_1$—$C_2$ alkoxy, $C_1$—$C_2$ alkoxymethyl, $OCF_2H$, $SCF_2H$, $OCH_2CH_2F$, $OCH_2CF_3$, $CF_3$, $OCH_2CH{=}CH_2$, $OCH_2C{\equiv}CH$, $NHCH_3$, $N(CH_3)_2$ or $CH(OCH_3)_2$; and

Z is CH or N;

and their agriculturally suitable salts; provided that

a) when W is S, then R is H;

b) the total number of carbon atoms in $R_3$ and $R_4$ is less than or equal to 4;

c) when $R_5$ is $CH_3$, then n is O;

d) when J is $J_{24}$, then $R_4$ and $R_5$ are not both H;

e) the total number of carbon atoms in $R_9$ and $R_{10}$ is less than or equal to 3;

f) in Formula $J_{28}$, when $R_9$ and $R_{10}$ are other than H, then $R_{11}$ is H;

g) in Formula $J_{29}$, when $R_9$ is other than H, then $R_{11}$ is H;

h) when Q is S, then $R_8$ is not $SO_2NR_{15}R_{16}$;

i) in Formulae $J_{29}$ and $J_{32}$, Q may not be O;

j) in Formula $J_{33}$, $R_8$ is not $CH_3$;

k) in Formulae $J_{36}$ and $J_{37}$, when $R_{21}$ is $NO_2$, then $R_{22}$ is $C_1$—$C_4$ alkyl and $R_{23}$ is $CH_3$;

l) in Formulae $J_{34}$ and $J_{35}$, when Q is SO, then W is O;

m) when $R_{20}$ is other than H, then $R_{21}$ is H;

n) $R_{17}$ and $R_{18}$ taken together are not more than four carbon atoms;

o) when J is $J_{27}$, $J_{28}$, $J_{29}$, $J_{30}$, $J_{3)}$ or $J_{32}$, then Q is O, S or $SO_2$; and

p) when Y is $OCF_2H$ then Z is CH.

Preferred Compounds

Preferred for reasons of their higher herbicidal activity, greater plant growth regulant activity or more favorable ease of synthesis are:

1) Compounds of Formula I where

W is O;

$R_3$ and $R_4$ are independently H or $C_1$—$C_3$ alkyl;

$R_{17}$ is H or $C_1$—$C_2$ alkyl;

$R_{18}$ is H or $CH_3$;

$R_{19}$ is H;

$R_{22}$ is H or $C_1$—$C_2$ alkyl;

$R_{23}$ is H; and

$R_1$ is bonded to the ortho or meta position of the ring relative to the sulfonylurea moiety.

2) Compounds of Preferred 1 where

R is H;

$R_1$ is H, F, Cl, Br, $CH_3$, $OCH_3$, $CF_3$ or $SCH_3$;

$R_8$ is H, $CH_3$, $OCH_3$ or Cl;

$R_{20}$ is H, Cl, $CH_3$ or $OCH_3$; and

$R_{21}$ is H, Cl, $CH_3$, $OCH_3$, $CO_2R_{24}$ or $SO_2R_{25}$.

3) Compounds of Preferred 2 where

$R_1$ is H;

$R_5$ is H;

$R_8$ is H;

$R_{20}$ is H;

$R_{21}$ is H; and

Y is $CH_3$, $OCH_3$, or $CH_2OCH_3$.

4) Compounds of Preferred 3 where J is $J_1$.
5) Compounds of Preferred 3 where J is $J_2$.
6) Compounds of Preferred 3 where J is $J_3$.
7) Compounds of Preferred 3 where J is $J_4$.
8) Compounds of Preferred 3 where J is $J_5$.
9) Compounds of Preferred 3 where J is $J_6$.
10) Compounds of Preferred 3 where J is $J_7$.
11) Compounds of Preferred 3 where J is $J_8$.
12) Compounds of Preferred 3 where J is $J_9$.
13) Compounds of Preferred 3 where J is $J_{10}$.
14) Compounds of Preferred 3 where J is $J_{11}$.
15) Compounds of Preferred 3 where J is $J_{12}$.
16) Compounds of Preferred 3 where J is $J_{13}$.
17) Compounds of Preferred 3 where J is $J_{14}$.
18) Compounds of Preferred 3 where J is $J_{15}$.
19) Compounds of Preferred 3 where J is $J_{16}$.
20) Compounds of Preferred 3 where J is $J_{17}$.
21) Compounds of Preferred 3 where J is $J_{18}$.
22) Compounds of Preferred 3 where J is $J_{19}$.
23) Compounds of Preferred 3 where J is $J_{20}$.
24) Compounds of Preferred 3 where J is $J_{21}$.
25) Compounds of Preferred 3 where J is $J_{22}$.
26) Compounds of Preferred 3 where J is $J_{23}$.
27) Compounds of Preferred 3 where J is $J_{24}$.
28) Compounds of Preferred 3 where J is $J_{25}$.
29) Compounds of Preferred 3 where J is $J_{26}$.
30) Compounds of Preferred 3 where J is $J_{27}$.
31) Compounds of Preferred 3 where J is $J_{28}$.
32) Compounds of Preferred 3 where J is $J_{29}$.
33) Compounds of Preferred 3 where J is $J_{30}$.
34) Compounds of Preferred 3 where J is $J_{31}$.
35) Compounds of Preferred 3 where J is $J_{32}$.
36) Compounds of Preferred 3 where J is $J_{33}$.
37) Compounds of Preferred 3 where J is $J_{34}$.
38) Compounds of Preferred 3 where J is $J_{35}$.
39) Compounds of Preferred 3 where J is $J_{36}$.
40) Compounds of Preferred 3 where J is $J_{37}$.

## Detailed Description of the Invention

### Synthesis

The following discussion represents a general outline for the preparation of the compounds of this invention. All of the syntheses described below are multistep with one or more methods being taught for each step. This allows for a wide variety of possible synthetic pathways to prepare a particular compound of Formula I. The proper choice of the synthetic pathway and the best ordering of the reaction sequences for each individual compound will be known to one skilled in the art.

The compounds of Formula I can be prepared by one or more of the methods described below in Equations 1 through 5. Reaction conditions are given by way of example only.

As shown in Equation 1, many of the compounds of Formula I where J is other than $J_6$, $R_{12}$, $J_{22}$ and $J_{23}$, and when Q is S or O, J is other than $J_{28}$, $J_{29}$, $J_{31}$ and $J_{32}$, can be prepared by reacting a sulfonylisocyanate (W = O) or a sulfonylisothiocyanate (W = S) of Formula II with an appropriate heterocyclic amine of Formula III. R, A and W are as previously defined.

Equation 1

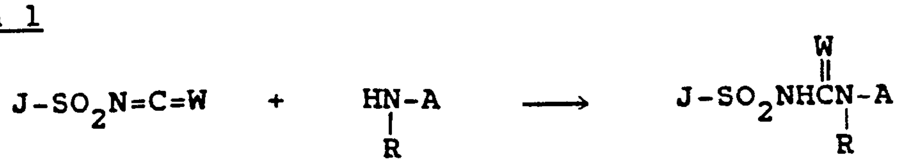

II III I

The reaction is carried out at 25° to 100°C in an inert, aprotic solvent such as methylene chloride or xylene for 0.5 to 24 hours as taught in U.S. Patent 4,127,405.

Compounds of Formula I, where W is S and R is H, (Ia) can be prepared by reacting the appropriate sulfonamide of Formula IV with a heterocyclic isothiocyanate of Formula V, as shown in Equation 2.

Equation 2

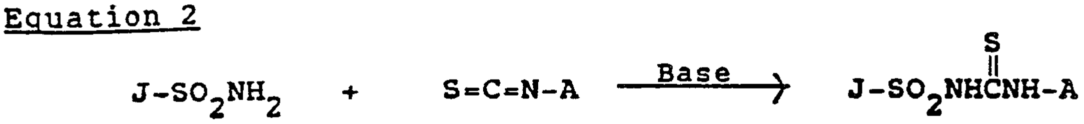


IV V Ia

The reaction is carried out at 25° to 80°C in an inert, aprotic solvent such as acetone or acetonitrile in the presence of a base such as potassium carbonate for 0.5 to 24 hours. The required heterocyclic isothiocyanates V are prepared from the corresponding amines III as taught in EP—A—35893.

Compounds of Formula I, where W is O (Ib), can be prepared by reacting a sulfonylcarbamate of Formula VI with an appropriate amine of Formula III, as shown in Equation 3.

Equation 3

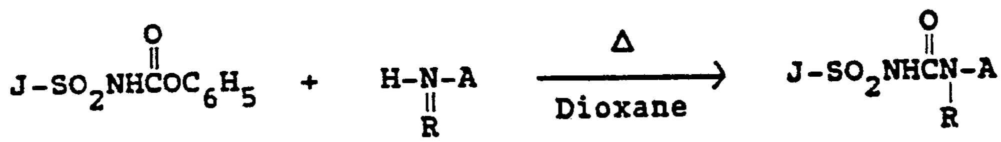


VI III Ib

The reaction is carried out at 50° to 100°C in a solvent such as dioxane for 0.5 to 24 hours as taught in EP—A—44807. The required carbamates VII are prepared by reacting the corresponding sulfonamides IV with diphenylcarbonate in the presence of a strong base.

Compounds of Formula Ib can also be prepared, as shown in Equation 4, by reacting a heterocyclic carbamite of Formula VII with an appropriate sulfonamide of Formula IV.

Equation 4

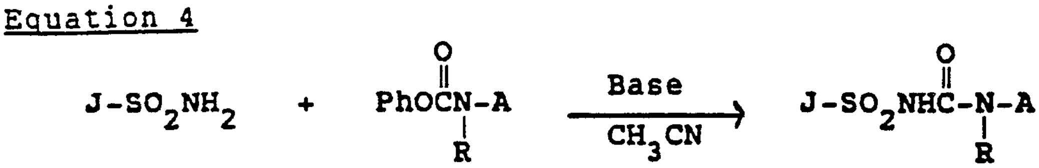


IV VII Ib

The reaction is carried out at 0° to 50°C in a solvent such as acetonitrile or dioxane in the presence of a non-nucleophilic base such as DBU for 0.2 to 24 hours as taught in South African Patent Application 830441. The required phenylcarbamate VII are prepared by reacting the corresponding heterocyclic amines III with diphenylcarbonate or phenylchloroformate in the presence of a strong base.

Compounds of Formula Ib where J is other than $J_4$, $J_5$, $J_{10}$, $J_{11}$, $J_{16}$, $J_{17}$, $J_{21}$, $J_{24}$ and $J_{25}$ and when $R_8$ is other than $CO_2R_{12}$ and $R_{21}$ is other than $CO_2R_{24}$ can be prepared by reacting the sulfonamides of Formula IV with an appropriate methylcarbamate of Formula VIII in the presence of an equimolar amount of trimethylaluminum, as shown in Equation 5.

Equation 5

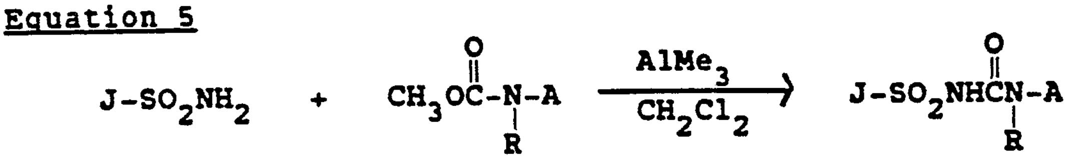


IV VIII Ib

The reaction is carried out at 25° to 40°C in a solvent such as methylene chloride for 10 to 96 hours under an inert atmosphere as taught in EP—A—84,244. The required carbamates VIII are prepared by reacting the corresponding amines III with dimethylcarbonate or methyl chloroformate in the presence of a strong base.

The intermediate sulfonylisocyanates (W=O) and sulfonylisothiocyanates (W=S) of Formula II from Equation 1 can be prepared as shown in Equations 6 through 8.

As shown in Equation 6, sulfonylisocyanates of Formula IIa where J is other than $J_6$, $J_{12}$, $J_{22}$ and $J_{23}$ can be prepared by the reaction of sulfonamides of Formula IV with phosgene, in the presence of *n*-butylisocyanate and a tertiary amine catalyst, at reflux in a solvent such as xylene by the method of U.S. Patent 4,238,621.

Equation 6

$$J\text{-}SO_2NH_2 \xrightarrow[\text{DABCO/XYLENE}/\Delta]{COCl_2/n\text{-BuNCO}} J\text{-}SO_2N{=}C{=}O$$

IV IIa

The sulfonylisocyanates can also be prepared from the sulfonamides by a two step procedure involving (a) reacting the sulfonamides with *n*-butylisocyanate in the presence of a base such as $K_2CO_3$ at reflux in an inert solvent such as 2-butanone forming a *n*-butylsulfonylurea; and (b) reacting this compound with phosgene and a tertiary amine catalyst at reflux in xylene solvent. The method is similar to a procedure taught by Ulricht and Sayigh. *Newer Methods of Preparative Organic Chemistry,* Vol. VI, P. 223—241, Academic Press, New York and London, W. Foerst Ed.

Alternatively, as shown in Equation 7, many of the sulfonylisocyanates of Formula IIa can be prepared by reacting the corresponding sulfonyl chlorides IX with cyanic acid salts.

Equation 7

$$J\text{-}SO_2Cl \xrightarrow{M^{\oplus}OCN^{\ominus}} J\text{-}SO_2N{=}C{=}O$$

IX IIa

The reaction is carried out at 25° to 100° in an inert aprotic solvent such as acetonitrile for 0.5—24 hours in the presence of phosphorus pentoxide and an alkali metal salt such as lithium iodide according to the teachings of Japanese Patent No. 76/26,816 (*Chem. Abst.*, 85:77892e (1976)).

The sulfonylisothiocyanates of Formula IIB where J is other than $J_6$, $J_{12}$, $J_{22}$ and $J_{23}$ can be prepared, as shown in Equation 8, by contacting the sulfonamides of Formula IV with carbon disulfide in the presence of two equivalents of a strong base. The resulting salt is then reacted with phosgene according to the teachings of K. Hartke, *Arch. Pharm., 299*, 174 (1966).

Equation 8

$$J\text{-}SO_2NH_2 \xrightarrow[\text{2) } COCl_2]{\text{1) } CS_2/\text{BASE}} J\text{-}SO_2N{=}C{=}S$$

IV IIb

The sulfonamides of Formula IV with J values $J_1$—$J_{26}$ can be prepared as taught in EP—A—107,979. The sulfonamides of Formula IV with J values $J_{27}$—$J_{37}$ can be prepared as taught in EP—A—79,683.

The preparation of compounds of Formula I where J is $J_1$ to $J_{12}$ and $J_{22}$ and n=2 can be prepared using the procedures outlined above for the analogous compounds where n=1.

The amines of Formula III in Equations 1 and 3 are also important intermediates for the preparation of the compounds of this invention. The amines can be prepared by the methods taught in South African Patent Application 837,434 or by suitable modification thereof as known to one skilled in the art.

2-Aminopyrimidines and 2-aminotriazines are well known in the art. For a review of the synthesis and reactions of 2-aminopyrimidines see *The Chemistry of Heterocyclic Compounds*, Vol. 16, John Wiley and Sons, New York (1962). For a review of the synthesis and reactions of 2-amino-s-triazines see *The Chemistry of Heterocyclic Compounds*, Vol. 13, John Wiley, New York (1959). F. C. Schaefer, U.S. Patent 3,154,547 and F. C. Schaefer and K. R. Huffman, *J. Org. Chem.*, 28, 1812 (1963).

Agriculturally suitable salts of compounds of Formula I are also useful herbicides and can be prepared in a number of ways known to the art. For example, metal salts can be made by contacting compounds of Formula I with a solution of an alkali or alkaline earth metal salt having a sufficiently basic anion (e.g., hydroxide, alkoxide, carbonate or hydride). Quaternary amine salts can be made by similar techniques.

Salts of compounds of Formula I can also be prepared by exchange of one cation for another. Cationic exchange can be effected by direct contacting of an aqueous solution of a salt of a compound of Formula I (e.g., alkali metal or quaternary amine salt) with a solution containing the cation to be exchanged. This method is most effective when the desired salt containing the exchanged cation is insoluble in water, e.g., a copper salt, and can be separated by filtration.

Exchange may also be effected by passing an aqueous solution of a salt of a compound of Formula I (e.g., an alkali metal or quaternary amine salt) through a column packed with a cation exchange resin containing the cation to be exchanged. In this method, the cation of the resin is exchanged for that of the

original salt and the desired product is eluted from the column. This method is particularly useful when the desired salt is water-soluble, e.g. a potassium, sodium or calcium salt.

Acid addition salts, useful in this invention, can be obtained by reacting a compound of Formula I with a suitable acid, e.g., *p*-toluenesulfonic acid, trichloroacetic acid or the like.

Example 1

N-[(4-Cyclopropyl-6-methoxy-1,3,5-triazin-2-yl)aminocarbonyl]-2,3-dihydro-2-methylbenzothiophene-7-sulfonamide, 1,1-dioxide

To a mixture of 0.3 g of 2,3-dihydro-2-methyl benzothiophene-7-sulfonamide, 1,1-dioxide and 0.33 g of 2-amino-4-cyclopropyl-6-methoxytriazine phenylcarbamate in 20 mL of dry acetonitrile was added 0.18 mL of DBU. After stirring for 2 hours, the resulting solution was poured into 25 mL of water and acidified to pH 2 with 1N HCl. The water was decanted from the organic layer, methylene chloride was added and the solution was dried over magnesium sulfate. This solution was filtered. The volatiles were removed by rotary evaporation and the residue was triturated with 1-chlorobutane to give 0.2 g of a white solid, m.p. 205—210°C (d).

$^1H$ NMR ($CDCl_3$) δ:

1.2—1.8 (m, 4H, cyclopropyl)
1.55 (d, 3H, $CH_3$)
2.2 (m, 1H, cyclopropyl)
3.2 (m, 1H, CH)
3.6 (m, 2H, $CH_2$)
4.1 (s, 3H, $OCH_3$)
7.5—7.9 (m, 3H, arom and NH)
8.4 (m, 1H, arom)
12.7 (broad, 1H, NH)

IR (Nujol) 1710 $cm^{-1}$ (C=O)

Using the procedures outlined herein, compounds of Tables I—VI may be prepared.

Table I

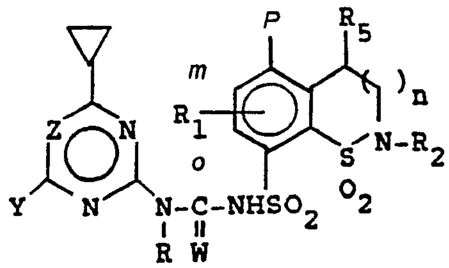

| W | n | R | $R_1$ | $R_2$ | $R_5$ | Y | Z | m.p.(°C) |
|---|---|---|---|---|---|---|---|---|
| O | 0 | H | H | $CH_3$ | H | $CH_3$ | CH | |
| O | 0 | H | H | $CH_3$ | H | $CH_2CH_3$ | CH | |
| O | 0 | H | H | $CH_3$ | H | $OCH_3$ | CH | |
| O | 0 | H | H | $CH_3$ | H | $OCH_2CH_3$ | CH | |
| O | 0 | H | H | $CH_3$ | H | $CH_2OCH_3$ | CH | |
| O | 0 | H | H | $CH_3$ | H | $OCF_2H$ | CH | |
| O | 0 | H | H | $CH_3$ | H | $SCF_2H$ | CH | |
| O | 0 | H | H | $CH_3$ | H | $OCH_2CF_3$ | CH | |
| O | 0 | H | H | $CH_3$ | H | $CF_3$ | CH | |
| O | 0 | H | H | $CH_3$ | H | $OCH_2CH{=}CH_2$ | CH | |
| O | 0 | H | H | $CH_3$ | H | $OCH_2C{\equiv}CH$ | CH | |
| O | 0 | H | H | $CH_3$ | H | $NHCH_3$ | CH | |
| O | 0 | H | H | $CH_3$ | H | $N(CH_3)_2$ | CH | |
| O | 0 | H | H | $CH_3$ | H | $CH(OCH_3)_2$ | CH | |
| O | 0 | H | H | $CH_3$ | H | $CH_3$ | N | |
| O | 0 | H | H | $CH_3$ | H | $CH_2CH_3$ | N | |
| O | 0 | H | H | $CH_3$ | H | $OCH_3$ | N | |
| O | 0 | H | H | $CH_3$ | H | $OCH_2CH_3$ | N | |
| O | 0 | H | H | $CH_3$ | H | $CH_2OCH_3$ | N | |
| O | 0 | H | H | $CH_3$ | H | $OCF_2H$ | N | |
| O | 0 | H | H | $CH_3$ | H | $SCF_2H$ | N | |
| O | 0 | H | H | $CH_3$ | H | $OCH_2CF_3$ | N | |
| O | 0 | H | H | $CH_3$ | H | $CF_3$ | N | |
| O | 0 | H | H | $CH_3$ | H | $OCH_2CH{=}CH_2$ | N | |

Table I (continued)

| W | n | R | $R_1$ | $R_2$ | $R_5$ | Y | Z | m.p.(°C) |
|---|---|---|---|---|---|---|---|---|
| O | 0 | H | H | $CH_3$ | H | $OCH_2C{\equiv}CH$ | N | |
| O | 0 | H | H | $CH_3$ | H | $NHCH_3$ | N | |
| O | 0 | H | H | $CH_3$ | H | $N(CH_3)_2$ | N | |
| O | 0 | H | H | $CH_3$ | H | $CH(OCH_3)_2$ | N | |
| O | 0 | H | H | $CH_2CH_3$ | H | $OCH_3$ | N | 152-166 |
| O | 0 | H | H | $CH_2CH_2CH_3$ | H | $OCH_3$ | N | |
| O | 0 | H | H | $CH(CH_3)_2$ | H | $OCH_3$ | N | |
| O | 0 | H | H | $CH_2CH_2CH_2CH_3$ | H | $OCH_3$ | N | |
| O | 0 | H | H | $CH_2CH(CH_3)_2$ | H | $OCH_3$ | N | 138-144 |
| O | 0 | H | H | $CH(CH_3)CH_2CH_3$ | H | $OCH_3$ | N | |
| O | 0 | H | H | $C(CH_3)_3$ | H | $OCH_3$ | N | |
| O | 0 | H | H | H | H | $OCH_3$ | N | |
| O | 0 | H | H | $CH_3$ | $CH_3$ | $OCH_3$ | N | |
| O | 0 | $CH_3$ | H | $CH_3$ | H | $OCH_3$ | N | |
| S | 0 | H | H | $CH_3$ | H | $OCH_3$ | N | |
| O | 1 | H | H | $CH_3$ | H | $OCH_3$ | N | |
| O | 1 | H | H | $CH_2CH_3$ | H | $OCH_3$ | N | |
| O | 1 | H | H | $CH_2CH_2CH_3$ | H | $OCH_3$ | N | |
| O | 1 | H | H | $CH(CH_3)_2$ | H | $OCH_3$ | N | |
| O | 1 | H | H | $CH_2CH_2CH_2CH_3$ | H | $OCH_3$ | N | |
| O | 1 | H | H | $CH_2CH(CH_3)_2$ | H | $OCH_3$ | N | |
| O | 1 | H | H | $CH(CH_3)CH_2CH_3$ | H | $OCH_3$ | N | |
| O | 1 | H | H | $C(CH_3)_3$ | H | $OCH_3$ | N | |
| O | 1 | H | H | H | H | $OCH_3$ | N | |
| O | 0 | H | *o*-F | $CH_3$ | H | $OCH_3$ | N | |
| O | 0 | H | *m*-F | $CH_3$ | H | $OCH_3$ | N | |
| O | 0 | H | *o*-Cl | $CH_3$ | H | $OCH_3$ | N | |
| O | 0 | H | *m*-Cl | $CH_3$ | H | $OCH_3$ | N | 163-167(d) |
| O | 0 | H | *o*-Br | $CH_3$ | H | $OCH_3$ | N | |
| O | 0 | H | *m*-Br | $CH_3$ | H | $OCH_3$ | N | |
| O | 0 | H | *o*-$CH_3$ | $CH_3$ | H | $OCH_3$ | N | |
| O | 0 | H | *m*-$CH_3$ | $CH_3$ | H | $OCH_3$ | N | |

Table I (continued)

| W | n | R | $R_1$ | $R_2$ | $R_5$ | Y | Z | m.p. (°C) |
|---|---|---|---|---|---|---|---|---|
| O | 0 | H | *o*-$OCH_3$ | $CH_3$ | H | $OCH_3$ | N | |
| O | 0 | H | *m*-$OCH_3$ | $CH_3$ | H | $OCH_3$ | N | |
| O | 0 | H | *o*-$CF_3$ | $CH_3$ | H | $OCH_3$ | N | |
| O | 0 | H | *m*-$CF_3$ | $CH_3$ | H | $OCH_3$ | N | |
| O | 0 | H | *o*-$SCH_3$ | $CH_3$ | H | $OCH_3$ | N | |
| O | 0 | H | *m*-$SCH_3$ | $CH_3$ | H | $OCH_3$ | N | |
| O | 0 | H | *o*-$OCF_2H$ | $CH_3$ | H | $OCH_3$ | N | |
| O | 0 | H | *m*-$OCF_2H$ | $CH_3$ | H | $OCH_3$ | N | |
| O | 0 | H | *p*-F | $CH_3$ | H | $OCH_3$ | N | |
| O | 0 | H | *p*-Cl | $CH_3$ | H | $OCH_3$ | N | |
| O | 2 | H | H | $CH_3$ | H | $OCH_3$ | N | |

Table II

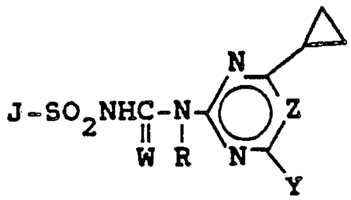

| J | W | n | $W_1$ | R | $R_1$ | $R_2$ | $R_5$ | $R_6$ | $R_7$ | Y | Z | m.p. (°C) |
|---|---|---|---|---|---|---|---|---|---|---|---|---|
| J-2 | O | 0 | - | H | H | - | H | - | - | $OCH_3$ | N | |
| J-2 | O | 1 | - | H | H | - | H | - | - | $OCH_3$ | N | |
| J-3 | O | 0 | - | H | H | $CH_3$ | H | - | - | $OCH_3$ | N | |
| J-3 | O | 1 | - | H | H | $CH_3$ | H | - | - | $OCH_3$ | N | |
| J-4 | O | 0 | - | H | H | - | H | - | - | $OCH_3$ | N | 159-164 |
| J-4 | O | 0 | - | H | *o*-Cl | - | H | - | - | $OCH_3$ | N | |
| J-4 | O | 0 | - | H | *m*-Cl | - | H | - | - | $OCH_3$ | N | |
| J-4 | O | 0 | - | H | *p*-Cl | - | H | - | - | $OCH_3$ | N | |
| J-4 | O | 0 | - | H | *m*-$CH_3$ | - | H | - | - | $OCH_3$ | N | |
| J-4 | O | 0 | - | H | *o*-$OCH_3$ | - | H | - | - | $OCH_3$ | N | |
| J-4 | O | 1 | - | H | H | - | H | - | - | $OCH_3$ | N | |
| J-4 | O | 1 | - | H | *o*-Cl | - | H | - | - | $OCH_3$ | N | |
| J-4 | O | 1 | - | H | *m*-Cl | - | H | - | - | $OCH_3$ | N | |
| J-4 | O | 1 | - | H | *p*-Cl | - | H | - | - | $OCH_3$ | N | |
| J-4 | O | 1 | - | H | *m*-$CH_3$ | - | H | - | - | $OCH_3$ | N | |
| J-4 | O | 1 | - | H | *o*-$OCH_3$ | - | H | - | - | $OCH_3$ | N | |
| J-5 | O | 0 | - | H | H | - | H | - | - | $OCH_3$ | N | |
| J-5 | O | 1 | - | H | H | - | H | - | - | $OCH_3$ | N | |
| J-7 | O | 0 | - | H | H | $CH_3$ | H | - | - | $OCH_3$ | N | |
| J-7 | O | 1 | - | H | H | $CH_3$ | H | - | - | $OCH_3$ | N | |
| J-8 | O | 0 | - | H | H | - | H | - | - | $OCH_3$ | N | |
| J-8 | O | 1 | - | H | H | - | H | - | - | $OCH_3$ | N | |
| J-9 | O | 0 | - | H | H | $CH_3$ | H | - | - | $OCH_3$ | N | |
| J-9 | O | 1 | - | H | H | $CH_3$ | H | - | - | $OCH_3$ | N | |
| J-8 | O | 1 | - | H | *m*-Br | - | H | - | - | $OCH_3$ | N | 174-179 |

Table II (continued)

| J | W | n | W1 | R | R1 | R2 | R5 | R6 | R7 | Y | Z | m.p. (°C) |
|---|---|---|---|---|---|---|---|---|---|---|---|---|
| J-10 | O | 0 | O | H | H | - | H | - | - | $OCH_3$ | N | |
| J-10 | O | 0 | S | H | H | - | H | - | - | $OCH_3$ | N | |
| J-10 | O | 1 | O | H | H | - | H | - | - | $OCH_3$ | N | |
| J-10 | O | 1 | S | H | H | - | H | - | - | $OCH_3$ | N | |
| J-11 | O | 0 | O | H | H | - | H | - | - | $OCH_3$ | N | |
| J-11 | O | 0 | O | H | H | - | H | $CH_3$ | - | $OCH_3$ | N | |
| J-11 | O | 0 | O | H | H | - | H | $CH_3$ | $CH_3$ | $OCH_3$ | N | |
| J-11 | O | 0 | S | H | H | - | H | H | H | $OCH_3$ | N | |
| J-11 | O | 1 | O | H | H | - | H | H | H | $OCH_3$ | N | |
| J-11 | O | 1 | S | H | H | - | H | H | H | $OCH_3$ | N | |
| J-12 | O | 0 | O | H | H | - | H | H | H | $OCH_3$ | N | |
| J-12 | O | 1 | O | H | H | - | H | H | H | $OCH_3$ | N | |
| J-13 | O | - | - | H | H | $CH_3$ | - | - | - | $OCH_3$ | N | |
| J-14 | O | - | - | H | H | - | - | - | - | $OCH_3$ | N | |
| J-15 | O | - | - | H | H | $CH_3$ | - | - | - | $OCH_3$ | N | |
| J-17 | O | - | - | H | H | - | - | - | - | $OCH_3$ | N | |
| J-18 | O | - | - | H | H | $CH_3$ | - | - | - | $OCH_3$ | N | |
| J-19 | O | - | - | H | H | - | - | - | - | $OCH_3$ | N | |
| J-20 | O | - | - | H | H | $CH_3$ | - | - | - | $OCH_3$ | N | |
| J-25 | O | - | O | H | H | - | - | H | H | $OCH_3$ | N | |
| J-25 | O | - | S | H | H | - | - | H | H | $OCH_3$ | N | |
| J-26 | O | - | O | H | H | $CH_3$ | - | H | H | $OCH_3$ | N | |
| J-26 | O | - | S | H | H | $CH_3$ | - | H | H | $OCH_3$ | N | |
| J-4 | O | 0 | - | H | H | - | H | - | - | $OCH_3$ | CH | |
| J-4 | O | 0 | - | H | H | - | H | - | - | $CH_3$ | CH | |
| J-4 | O | 0 | - | H | H | - | H | - | - | $OCH_2CH_3$ | N | |
| J-4 | O | 1 | - | H | H | - | H | - | - | $OCH_3$ | CH | |
| J-4 | O | 1 | - | H | H | - | H | - | - | $CH_3$ | CH | |
| J-4 | O | 1 | - | H | H | - | H | - | - | $OCH_2CH_3$ | N | |

Table III

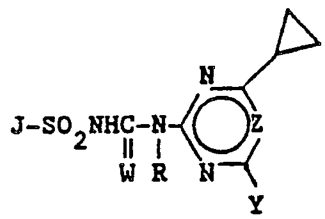

| J | W | n | $W_1$ | R | $R_1$ | $R_3$ | $R_4$ | $R_5$ | Y | Z | m.p. (°C) |
|---|---|---|---|---|---|---|---|---|---|---|---|
| J-6 | O | 0 | – | H | H | H | H | H | $OCH_3$ | N | |
| J-6 | O | 0 | – | H | H | $CH_3$ | H | H | $OCH_3$ | N | |
| J-6 | O | 0 | – | H | H | $CH_2CH_3$ | H | H | $OCH_3$ | N | |
| J-6 | O | 0 | – | H | H | $CH_2CH_2CH_3$ | H | H | $OCH_3$ | N | |
| J-6 | O | 0 | – | H | H | $CH(CH_3)_2$ | H | H | $OCH_3$ | N | |
| J-6 | O | 0 | – | H | H | $CH_2CH_2CH_2CH_3$ | H | H | $OCH_3$ | N | |
| J-6 | O | 0 | – | H | H | $CH_2CH(CH_3)_2$ | H | H | $OCH_3$ | N | |
| J-6 | O | 0 | – | H | H | $CH(CH_3)CH_2CH_3$ | H | H | $OCH_3$ | N | |
| J-6 | O | 0 | – | H | H | Cl | H | H | $OCH_3$ | N | |
| J-6 | O | 0 | – | H | H | Cl | Cl | H | $OCH_3$ | N | |
| J-6 | O | 0 | – | H | H | Br | Br | H | $OCH_3$ | N | |
| J-6 | O | 0 | – | H | H | H | H | H | $OCH_3$ | N | |
| J-6 | O | 1 | – | H | H | H | H | H | $OCH_3$ | N | |
| J-6 | O | 0 | – | H | H | $CH_3$ | $CH_3$ | H | $OCH_3$ | N | |
| J-16 | O | – | – | H | H | – | H | – | $OCH_3$ | N | |
| J-16 | O | – | – | H | H | – | $CH_3$ | – | $OCH_3$ | N | |
| J-16 | O | – | – | H | H | – | $CH_2CH_3$ | – | $OCH_3$ | N | |
| J-16 | O | – | – | H | H | – | $CH_2CH_2CH_3$ | – | $OCH_3$ | N | |
| J-16 | O | – | – | H | H | – | $CH_2CH_2CH_2CH_3$ | – | $OCH_3$ | N | |
| J-16 | O | – | – | H | *m*-Cl | – | H | – | $OCH_3$ | N | |
| J-16 | O | – | – | H | *o*-Cl | – | H | – | $OCH_3$ | N | |
| J-16 | O | – | – | H | *p*-Cl | – | H | – | $OCH_3$ | N | |
| J-21 | O | – | – | H | H | H | – | – | $OCH_3$ | N | |
| J-21 | O | – | – | H | H | $CH_3$ | – | – | $OCH_3$ | N | |
| J-22 | O | O | O | H | H | H | H | – | $OCH_3$ | N | |

Table III (continued)

| J | W | n | $W_1$ | R | $R_1$ | $R_3$ | $R_4$ | $R_5$ | Y | Z | m.p. (°C) |
|---|---|---|---|---|---|---|---|---|---|---|---|
| J-22 | O | O | S | H | H | H | H | - | $OCH_3$ | N | |
| J-22 | O | 1 | O | H | H | H | H | - | $OCH_3$ | N | |
| J-23 | O | - | - | H | H | H | - | - | $OCH_3$ | N | |
| J-24 | O | - | - | H | H | - | $CH_3$ | $CH_3$ | $OCH_3$ | N | |
| J-24 | O | - | - | H | H | - | $CH_3$ | H | $OCH_3$ | N | 182-185 |
| J-24 | O | - | - | H | H | - | $CH_2CH_3$ | H | $OCH_3$ | N | |
| J-24 | O | - | - | H | H | - | $CH_2CH_2CH_3$ | H | $OCH_3$ | N | |
| J-24 | O | - | - | H | H | - | $CH(CH_3)_2$ | H | $OCH_3$ | N | |
| J-24 | O | - | - | H | H | - | $CH_2CH_2CH_2CH_3$ | H | $OCH_3$ | N | |
| J-24 | O | - | - | H | H | - | $CH_2CH(CH_3)_2$ | H | $OCH_3$ | N | |
| J-24 | O | - | - | H | H | - | $CH(CH_3)CH_2CH_3$ | H | $OCH_3$ | N | |
| J-24 | O | - | - | H | H | - | $C(CH_3)_3$ | H | $OCH_3$ | N | |
| J-24 | O | - | - | H | H | - | Cl | H | $OCH_3$ | N | |
| J-24 | O | - | - | H | H | - | Br | H | $OCH_3$ | N | |
| J-24 | O | - | - | H | *o*-Cl | - | $CH_3$ | H | $OCH_3$ | N | |
| J-24 | O | - | - | H | *m*-Cl | - | $CH_3$ | H | $OCH_3$ | N | |
| J-24 | O | - | - | H | *p*-Cl | - | $CH_3$ | H | $OCH_3$ | N | |
| J-24 | O | - | - | H | *m*-$CH_3$ | - | $CH_3$ | H | $OCH_3$ | N | |
| J-24 | O | - | - | H | *m*-$OCH_3$ | - | $CH_3$ | H | $OCH_3$ | N | |
| J-16 | O | - | - | H | H | - | H | - | $OCH_3$ | CH | |
| J-16 | O | - | - | H | H | - | H | - | $CH_3$ | CH | |
| J-16 | O | - | - | H | H | - | H | - | $OCH_2CH_3$ | N | |
| J-24 | O | 1 | - | H | H | - | $CH_3$ | H | $OCH_3$ | CH | |
| J-24 | O | 1 | - | H | H | - | $CH_3$ | H | $CH_3$ | CH | |
| J-24 | O | 1 | - | H | H | - | $CH_3$ | H | $OCH_2CH_3$ | N | |

Table IV

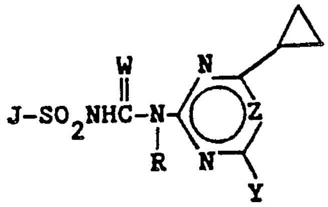

wherein W is O.

| J | Q | R | $R_8$ | $R_9$ | $R_{10}$ | $R_{11}$ | Y | Z | m.p. (°C) |
|---|---|---|---|---|---|---|---|---|---|
| $J_{27}$ | O | H | H | $CH_3$ | H | H | $CH_3$ | N | |
| $J_{27}$ | O | H | H | $CH_3$ | H | H | $OCH_3$ | N | |
| $J_{27}$ | O | H | H | $CH_3$ | H | H | $OCH_2CH_3$ | N | |
| $J_{27}$ | O | H | H | $CH_3$ | H | H | $CH_2OCH_3$ | N | |
| $J_{27}$ | O | H | H | $CH_3$ | H | H | $NHCH_3$ | N | |
| $J_{27}$ | O | H | H | $CH_3$ | H | H | $N(CH_3)_2$ | N | |
| $J_{27}$ | O | H | H | $CH_3$ | H | H | $CH_3$ | CH | |
| $J_{27}$ | O | H | H | $CH_3$ | H | H | $OCH_3$ | CH | |
| $J_{27}$ | O | H | H | $CH_3$ | H | H | $OCH_2CH_3$ | CH | |
| $J_{27}$ | O | H | H | $CH_3$ | H | H | $CH_2OCH_3$ | CH | |
| $J_{27}$ | O | H | H | $CH_3$ | H | H | $NHCH_3$ | CH | |
| $J_{27}$ | O | H | H | $CH_3$ | H | H | $N(CH_3)_2$ | CH | |
| $J_{27}$ | O | $CH_3$ | H | $CH_3$ | H | H | $OCH_3$ | N | |
| $J_{27}$ | O | $CH_3$ | H | $CH_3$ | H | H | $OCH_3$ | CH | |
| $J_{27}$ | O | H | $CH_3$ | $CH_3$ | H | H | $OCH_3$ | N | |
| $J_{27}$ | O | H | $CH_3$ | $CH_3$ | H | H | $OCH_2CH_3$ | N | |
| $J_{27}$ | O | H | $OCH_3$ | $CH_3$ | H | H | $OCH_3$ | N | |
| $J_{27}$ | O | H | $OCH_3$ | $CH_3$ | H | H | $OCH_2CH_3$ | N | |
| $J_{27}$ | O | H | Cl | $CH_3$ | H | H | $OCH_3$ | N | |
| $J_{27}$ | O | H | Cl | $CH_3$ | H | H | $OCH_2CH_3$ | N | |
| $J_{27}$ | O | H | Br | $CH_3$ | H | H | $OCH_3$ | N | |
| $J_{27}$ | O | H | $CO_2CH_3$ | $CH_3$ | H | H | $OCH_3$ | N | |
| $J_{27}$ | O | H | $SO_2CH_3$ | $CH_3$ | H | H | $OCH_3$ | N | |
| $J_{27}$ | O | H | $SO_2CH_2CH_3$ | $CH_3$ | H | H | $OCH_3$ | N | |
| $J_{27}$ | O | H | $OSO_2CH_3$ | $CH_3$ | H | H | $OCH_3$ | N | |
| $J_{27}$ | O | H | $OSO_2CH_2CH_3$ | $CH_3$ | H | H | $OCH_3$ | N | |
| $J_{27}$ | O | H | $SO_2N(CH_3)_2$ | $CH_3$ | H | H | $OCH_3$ | N | |
| $J_{27}$ | O | H | H | H | H | H | $OCH_3$ | N | 188-191 |

Table IV (continued)

wherein W is O.

| J | Q | R | $R_8$ | $R_9$ | $R_{10}$ | $R_{11}$ | Y | Z |
|---|---|---|---|---|---|---|---|---|
| $J_{27}$ | O | H | H | H | H | H | $OCH_2CH_3$ | N |
| $J_{27}$ | O | H | H | H | H | H | $OCH_3$ | CH |
| $J_{27}$ | O | H | H | $CH_3$ | $CH_3$ | H | $OCH_3$ | N |
| $J_{27}$ | O | H | H | $CH_3$ | $CH_3$ | H | $OCH_2CH_3$ | N |
| $J_{27}$ | O | H | H | $CH_3$ | $CH_3$ | H | $OCH_3$ | CH |
| $J_{27}$ | O | H | H | $CH_2CH_3$ | H | H | $OCH_3$ | N |
| $J_{27}$ | O | H | H | $CH_2CH_2CH_3$ | H | H | $OCH_3$ | N |
| $J_{27}$ | O | H | H | $CH_2CH_2$ | $CH_3$ | H | $OCH_3$ | N |
| $J_{27}$ | O | H | $OCH_3$ | $CH_3$ | $CH_3$ | H | $OCH_3$ | N |
| $J_{27}$ | O | H | Cl | $CH_3$ | $CH_3$ | H | $OCH_3$ | N |
| $J_{27}$ | O | H | H | $CH_3$ | H | $CH_3$ | $OCH_3$ | N |
| $J_{27}$ | O | H | H | $CH_3$ | H | $CH_3$ | $OCH_3$ | CH |
| $J_{27}$ | O | H | H | $CH_2CH_3$ | H | $CH_3$ | $OCH_3$ | N |
| $J_{27}$ | O | H | Cl | $CH_3$ | H | $CH_3$ | $OCH_3$ | N |
| $J_{27}$ | S | H | H | $CH_3$ | H | H | $CH_3$ | N |
| $J_{27}$ | S | H | H | $CH_3$ | H | H | $OCH_3$ | N |
| $J_{27}$ | S | H | H | $CH_3$ | H | H | $OCH_2CH_3$ | N |
| $J_{27}$ | S | H | H | $CH_3$ | H | H | $CH_2OCH_3$ | N |
| $J_{27}$ | S | H | H | $CH_3$ | H | H | $NHCH_3$ | N |
| $J_{27}$ | S | H | H | $CH_3$ | H | H | $N(CH_3)_2$ | N |
| $J_{27}$ | S | H | H | $CH_3$ | H | H | $CH_3$ | CH |
| $J_{27}$ | S | H | H | $CH_3$ | H | H | $OCH_3$ | CH |
| $J_{27}$ | S | H | H | $CH_3$ | H | H | $OCH_2CH_3$ | CH |
| $J_{27}$ | S | H | H | $CH_3$ | H | H | $CH_2OCH_3$ | CH |
| $J_{27}$ | S | H | H | $CH_3$ | H | H | $NHCH_3$ | CH |
| $J_{27}$ | S | H | H | $CH_3$ | H | H | $N(CH_3)_2$ | CH |
| $J_{27}$ | S | $CH_3$ | H | $CH_3$ | H | H | $OCH_3$ | N |
| $J_{27}$ | S | $CH_3$ | H | $CH_3$ | H | H | $OCH_3$ | CH |
| $J_{27}$ | S | H | $CH_3$ | $CH_3$ | H | H | $OCH_3$ | N |
| $J_{27}$ | S | H | $CH_3$ | $CH_3$ | H | H | $OCH_2CH_3$ | N |
| $J_{27}$ | S | H | $OCH_3$ | $CH_3$ | H | H | $OCH_3$ | N |
| $J_{27}$ | S | H | $OCH_3$ | $CH_3$ | H | H | $OCH_2CH_3$ | N |

Table IV (continued)

wherein W is O.

| J | Q | R | $R_8$ | $R_9$ | $R_{10}$ | $R_{11}$ | Y | Z |
|---|---|---|---|---|---|---|---|---|
| $J_{27}$ | S | H | Cl | $CH_3$ | H | H | $OCH_3$ | N |
| $J_{27}$ | S | H | Cl | $CH_3$ | H | H | $OCH_2CH_3$ | N |
| $J_{27}$ | S | H | Br | $CH_3$ | H | H | $OCH_3$ | N |
| $J_{27}$ | S | H | $CO_2CH_3$ | $CH_3$ | H | H | $OCH_3$ | N |
| $J_{27}$ | S | H | $SO_2CH_3$ | $CH_3$ | H | H | $OCH_3$ | N |
| $J_{27}$ | S | H | $SO_2CH_2CH_3$ | $CH_3$ | H | H | $OCH_3$ | N |
| $J_{27}$ | S | H | $OSO_2CH_3$ | $CH_3$ | H | H | $OCH_3$ | N |
| $J_{27}$ | S | H | $OSO_2CH_2CH_3$ | $CH_3$ | H | H | $OCH_3$ | N |
| $J_{27}$ | S | H | H | H | H | H | $OCH_3$ | N |
| $J_{27}$ | S | H | H | H | H | H | $OCH_2CH_3$ | N |
| $J_{27}$ | S | H | H | H | H | H | $OCH_3$ | CH |
| $J_{27}$ | S | H | H | $CH_3$ | $CH_3$ | H | $OCH_3$ | N |
| $J_{27}$ | S | H | H | $CH_3$ | $CH_3$ | H | $OCH_2CH_3$ | N |
| $J_{27}$ | S | H | H | $CH_3$ | $CH_3$ | H | $OCH_3$ | CH |
| $J_{27}$ | S | H | H | $CH_2CH_3$ | H | H | $OCH_3$ | N |
| $J_{27}$ | S | H | H | $CH_2CH_2CH_3$ | H | H | $OCH_3$ | N |
| $J_{27}$ | S | H | H | $CH_2CH_2$ | $CH_3$ | H | $OCH_3$ | N |
| $J_{27}$ | S | H | $OCH_3$ | $CH_3$ | $CH_3$ | H | $OCH_3$ | N |
| $J_{27}$ | S | H | Cl | $CH_3$ | $CH_3$ | H | $OCH_3$ | N |
| $J_{27}$ | S | H | H | $CH_3$ | H | $CH_3$ | $OCH_3$ | N |
| $J_{27}$ | S | H | H | $CH_3$ | H | $CH_3$ | $OCH_3$ | CH |
| $J_{27}$ | S | H | H | $CH_2CH_3$ | H | $CH_3$ | $OCH_3$ | N |
| $J_{27}$ | S | H | Cl | $CH_3$ | H | $CH_3$ | $OCH_3$ | N |
| $J_{27}$ | $SO_2$ | H | H | $CH_3$ | H | H | $CH_3$ | N |
| $J_{27}$ | $SO_2$ | H | H | $CH_3$ | H | H | $OCH_3$ | N |
| $J_{27}$ | $SO_2$ | H | H | $CH_3$ | H | H | $OCH_2CH_3$ | N |
| $J_{27}$ | $SO_2$ | H | H | $CH_3$ | H | H | $CH_2OCH_3$ | N |
| $J_{27}$ | $SO_2$ | H | H | $CH_3$ | H | H | $NHCH_3$ | N |
| $J_{27}$ | $SO_2$ | H | H | $CH_3$ | H | H | $N(CH_3)_2$ | N |
| $J_{27}$ | $SO_2$ | H | H | $CH_3$ | H | H | $CH_3$ | CH |
| $J_{27}$ | $SO_2$ | H | H | $CH_3$ | H | H | $OCH_3$ | CH |
| $J_{27}$ | $SO_2$ | H | H | $CH_3$ | H | H | $OCH_2CH_3$ | CH |

Table IV (continued)

wherein W is O.

| J | Q | R | $R_8$ | $R_9$ | $R_{10}$ | $R_{11}$ | Y | Z |
|---|---|---|---|---|---|---|---|---|
| $J_{27}$ | $SO_2$ | H | H | $CH_3$ | H | H | $CH_2OCH_3$ | CH |
| $J_{27}$ | $SO_2$ | H | H | $CH_3$ | H | H | $NHCH_3$ | CH |
| $J_{27}$ | $SO_2$ | H | H | $CH_3$ | H | H | $N(CH_3)_2$ | CH |
| $J_{27}$ | $SO_2$ | $CH_3$ | H | $CH_3$ | H | H | $OCH_3$ | N |
| $J_{27}$ | $SO_2$ | $CH_3$ | H | $CH_3$ | H | H | $OCH_3$ | CH |
| $J_{27}$ | $SO_2$ | H | $CH_3$ | $CH_3$ | H | H | $OCH_3$ | N |
| $J_{27}$ | $SO_2$ | H | $CH_3$ | $CH_3$ | H | H | $OCH_2CH_3$ | N |
| $J_{27}$ | $SO_2$ | H | $OCH_3$ | $CH_3$ | H | H | $OCH_3$ | N |
| $J_{27}$ | $SO_2$ | H | $OCH_3$ | $CH_3$ | H | H | $OCH_2CH_3$ | N |
| $J_{27}$ | $SO_2$ | H | Cl | $CH_3$ | H | H | $OCH_3$ | N |
| $J_{27}$ | $SO_2$ | H | Cl | $CH_3$ | H | H | $OCH_2CH_3$ | N |
| $J_{27}$ | $SO_2$ | H | Br | $CH_3$ | H | H | $OCH_3$ | N |
| $J_{27}$ | $SO_2$ | H | $CO_2CH_3$ | $CH_3$ | H | H | $OCH_3$ | N |
| $J_{27}$ | $SO_2$ | H | $SO_2CH_3$ | $CH_3$ | H | H | $OCH_3$ | N |
| $J_{27}$ | $SO_2$ | H | $SO_2CH_2CH_3$ | $CH_3$ | H | H | $OCH_3$ | N |
| $J_{27}$ | $SO_2$ | H | $OSO_2CH_3$ | $CH_3$ | H | H | $OCH_3$ | N |
| $J_{27}$ | $SO_2$ | H | $OSO_2CH_2CH_3$ | $CH_3$ | H | H | $OCH_3$ | N |
| $J_{27}$ | $SO_2$ | H | H | H | H | H | $OCH_3$ | N |
| $J_{27}$ | $SO_2$ | H | H | H | H | H | $OCH_2CH_3$ | N |
| $J_{27}$ | $SO_2$ | H | H | H | H | H | $OCH_3$ | CH |
| $J_{27}$ | $SO_2$ | H | H | $CH_3$ | $CH_3$ | H | $OCH_3$ | N |
| $J_{27}$ | $SO_2$ | H | H | $CH_3$ | $CH_3$ | H | $OCH_2CH_3$ | N |
| $J_{27}$ | $SO_2$ | H | H | $CH_3$ | $CH_3$ | H | $OCH_3$ | CH |
| $J_{27}$ | $SO_2$ | H | H | $CH_2CH_3$ | H | H | $OCH_3$ | N |
| $J_{27}$ | $SO_2$ | H | H | $CH_2CH_2CH_3$ | H | H | $OCH_3$ | N |
| $J_{27}$ | $SO_2$ | H | H | $CH_2CH_2$ | $CH_3$ | H | $OCH_3$ | N |
| $J_{27}$ | $SO_2$ | H | $OCH_3$ | $CH_3$ | $CH_3$ | H | $OCH_3$ | N |
| $J_{27}$ | $SO_2$ | H | Cl | $CH_3$ | $CH_3$ | H | $OCH_3$ | N |
| $J_{27}$ | $SO_2$ | H | H | $CH_3$ | H | $CH_3$ | $OCH_3$ | N |
| $J_{27}$ | $SO_2$ | H | H | $CH_3$ | H | $CH_3$ | $OCH_3$ | CH |
| $J_{27}$ | $SO_2$ | H | H | $CH_2CH_3$ | H | $CH_3$ | $OCH_3$ | N |
| $J_{27}$ | $SO_2$ | H | Cl | $CH_3$ | H | $CH_3$ | $OCH_3$ | N |

Table IV (continued)

wherein W is O.

| J | Q | R | $R_8$ | $R_9$ | $R_{10}$ | $R_{11}$ | Y | Z |
|---|---|---|---|---|---|---|---|---|
| $J_{30}$ | O | H | H | $CH_3$ | H | - | $CH_3$ | N |
| $J_{30}$ | O | H | H | $CH_3$ | H | - | $OCH_3$ | N |
| $J_{30}$ | O | H | H | $CH_3$ | H | - | $OCH_2CH_3$ | N |
| $J_{30}$ | O | H | H | $CH_3$ | H | - | $CH_2OCH_3$ | N |
| $J_{30}$ | O | H | H | $CH_3$ | H | - | $NHCH_3$ | N |
| $J_{30}$ | O | H | H | $CH_3$ | H | - | $N(CH_3)_2$ | N |
| $J_{30}$ | O | H | H | $CH_3$ | H | - | $CH_3$ | CH |
| $J_{30}$ | O | H | H | $CH_3$ | H | - | $OCH_3$ | CH |
| $J_{30}$ | O | H | H | $CH_3$ | H | - | $OCH_2CH_3$ | CH |
| $J_{30}$ | O | H | H | $CH_3$ | H | - | $CH_2OCH_3$ | CH |
| $J_{30}$ | O | H | H | $CH_3$ | H | - | $NHCH_3$ | CH |
| $J_{30}$ | O | H | H | $CH_3$ | H | - | $N(CH_3)_2$ | CH |
| $J_{30}$ | O | $CH_3$ | H | $CH_3$ | H | - | $OCH_3$ | N |
| $J_{30}$ | O | $CH_3$ | H | $CH_3$ | H | - | $OCH_3$ | CH |
| $J_{30}$ | O | H | $CH_3$ | $CH_3$ | H | - | $OCH_3$ | N |
| $J_{30}$ | O | H | $CH_3$ | $CH_3$ | H | - | $OCH_2CH_3$ | N |
| $J_{30}$ | O | H | $OCH_3$ | $CH_3$ | H | - | $OCH_3$ | N |
| $J_{30}$ | O | H | $OCH_3$ | $CH_3$ | H | - | $OCH_2CH_3$ | N |
| $J_{30}$ | O | H | Cl | $CH_3$ | H | - | $OCH_3$ | N |
| $J_{30}$ | O | H | Cl | $CH_3$ | H | - | $OCH_2CH_3$ | N |
| $J_{30}$ | O | H | Br | $CH_3$ | H | - | $OCH_3$ | N |
| $J_{30}$ | O | H | $CO_2CH_3$ | $CH_3$ | H | - | $OCH_3$ | N |
| $J_{30}$ | O | H | $SO_2CH_3$ | $CH_3$ | H | - | $OCH_3$ | N |
| $J_{30}$ | O | H | $SO_2CH_2CH_3$ | $CH_3$ | H | - | $OCH_3$ | N |
| $J_{30}$ | O | H | $OSO_2CH_3$ | $CH_3$ | H | - | $OCH_3$ | N |
| $J_{30}$ | O | H | $OSO_2CH_2CH_3$ | $CH_3$ | H | - | $OCH_3$ | N |
| $J_{30}$ | O | H | $SO_2N(CH_3)_2$ | $CH_3$ | H | - | $OCH_3$ | N |
| $J_{30}$ | O | H | H | H | H | - | $OCH_3$ | N |
| $J_{30}$ | O | H | H | H | H | - | $OCH_2CH_3$ | N |
| $J_{30}$ | O | H | H | H | H | - | $OCH_3$ | CH |
| $J_{30}$ | O | H | H | $CH_3$ | $CH_3$ | - | $OCH_3$ | N |
| $J_{30}$ | O | H | H | $CH_3$ | $CH_3$ | - | $OCH_2CH_3$ | N |

Table IV (continued)

wherein W is O.

| J | Q | R | $R_8$ | $R_9$ | $R_{10}$ | $R_{11}$ | Y | Z |
|---|---|---|---|---|---|---|---|---|
| $J_{30}$ | O | H | H | $CH_3$ | $CH_3$ | - | $OCH_3$ | CH |
| $J_{30}$ | O | H | H | $CH_2CH_3$ | H | - | $OCH_3$ | N |
| $J_{30}$ | O | H | H | $CH_2CH_2CH_3$ | H | - | $OCH_3$ | N |
| $J_{30}$ | O | H | H | $CH_2CH_2$ | $CH_3$ | - | $OCH_3$ | N |
| $J_{30}$ | O | H | $OCH_3$ | $CH_3$ | $CH_3$ | - | $OCH_3$ | N |
| $J_{30}$ | O | H | Cl | $CH_3$ | $CH_3$ | - | $OCH_3$ | N |
| $J_{30}$ | S | H | H | $CH_3$ | H | - | $CH_3$ | N |
| $J_{30}$ | S | H | H | $CH_3$ | H | - | $OCH_3$ | N |
| $J_{30}$ | S | H | H | $CH_3$ | H | - | $OCH_2CH_3$ | N |
| $J_{30}$ | S | H | H | $CH_3$ | H | - | $CH_2OCH_3$ | N |
| $J_{30}$ | S | H | H | $CH_3$ | H | - | $NHCH_3$ | N |
| $J_{30}$ | S | H | H | $CH_3$ | H | - | $N(CH_3)_2$ | N |
| $J_{30}$ | S | H | H | $CH_3$ | H | - | $CH_3$ | CH |
| $J_{30}$ | S | H | H | $CH_3$ | H | - | $OCH_3$ | CH |
| $J_{30}$ | S | H | H | $CH_3$ | H | - | $OCH_2CH_3$ | CH |
| $J_{30}$ | S | H | H | $CH_3$ | H | - | $CH_2OCH_3$ | CH |
| $J_{30}$ | S | H | H | $CH_3$ | H | - | $NHCH_3$ | CH |
| $J_{30}$ | S | H | H | $CH_3$ | H | - | $N(CH_3)_2$ | CH |
| $J_{30}$ | S | $CH_3$ | H | $CH_3$ | H | - | $OCH_3$ | N |
| $J_{30}$ | S | $CH_3$ | H | $CH_3$ | H | - | $OCH_3$ | CH |
| $J_{30}$ | S | H | $CH_3$ | $CH_3$ | H | - | $OCH_3$ | N |
| $J_{30}$ | S | H | $CH_3$ | $CH_3$ | H | - | $OCH_2CH_3$ | N |
| $J_{30}$ | S | H | $OCH_3$ | $CH_3$ | H | - | $OCH_3$ | N |
| $J_{30}$ | S | H | $OCH_3$ | $CH_3$ | H | - | $OCH_2CH_3$ | N |
| $J_{30}$ | S | H | Cl | $CH_3$ | H | - | $OCH_3$ | N |
| $J_{30}$ | S | H | Cl | $CH_3$ | H | - | $OCH_2CH_3$ | N |
| $J_{30}$ | S | H | Br | $CH_3$ | H | - | $OCH_3$ | N |
| $J_{30}$ | S | H | $CO_2CH_3$ | $CH_3$ | H | - | $OCH_3$ | N |
| $J_{30}$ | S | H | $SO_2CH_3$ | $CH_3$ | H | - | $OCH_3$ | N |
| $J_{30}$ | S | H | $SO_2CH_2CH_3$ | $CH_3$ | H | - | $OCH_3$ | N |
| $J_{30}$ | S | H | $OSO_2CH_3$ | $CH_3$ | H | - | $OCH_3$ | N |
| $J_{30}$ | S | H | $OSO_2CH_2CH_3$ | $CH_3$ | H | - | $OCH_3$ | N |

Table IV (continued)

wherein W is O.

| J | Q | R | $R_8$ | $R_9$ | $R_{10}$ | $R_{11}$ | Y | Z |
|---|---|---|---|---|---|---|---|---|
| $J_{30}$ | S | H | H | H | H | - | $OCH_3$ | N |
| $J_{30}$ | S | H | H | H | H | - | $OCH_2CH_3$ | N |
| $J_{30}$ | S | H | H | H | H | - | $OCH_3$ | CH |
| $J_{30}$ | S | H | H | $CH_3$ | $CH_3$ | - | $OCH_3$ | N |
| $J_{30}$ | S | H | H | $CH_3$ | $CH_3$ | - | $OCH_2CH_3$ | N |
| $J_{30}$ | S | H | H | $CH_3$ | $CH_3$ | - | $OCH_3$ | CH |
| $J_{30}$ | S | H | H | $CH_2CH_3$ | H | - | $OCH_3$ | N |
| $J_{30}$ | S | H | H | $CH_2CH_2CH_3$ | H | - | $OCH_3$ | N |
| $J_{30}$ | S | H | H | $CH_2CH_2$ | $CH_3$ | - | $OCH_3$ | N |
| $J_{30}$ | S | H | $OCH_3$ | $CH_3$ | $CH_3$ | - | $OCH_3$ | N |
| $J_{30}$ | S | H | Cl | $CH_3$ | $CH_3$ | - | $OCH_3$ | N |
| $J_{30}$ | $SO_2$ | H | H | $CH_3$ | H | - | $CH_3$ | N |
| $J_{30}$ | $SO_2$ | H | H | $CH_3$ | H | - | $OCH_3$ | N |
| $J_{30}$ | $SO_2$ | H | H | $CH_3$ | H | - | $OCH_2CH_3$ | N |
| $J_{30}$ | $SO_2$ | H | H | $CH_3$ | H | - | $CH_2OCH_3$ | N |
| $J_{30}$ | $SO_2$ | H | H | $CH_3$ | H | - | $NHCH_3$ | N |
| $J_{30}$ | $SO_2$ | H | H | $CH_3$ | H | - | $N(CH_3)_2$ | N |
| $J_{30}$ | $SO_2$ | H | H | $CH_3$ | H | - | $CH_3$ | CH |
| $J_{30}$ | $SO_2$ | H | H | $CH_3$ | H | - | $OCH_3$ | CH |
| $J_{30}$ | $SO_2$ | H | H | $CH_3$ | H | - | $OCH_2CH_3$ | CH |
| $J_{30}$ | $SO_2$ | H | H | $CH_3$ | H | - | $CH_2OCH_3$ | CH |
| $J_{30}$ | $SO_2$ | H | H | $CH_3$ | H | - | $NHCH_3$ | CH |
| $J_{30}$ | $SO_2$ | H | H | $CH_3$ | H | - | $N(CH_3)_2$ | CH |
| $J_{30}$ | $SO_2$ | $CH_3$ | H | $CH_3$ | H | - | $OCH_3$ | N |
| $J_{30}$ | $SO_2$ | $CH_3$ | H | $CH_3$ | H | - | $OCH_3$ | CH |
| $J_{30}$ | $SO_2$ | H | $CH_3$ | $CH_3$ | H | - | $OCH_3$ | N |
| $J_{30}$ | $SO_2$ | H | $CH_3$ | $CH_3$ | H | - | $OCH_2CH_3$ | N |
| $J_{30}$ | $SO_2$ | H | $OCH_3$ | $CH_3$ | H | - | $OCH_3$ | N |
| $J_{30}$ | $SO_2$ | H | $OCH_3$ | $CH_3$ | H | - | $OCH_2CH_3$ | N |
| $J_{30}$ | $SO_2$ | H | Cl | $CH_3$ | H | - | $OCH_3$ | N |
| $J_{30}$ | $SO_2$ | H | Cl | $CH_3$ | H | - | $OCH_2CH_3$ | N |
| $J_{30}$ | $SO_2$ | H | Br | $CH_3$ | H | - | $OCH_3$ | N |

Table IV (continued)

wherein W is O.

| J | Q | R | $R_8$ | $R_9$ | $R_{10}$ | $R_{11}$ | Y | Z |
|---|---|---|---|---|---|---|---|---|
| $J_{30}$ | $SO_2$ | H | $CO_2CH_3$ | $CH_3$ | H | - | $OCH_3$ | N |
| $J_{30}$ | $SO_2$ | H | $SO_2CH_3$ | $CH_3$ | H | - | $OCH_3$ | N |
| $J_{30}$ | $SO_2$ | H | $SO_2CH_2CH_3$ | $CH_3$ | H | - | $OCH_3$ | N |
| $J_{30}$ | $SO_2$ | H | $OSO_2CH_3$ | $CH_3$ | H | - | $OCH_3$ | N |
| $J_{30}$ | $SO_2$ | H | $OSO_2CH_2CH_3$ | $CH_3$ | H | - | $OCH_3$ | N |
| $J_{30}$ | $SO_2$ | H | $SO_2N(CH_3)_2$ | $CH_3$ | H | - | $OCH_3$ | N |
| $J_{30}$ | $SO_2$ | H | H | H | H | - | $OCH_3$ | N |
| $J_{30}$ | $SO_2$ | H | H | H | H | - | $OCH_2CH_3$ | N |
| $J_{30}$ | $SO_2$ | H | H | H | H | - | $OCH_3$ | CH |
| $J_{30}$ | $SO_2$ | H | H | $CH_3$ | $CH_3$ | - | $OCH_3$ | N |
| $J_{30}$ | $SO_2$ | H | H | $CH_3$ | $CH_3$ | - | $OCH_2CH_3$ | N |
| $J_{30}$ | $SO_2$ | H | H | $CH_3$ | $CH_3$ | - | $OCH_3$ | CH |
| $J_{30}$ | $SO_2$ | H | H | $CH_2CH_3$ | H | - | $OCH_3$ | N |
| $J_{30}$ | $SO_2$ | H | H | $CH_2CH_2CH_3$ | H | - | $OCH_3$ | N |
| $J_{30}$ | $SO_2$ | H | H | $CH_2CH_2$ | $CH_3$ | - | $OCH_3$ | N |
| $J_{30}$ | $SO_2$ | H | $OCH_3$ | $CH_3$ | $CH_3$ | - | $OCH_3$ | N |
| $J_{30}$ | $SO_2$ | H | Cl | $CH_3$ | $CH_3$ | - | $OCH_3$ | N |
| $J_{28}$ | O | H | H | H | H | H | $OCH_3$ | N |
| $J_{28}$ | O | H | H | $CH_3$ | H | H | $OCH_3$ | N |
| $J_{28}$ | O | H | H | $CH_3$ | H | H | $OCH_2CH_3$ | N |
| $J_{28}$ | O | H | H | $CH_3$ | $CH_3$ | H | $OCH_3$ | N |
| $J_{28}$ | O | H | H | $CH_3$ | $CH_3$ | H | $OCH_2CH_3$ | N |
| $J_{28}$ | O | H | H | $CH_3$ | $CH_3$ | H | $OCH_3$ | CH |
| $J_{28}$ | O | H | Cl | $CH_3$ | $CH_3$ | H | $OCH_3$ | N |
| $J_{28}$ | O | H | $OCH_3$ | $CH_3$ | $CH_3$ | H | $OCH_3$ | N |
| $J_{28}$ | O | $CH_3$ | H | $CH_3$ | $CH_3$ | H | $OCH_3$ | N |
| $J_{28}$ | O | H | H | H | H | $CH_3$ | $OCH_3$ | N |
| $J_{28}$ | O | H | H | $CH_2CH_3$ | $CH_3$ | H | $OCH_3$ | N |
| $J_{31}$ | O | H | H | H | H | - | $OCH_3$ | N |
| $J_{31}$ | O | H | H | $CH_3$ | H | - | $OCH_3$ | N |
| $J_{31}$ | O | H | H | $CH_3$ | H | - | $OCH_2CH_3$ | N |
| $J_{31}$ | O | H | H | $CH_3$ | H | - | $OCH_3$ | CH |

Table IV (continued)

wherein W is O.

| J | Q | R | $R_8$ | $R_9$ | $R_{10}$ | $R_{11}$ | Y | Z |
|---|---|---|---|---|---|---|---|---|
| $J_{31}$ | O | H | H | $CH_3$ | H | - | $CH_3$ | CH |
| $J_{31}$ | O | H | Cl | $CH_3$ | H | - | $OCH_3$ | N |
| $J_{31}$ | O | H | H | $CH_3$ | $CH_3$ | - | $OCH_3$ | N |
| $J_{31}$ | O | H | Cl | $CH_3$ | $CH_3$ | - | $OCH_3$ | N |
| $J_{31}$ | O | H | $OCH_3$ | $CH_3$ | $CH_3$ | - | $OCH_3$ | N |
| $J_{28}$ | S | H | H | H | H | H | $OCH_3$ | N |
| $J_{28}$ | S | H | H | $CH_3$ | H | H | $OCH_3$ | N |
| $J_{28}$ | S | H | H | $CH_3$ | H | H | $OCH_2CH_3$ | N |
| $J_{28}$ | S | H | H | $CH_3$ | $CH_3$ | H | $OCH_3$ | N |
| $J_{28}$ | S | H | H | $CH_3$ | $CH_3$ | H | $OCH_2CH_3$ | N |
| $J_{28}$ | S | H | H | $CH_3$ | $CH_3$ | H | $OCH_3$ | CH |
| $J_{28}$ | S | H | Cl | $CH_3$ | $CH_3$ | H | $OCH_3$ | N |
| $J_{28}$ | S | H | $OCH_3$ | $CH_3$ | $CH_3$ | H | $OCH_3$ | N |
| $J_{28}$ | S | $CH_3$ | H | $CH_3$ | $CH_3$ | H | $OCH_3$ | N |
| $J_{28}$ | S | H | H | H | H | $CH_3$ | $OCH_3$ | N |
| $J_{28}$ | S | H | H | $CH_2CH_3$ | $CH_3$ | H | $OCH_3$ | N |
| $J_{31}$ | S | H | H | H | H | - | $OCH_3$ | N |
| $J_{31}$ | S | H | H | $CH_3$ | H | - | $OCH_3$ | N |
| $J_{31}$ | S | H | H | $CH_3$ | H | - | $OCH_2CH_3$ | N |
| $J_{31}$ | S | H | H | $CH_3$ | H | - | $OCH_3$ | CH |
| $J_{31}$ | S | H | H | $CH_3$ | H | - | $CH_3$ | CH |
| $J_{31}$ | S | H | Cl | $CH_3$ | H | - | $OCH_3$ | N |
| $J_{31}$ | S | H | H | $CH_3$ | $CH_3$ | - | $OCH_3$ | N |
| $J_{31}$ | S | H | Cl | $CH_3$ | $CH_3$ | - | $OCH_3$ | N |
| $J_{31}$ | S | H | $OCH_3$ | $CH_3$ | $CH_3$ | - | $OCH_3$ | N |
| $J_{28}$ | $SO_2$ | H | H | H | H | H | $OCH_3$ | N |
| $J_{28}$ | $SO_2$ | H | H | $CH_3$ | H | H | $OCH_3$ | N |
| $J_{28}$ | $SO_2$ | H | H | $CH_3$ | H | H | $OCH_2CH_3$ | N |
| $J_{28}$ | $SO_2$ | H | H | $CH_3$ | $CH_3$ | H | $OCH_3$ | N |
| $J_{28}$ | $SO_2$ | H | H | $CH_3$ | $CH_3$ | H | $OCH_2CH_3$ | N |
| $J_{28}$ | $SO_2$ | H | H | $CH_3$ | $CH_3$ | H | $OCH_3$ | CH |
| $J_{28}$ | $SO_2$ | H | Cl | $CH_3$ | $CH_3$ | H | $OCH_3$ | N |

Table IV (continued)

wherein W is O.

| J | Q | R | $R_8$ | $R_9$ | $R_{10}$ | $R_{11}$ | Y | Z |
|---|---|---|---|---|---|---|---|---|
| $J_{28}$ | $SO_2$ | H | $OCH_3$ | $CH_3$ | $CH_3$ | H | $OCH_3$ | N |
| $J_{28}$ | $SO_2$ | $CH_3$ | H | $CH_3$ | $CH_3$ | H | $OCH_3$ | N |
| $J_{28}$ | $SO_2$ | H | H | H | H | $CH_3$ | $OCH_3$ | N |
| $J_{28}$ | $SO_2$ | H | H | $CH_2CH_3$ | $CH_3$ | H | $OCH_3$ | N |
| $J_{31}$ | $SO_2$ | H | H | H | H | - | $OCH_3$ | N |
| $J_{31}$ | $SO_2$ | H | H | $CH_3$ | H | - | $OCH_3$ | N |
| $J_{31}$ | $SO_2$ | H | H | $CH_3$ | H | - | $OCH_2CH_3$ | N |
| $J_{31}$ | $SO_2$ | H | H | $CH_3$ | H | - | $OCH_3$ | CH |
| $J_{31}$ | $SO_2$ | H | H | $CH_3$ | H | - | $CH_3$ | CH |
| $J_{31}$ | $SO_2$ | H | Cl | $CH_3$ | H | - | $OCH_3$ | N |
| $J_{31}$ | $SO_2$ | H | H | $CH_3$ | $CH_3$ | - | $OCH_3$ | N |
| $J_{31}$ | $SO_2$ | H | Cl | $CH_3$ | $CH_3$ | - | $OCH_3$ | N |
| $J_{31}$ | $SO_2$ | H | $OCH_3$ | $CH_3$ | $CH_3$ | - | $OCH_3$ | N |
| $J_{33}$ | - | H | H | H | - | - | $OCH_3$ | N |
| $J_{33}$ | - | H | H | H | - | - | $OCH_3$ | CH |
| $J_{33}$ | - | H | H | $CH_3$ | - | - | $OCH_3$ | N |
| $J_{33}$ | - | H | Cl | $CH_3$ | - | - | $OCH_3$ | N |
| $J_{33}$ | - | H | H | $CH_2CH_3$ | - | - | $OCH_3$ | N |
| $J_{33}$ | - | H | H | $CH_2CH_2CH_3$ | - | - | $OCH_3$ | N |
| $J_{29}$ | S | H | H | $CH_3$ | - | H | $OCH_3$ | N |
| $J_{29}$ | S | H | H | H | - | H | $OCH_3$ | N |
| $J_{29}$ | S | H | H | $CH_3$ | - | H | $OCH_2CH_3$ | N |
| $J_{29}$ | S | H | H | $CH_3$ | - | H | $OCH_3$ | CH |
| $J_{29}$ | S | H | H | $CH_3$ | - | H | $CH_2OCH_3$ | CH |
| $J_{29}$ | S | H | H | $CH_2CH_3$ | - | H | $OCH_3$ | N |
| $J_{29}$ | S | H | H | $CH(CH_3)_2$ | - | H | $OCH_3$ | N |
| $J_{29}$ | S | H | Cl | $CH_3$ | - | H | $OCH_3$ | N |
| $J_{29}$ | S | H | $OCH_3$ | $CH_3$ | - | H | $OCH_3$ | N |
| $J_{29}$ | S | H | H | H | - | $CH_3$ | $OCH_3$ | N |
| $J_{32}$ | S | H | H | H | - | - | $OCH_3$ | N |
| $J_{32}$ | S | H | H | H | - | - | $OCH_2CH_3$ | N |
| $J_{32}$ | S | H | H | H | - | - | $OCH_3$ | CH |

Table IV (continued)

wherein W is O.

| J | Q | R | $R_8$ | $R_9$ | $R_{10}$ | $R_{11}$ | Y | Z |
|---|---|---|---|---|---|---|---|---|
| $J_{32}$ | S | H | H | $CH_3$ | - | - | $NHCH_3$ | N |
| $J_{32}$ | S | H | H | $CH_3$ | - | - | $NH(CH_3)_2$ | N |
| $J_{32}$ | S | H | H | $CH_3$ | - | - | $OCH_3$ | N |
| $J_{32}$ | S | H | H | $CH_3$ | - | - | $OCH_2CH_3$ | N |
| $J_{32}$ | S | H | Cl | $CH_3$ | - | - | $OCH_3$ | N |
| $J_{32}$ | S | H | $CH_3$ | $CH_3$ | - | - | $OCH_3$ | N |
| $J_{32}$ | S | H | H | $CH_2CH_3$ | - | - | $OCH_3$ | N |
| $J_{32}$ | S | H | H | $CH(CH_3)_2$ | - | - | $OCH_3$ | N |
| $J_{32}$ | S | H | H | $CH_2CH_2CH_2$ | - | - | $OCH_3$ | N |
| $J_{29}$ | $SO_2$ | H | H | $CH_3$ | - | H | $OCH_3$ | N |
| $J_{29}$ | $SO_2$ | H | H | H | - | H | $OCH_3$ | N |
| $J_{29}$ | $SO_2$ | H | H | $CH_3$ | - | H | $OCH_2CH_3$ | N |
| $J_{29}$ | $SO_2$ | H | H | $CH_3$ | - | H | $OCH_3$ | CH |
| $J_{29}$ | $SO_2$ | H | H | $CH_3$ | - | H | $CH_2OCH_3$ | CH |
| $J_{29}$ | $SO_2$ | H | H | $CH_2CH_3$ | - | H | $OCH_3$ | N |
| $J_{29}$ | $SO_2$ | H | H | $CH(CH_3)_2$ | - | H | $OCH_3$ | N |
| $J_{29}$ | $SO_2$ | H | Cl | $CH_3$ | - | H | $OCH_3$ | N |
| $J_{29}$ | $SO_2$ | H | $OCH_3$ | $CH_3$ | - | H | $OCH_3$ | N |
| $J_{29}$ | $SO_2$ | H | H | H | - | $CH_3$ | $OCH_3$ | N |
| $J_{32}$ | $SO_2$ | H | H | H | - | - | $OCH_3$ | N |
| $J_{32}$ | $SO_2$ | H | H | H | - | - | $OCH_2CH_3$ | N |
| $J_{32}$ | $SO_2$ | H | H | H | - | - | $OCH_3$ | CH |
| $J_{32}$ | $SO_2$ | H | H | $CH_3$ | - | - | $NHCH_3$ | N |
| $J_{32}$ | $SO_2$ | H | H | $CH_3$ | - | - | $NH(CH_3)_2$ | N |
| $J_{32}$ | $SO_2$ | H | H | $CH_3$ | - | - | $OCH_3$ | N |
| $J_{32}$ | $SO_2$ | H | H | $CH_3$ | - | - | $OCH_2CH_3$ | N |
| $J_{32}$ | $SO_2$ | H | Cl | $CH_3$ | - | - | $OCH_3$ | N |
| $J_{32}$ | $SO_2$ | H | $CH_3$ | $CH_3$ | - | - | $OCH_3$ | N |
| $J_{32}$ | $SO_2$ | H | H | $CH_2CH_3$ | - | - | $OCH_3$ | N |
| $J_{32}$ | $SO_2$ | H | H | $CH(CH_3)_2$ | - | - | $OCH_3$ | N |
| $J_{32}$ | $SO_2$ | H | H | $CH_2CH_2CH_2$ | - | - | $OCH_3$ | N |

Table IV (continued)

wherein W is O; unless *, where W is S.

| J | Q | R | $R_8$ | $R_9$ | $R_{10}$ | $R_{11}$ | Y | Z | |
|---|---|---|---|---|---|---|---|---|---|
| $J_{27}$ | O | H | $CO_2CH_2CH_2CH_3$ | $CH_3$ | H | H | $OCH_3$ | N | |
| $J_{27}$ | O | H | $CO_2CH_2CH{=}CH_2$ | $CH_3$ | H | H | $OCH_3$ | N | |
| $J_{27}$ | O | H | $CO_2CH_2CH_2OCH_3$ | $CH_3$ | H | H | $OCH_3$ | N | |
| $J_{27}$ | O | H | $CO_2CH_2CH_2Cl$ | $CH_3$ | H | H | $OCH_3$ | N | |
| $J_{27}$ | O | H | $SO_2CH_2CH_2CH_3$ | $CH_3$ | H | H | $OCH_3$ | N | |
| $J_{27}$ | O | H | $SO_2CH(CH_3)_2$ | $CH_3$ | H | H | $OCH_3$ | N | |
| $J_{27}$ | O | H | $OSO_2CH_2CH_2CH_3$ | $CH_3$ | H | H | $OCH_3$ | N | |
| $J_{27}$ | O | H | $OSO_2CH(CH_3)_2$ | $CH_3$ | H | H | $OCH_3$ | N | |
| $J_{27}$ | O | H | $SO_2N(CH_3)CH_2CH_3$ | $CH_3$ | H | H | $OCH_3$ | N | |
| $J_{27}$ | O | H | $SO_2N(CH_2CH_3)_2$ | $CH_3$ | H | H | $OCH_3$ | N | |
| $J_{27}$ | O | H | $OSO_2CF_3$ | $CH_3$ | H | H | $OCH_3$ | N | |
| $J_{27}$ | $SO_2$ | H | $CO_2CH_2CH_2CH_3$ | $CH_3$ | H | H | $OCH_3$ | N | |
| $J_{27}$ | $SO_2$ | H | $CO_2CH_2CH{=}CH_2$ | $CH_3$ | H | H | $OCH_3$ | N | |
| $J_{27}$ | $SO_2$ | H | $CO_2CH_2CH_2OCH_3$ | $CH_3$ | H | H | $OCH_3$ | N | |
| $J_{27}$ | $SO_2$ | H | $CO_2CH_2CH_2Cl$ | $CH_3$ | H | H | $OCH_3$ | N | |
| $J_{27}$ | $SO_2$ | H | $SO_2CH_2CH_2CH_3$ | $CH_3$ | H | H | $OCH_3$ | N | |
| $J_{27}$ | $SO_2$ | H | $SO_2CH(CH_3)_2$ | $CH_3$ | H | H | $OCH_3$ | N | |
| $J_{27}$ | $SO_2$ | H | $OSO_2CH_2CH_2CH_3$ | $CH_3$ | H | H | $OCH_3$ | N | |
| $J_{27}$ | $SO_2$ | H | $OSO_2CH(CH_3)_2$ | $CH_3$ | H | H | $OCH_3$ | N | |
| $J_{27}$ | $SO_2$ | H | $SO_2N(CH_3)CH_2CH_3$ | $CH_3$ | H | H | $OCH_3$ | N | |
| $J_{27}$ | $SO_2$ | H | $SO_2N(CH_2CH_3)_2$ | $CH_3$ | H | H | $OCH_3$ | N | |
| $J_{27}$ | $SO_2$ | H | $OSO_2CF_3$ | $CH_3$ | H | H | $OCH_3$ | N | |
| $J_{27}$ | O | H | H | $CH_3$ | H | H | $CH_3$ | N | * |
| $J_{27}$ | O | H | H | $CH_3$ | H | H | $OCH_3$ | CH | * |

Table V

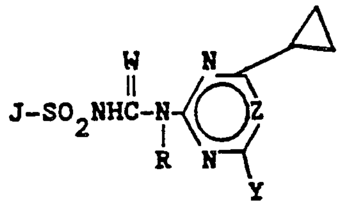

| J | $R_{17}$ | $R_{18}$ | $R_{19}$ | $R_{20}$ | $R_{21}$ | R | W | Q | Y | Z | m.p. (°C) |
|---|---|---|---|---|---|---|---|---|---|---|---|
| $J_{34}$ | H | H | H | H | H | H | O | O | $CH_3$ | CH | |
| $J_{34}$ | H | H | H | H | H | H | O | O | $C_2H_5$ | CH | |
| $J_{34}$ | H | H | H | H | H | H | O | O | $CH_2OCH_3$ | CH | |
| $J_{34}$ | H | H | H | H | H | H | O | O | $CH_2OC_2H_5$ | CH | |
| $J_{34}$ | H | H | H | H | H | H | O | O | $OCH_3$ | CH | |
| $J_{34}$ | H | H | H | H | H | H | O | O | $OC_2H_5$ | CH | |
| $J_{34}$ | H | H | H | H | H | H | O | O | $OCF_2H$ | CH | |
| $J_{34}$ | H | H | H | H | H | H | O | O | $SCF_2H$ | CH | |
| $J_{34}$ | H | H | H | H | H | H | O | O | $OCH_2CF_3$ | CH | |
| $J_{34}$ | H | H | H | H | H | H | O | O | $OCH_2CH_2F$ | CH | |
| $J_{34}$ | H | H | H | H | H | H | O | O | $OCH_2CH{=}CH_2$ | CH | |
| $J_{34}$ | H | H | H | H | H | H | O | O | $OCH_2C{\equiv}CH$ | CH | |
| $J_{34}$ | H | H | H | H | H | H | O | O | $NHCH_3$ | CH | |
| $J_{34}$ | H | H | H | H | H | H | O | O | $N(CH_3)_2$ | CH | |
| $J_{34}$ | H | H | H | H | H | H | O | O | $CH(OCH_3)_2$ | CH | |
| $J_{34}$ | $CH_3$ | H | H | H | H | H | O | O | $CH_3$ | CH | |
| $J_{34}$ | $CH_3$ | H | H | H | H | H | O | O | $C_2H_5$ | CH | |
| $J_{34}$ | $CH_3$ | H | H | H | H | H | O | O | $CH_2OCH_3$ | CH | |
| $J_{34}$ | H | $CH_3$ | H | H | H | H | O | O | $CH_2OC_2H_5$ | CH | |
| $J_{34}$ | H | $CH_3$ | H | H | H | H | O | O | $OCH_3$ | CH | |
| $J_{34}$ | H | $CH_3$ | H | H | H | H | O | O | $OC_2H_5$ | CH | |
| $J_{34}$ | H | $CH_3$ | H | H | H | H | O | O | $OCF_2H$ | CH | |
| $J_{34}$ | $CH_3$ | H | H | H | H | H | O | O | $SCF_2H$ | CH | |

Table V Continued

| J | $R_{17}$ | $R_{18}$ | $R_{19}$ | $R_{20}$ | $R_{21}$ | R | W | Q | Y | Z | m.p. (°C) |
|---|---|---|---|---|---|---|---|---|---|---|---|
| $J_{34}$ | $CH_3$ | H | H | H | H | H | O | O | $OCH_2CF_3$ | CH | |
| $J_{34}$ | $CH_3$ | H | H | H | H | H | O | O | $OCH_2CH_2F$ | CH | |
| $J_{34}$ | $CH_3$ | H | H | H | H | H | O | O | $OCH_2CH=CH_2$ | CH | |
| $J_{34}$ | $CH_3$ | H | H | H | H | H | O | O | $OCH_2C\equiv CH$ | CH | |
| $J_{34}$ | $CH_3$ | H | H | H | H | H | O | O | $NHCH_3$ | CH | |
| $J_{34}$ | $CH_3$ | H | H | H | H | H | O | O | $N(CH_3)_2$ | CH | |
| $J_{34}$ | $CH_3$ | H | H | H | H | H | O | O | $CH(OCH_3)_2$ | CH | |
| $J_{34}$ | $CH_3$ | $CH_3$ | H | H | H | H | O | O | $CH_3$ | CH | |
| $J_{34}$ | $CH_3$ | $CH_3$ | H | H | H | H | O | O | $C_2H_5$ | CH | |
| $J_{34}$ | $CH_3$ | $CH_3$ | H | H | H | H | O | O | $CH_2OCH_3$ | CH | |
| $J_{34}$ | $CH_3$ | $CH_3$ | H | H | H | H | O | O | $OCH_3$ | CH | |
| $J_{34}$ | $CH_3$ | $CH_3$ | H | H | H | H | O | O | $OC_2H_5$ | CH | |
| $J_{34}$ | $CH_3$ | $CH_3$ | H | H | H | H | O | O | $OCF_2H$ | CH | |
| $J_{34}$ | $CH_3$ | $CH_3$ | H | H | H | H | O | O | $OCH_2CF_3$ | CH | |
| $J_{34}$ | $CH_3$ | $CH_3$ | H | H | H | H | O | O | $OCH_2CH_2F$ | CH | |
| $J_{34}$ | $CH_3$ | $CH_3$ | H | H | H | H | O | O | $OCH_2CH=CH_2$ | CH | |
| $J_{34}$ | $CH_3$ | $CH_3$ | H | H | H | H | O | O | $OCH_2C\equiv CH$ | CH | |
| $J_{34}$ | $CH_3$ | $CH_3$ | H | H | H | H | O | O | $NHCH_3$ | CH | |
| $J_{34}$ | $CH_3$ | $CH_3$ | H | H | H | H | O | O | $N(CH_3)_2$ | CH | |
| $J_{34}$ | $CH_3$ | $CH_3$ | H | H | H | H | O | O | $CH(OCH_3)_2$ | CH | |
| $J_{34}$ | $CH_3$ | H | $CH_3$ | H | H | H | O | O | $CH_3$ | CH | |
| $J_{34}$ | H | $CH_3$ | $CH_3$ | H | H | H | O | O | $C_2H_5$ | CH | |
| $J_{34}$ | H | $CH_3$ | $CH_3$ | H | H | H | O | O | $CH_2OCH_3$ | CH | |
| $J_{34}$ | $CH_3$ | H | $CH_3$ | H | H | H | O | O | $OCH_3$ | CH | |
| $J_{34}$ | $CH_3$ | H | $CH_3$ | H | H | H | O | O | $OC_2H_5$ | CH | |
| $J_{34}$ | $CH_3$ | H | $CH_3$ | H | H | H | O | O | $OCF_2H$ | CH | |
| $J_{34}$ | $CH_3$ | H | $CH_3$ | H | H | H | O | O | $OCH_2CF_3$ | CH | |
| $J_{34}$ | $CH_3$ | H | $CH_3$ | H | H | H | O | O | $OCH_2CH_2F$ | CH | |
| $J_{34}$ | $CH_3$ | H | $CH_3$ | H | H | H | O | O | $OCH_2CH=CH_2$ | CH | |
| $J_{34}$ | $CH_3$ | H | $CH_3$ | H | H | H | O | O | $CH(OCH_3)_2$ | CH | |
| $J_{34}$ | $C_2H_5$ | H | H | H | H | H | O | O | $CH_3$ | CH | |
| $J_{34}$ | $n$-$C_3H_7$ | H | H | H | H | H | O | O | $CH_3$ | CH | |

Table V Continued

| J | $R_{17}$ | $R_{18}$ | $R_{19}$ | $R_{20}$ | $R_{21}$ | R | W | Q | Y | Z | m.p. (°C) |
|---|---|---|---|---|---|---|---|---|---|---|---|
| $J_{34}$ | $CH(CH_3)_2$ | H | H | H | H | H | O | O | $CH_3$ | CH | |
| $J_{34}$ | H | H | H | Cl | CH | H | O | O | $CH_3$ | CH | |
| $J_{34}$ | H | H | H | Cl | H | H | O | O | $OCH_3$ | CH | |
| $J_{34}$ | $CH_3$ | H | H | Cl | H | H | O | O | $OCH_3$ | CH | |
| $J_{34}$ | H | H | H | Cl | H | H | O | O | $OC_2H_5$ | CH | |
| $J_{34}$ | H | $CH_3$ | H | Cl | H | H | O | O | $OCF_2H$ | CH | |
| $J_{34}$ | H | H | H | Cl | H | H | O | O | $OCH_2CF_3$ | CH | |
| $J_{34}$ | H | $CH_3$ | H | Cl | H | H | O | O | $OCH_2CH_2F$ | CH | |
| $J_{34}$ | H | H | H | Cl | H | H | O | O | $CH(OCH_3)_2$ | CH | |
| $J_{34}$ | $CH_3$ | H | H | Br | H | H | O | O | $CH_3$ | CH | |
| $J_{34}$ | H | $CH_3$ | H | Br | H | H | O | O | $C_2H_5$ | CH | |
| $J_{34}$ | $CH_3$ | H | H | Br | H | H | O | O | $OCH_3$ | CH | |
| $J_{34}$ | H | H | H | Br | H | H | O | O | $OCH_3$ | CH | |
| $J_{34}$ | H | H | H | Br | H | H | O | O | $OCF_2H$ | CH | |
| $J_{34}$ | $CH_3$ | H | H | Br | H | H | O | O | $OCH_2CF_3$ | CH | |
| $J_{34}$ | $CH_3$ | H | H | Br | H | H | O | O | $OCH_2CH_2F$ | CH | |
| $J_{34}$ | $CH_3$ | H | H | Br | H | H | O | O | $CH(OCH_3)_2$ | CH | |
| $J_{34}$ | $CH_3$ | H | H | $CH_3$ | H | H | O | O | $CH_3$ | CH | |
| $J_{34}$ | $CH_3$ | H | H | $CH_3$ | H | H | O | O | $OCH_3$ | CH | |
| $J_{34}$ | $CH_3$ | H | H | $OCH_3$ | H | H | O | O | $CH_3$ | CH | |
| $J_{34}$ | $CH_3$ | H | H | $OCH_3$ | H | H | O | O | $OCH_3$ | CH | |
| $J_{34}$ | H | $CH_3$ | H | $CF_3$ | H | H | O | O | $OCH_3$ | CH | |
| $J_{34}$ | H | $CH_3$ | H | $CF_3$ | H | H | O | O | $CH_3$ | CH | |
| $J_{34}$ | H | H | H | H | H | H | O | $SO_2$ | $CH_3$ | CH | |
| $J_{34}$ | H | H | H | H | H | H | O | $SO_2$ | $C_2H_5$ | CH | |
| $J_{34}$ | H | H | H | H | H | H | O | $SO_2$ | $OCH_3$ | CH | |
| $J_{34}$ | H | H | H | H | H | H | O | $SO_2$ | $OC_2H_5$ | CH | |
| $J_{34}$ | H | H | H | H | H | H | O | $SO_2$ | $OCF_2H$ | CH | |
| $J_{34}$ | H | H | H | H | H | H | O | $SO_2$ | $OCH_2CF_3$ | CH | |
| $J_{34}$ | H | H | H | H | H | H | O | $SO_2$ | $OCH_2CH_2F$ | CH | |
| $J_{34}$ | $CH_3$ | H | H | H | H | H | O | $SO_2$ | $CH_3$ | CH | |

Table V Continued

| J | $R_{17}$ | $R_{18}$ | $R_{19}$ | $R_{20}$ | $R_{21}$ | R | W | Q | Y | Z | m.p. (°C) |
|---|---|---|---|---|---|---|---|---|---|---|---|
| $J_{34}$ | $CH_3$ | H | H | H | H | H | O | $SO_2$ | $C_2H_5$ | CH | |
| $J_{34}$ | $CH_3$ | H | H | H | H | H | O | $SO_2$ | $CH_2OCH_3$ | CH | |
| $J_{34}$ | $CH_3$ | H | H | H | H | H | O | $SO_2$ | $CH_2OC_2H_5$ | CH | |
| $J_{34}$ | $CH_3$ | H | H | H | H | H | O | $SO_2$ | $OCH_3$ | CH | |
| $J_{34}$ | $CH_3$ | H | H | H | H | H | O | $SO_2$ | $OC_2H_5$ | CH | |
| $J_{34}$ | $CH_3$ | H | H | H | H | H | O | $SO_2$ | $OCF_2H$ | CH | |
| $J_{34}$ | H | $CH_3$ | H | H | H | H | O | $SO_2$ | $SCF_2H$ | CH | |
| $J_{34}$ | H | $CH_3$ | H | H | H | H | O | $SO_2$ | $OCH_2CF_3$ | CH | |
| $J_{34}$ | H | $CH_3$ | H | H | H | H | O | $SO_2$ | $OCH_2CH_2F$ | CH | |
| $J_{34}$ | H | $CH_3$ | H | H | H | H | O | $SO_2$ | $OCH_2CH=CH_2$ | CH | |
| $J_{34}$ | H | $CH_3$ | H | H | H | H | O | $SO_2$ | $OCH_2C\equiv CH$ | CH | |
| $J_{34}$ | H | $CH_3$ | H | H | H | H | O | $SO_2$ | $NHCH_3$ | CH | |
| $J_{34}$ | H | $CH_3$ | H | H | H | H | O | $SO_2$ | $N(CH_3)_2$ | CH | |
| $J_{34}$ | H | $CH_3$ | H | H | H | H | O | $SO_2$ | $CH(OCH_3)_2$ | CH | |
| $J_{34}$ | $CH_3$ | $CH_3$ | H | H | H | H | O | $SO_2$ | $CH_3$ | CH | |
| $J_{34}$ | $CH_3$ | $CH_3$ | H | H | H | H | O | $SO_2$ | $OCH_3$ | CH | |
| $J_{34}$ | $CH_3$ | $CH_3$ | H | H | H | H | O | $SO_2$ | $OC_2H_5$ | CH | |
| $J_{34}$ | $CH_3$ | $CH_3$ | H | H | H | H | O | $SO_2$ | $OCF_2H$ | CH | |
| $J_{34}$ | $CH_3$ | $CH_3$ | H | H | H | H | O | $SO_2$ | $C_2H_5$ | CH | |
| $J_{34}$ | $C_2H_5$ | H | H | H | H | H | O | $SO_2$ | $CH_3$ | CH | |
| $J_{34}$ | $n-C_3H_7$ | H | H | H | H | H | O | $SO_2$ | $CH_3$ | CH | |
| $J_{34}$ | H | H | $CH_3$ | H | H | H | O | $SO_2$ | $CH_3$ | CH | |
| $J_{34}$ | H | H | H | H | H | H | O | S | $CH_3$ | CH | |
| $J_{34}$ | $CH_3$ | H | H | H | H | H | O | S | $OCH_3$ | CH | |
| $J_{34}$ | $CH_3$ | H | H | H | H | H | O | S | $CH_3$ | CH | |
| $J_{34}$ | $CH_3$ | $CH_3$ | H | H | H | H | O | S | $OCH_3$ | CH | |
| $J_{34}$ | $CH_3$ | $CH_3$ | H | H | H | H | O | S | $CH_3$ | CH | |
| $J_{34}$ | $C_2H_5$ | H | H | H | H | H | O | S | $CH_3$ | CH | |
| $J_{34}$ | $n-C_3H_7$ | H | H | H | H | H | O | S | $CH_3$ | CH | |
| $J_{34}$ | $CH_3$ | H | $CH_3$ | H | H | H | O | S | $CH_3$ | CH | |
| $J_{34}$ | $CH_3$ | H | H | $CH_3$ | H | H | O | $SO_2$ | $CH_3$ | CH | |

Table V Continued

| J | $R_{17}$ | $R_{18}$ | $R_{19}$ | $R_{20}$ | $R_{21}$ | R | W | Q | Y | Z | m.p. (°C) |
|---|---|---|---|---|---|---|---|---|---|---|---|
| $J_{34}$ | $CH_3$ | H | H | H | H | $CH_3$ | O | O | $CH_3$ | CH | |
| $J_{34}$ | $CH_3$ | H | H | H | H | $CH_3$ | O | $SO_2$ | $CH_3$ | CH | |
| $J_{34}$ | $CH_3$ | H | H | H | H | H | S | O | $CH_3$ | CH | |
| $J_{34}$ | $CH_3$ | H | H | H | H | H | S | $SO_2$ | $CH_3$ | CH | |
| $J_{34}$ | $CH_3$ | H | H | H | $CH_3$ | H | O | $SO_2$ | $CH_3$ | CH | |
| $J_{34}$ | $CH_3$ | H | H | H | $OCH_3$ | H | O | O | $OCH_3$ | CH | |
| $J_{34}$ | $CH_3$ | $CH_3$ | H | H | Cl | H | O | S | $OCH_3$ | CH | |
| $J_{34}$ | $CH_3$ | H | H | H | Br | H | O | O | $OCH_3$ | N | |
| $J_{34}$ | $CH_3$ | H | H | H | $NO_2$ | H | O | O | $CH_3$ | CH | |
| $J_{34}$ | $CH_3$ | H | H | H | $CO_2CH_3$ | H | O | O | $OC_2H_5$ | N | |
| $J_{34}$ | $CH_3$ | H | H | H | $CO_2C_2H_5$ | H | O | $SO_2$ | $CH_3$ | CH | |
| $J_{34}$ | $CH_3$ | H | H | H | $CO_2CH_2CH_2CH_3$ | H | O | O | $CH_3$ | CH | |
| $J_{34}$ | $CH_3$ | $CH_3$ | H | H | $CO_2CH(CH_3)_2$ | H | O | S | $OCH_3$ | CH | |
| $J_{34}$ | $CH_3$ | H | H | H | $CO_2CH_2CH{=}CH_2$ | H | O | O | $CH_3$ | CH | |
| $J_{34}$ | $CH_3$ | H | H | H | $CO_2CH_2CH_2OCH_3$ | H | O | O | $CH_3$ | CH | |
| $J_{34}$ | $CH_3$ | H | H | H | $CO_2CH_2CH_2Cl$ | H | O | O | $OCH_3$ | CH | |
| $J_{34}$ | $CH_3$ | $CH_3$ | H | H | $SO_2CH_3$ | H | O | O | $OCF_2H$ | N | |
| $J_{34}$ | $CH_3$ | $CH_3$ | H | H | $SO_2C_2H_5$ | H | O | O | $CH_3$ | CH | |
| $J_{34}$ | $CH_3$ | H | H | H | $SO_2CH_2CH_2CH_3$ | H | O | O | $OCH_3$ | CH | |
| $J_{34}$ | $CH_3$ | H | H | H | $SO_2CH_2(CH_3)_2$ | H | O | O | $CH_3$ | CH | |
| $J_{34}$ | H | $CH_3$ | H | H | $OSO_2CH_3$ | H | O | $SO_2$ | $CH_3$ | CH | |
| $J_{34}$ | $CH_3$ | H | H | H | $OSO_2C_2H_5$ | H | O | O | $CH_3$ | CH | |
| $J_{34}$ | $CH_3$ | H | H | H | $OSO_2CH_2CH_2CH_3$ | H | O | O | $CH_3$ | CH | |
| $J_{34}$ | $CH_3$ | H | H | H | $OSO_2CH(CH_3)_2$ | H | O | S | $CH_3$ | CH | |
| $J_{34}$ | $CH_3$ | $CH_3$ | H | H | $OSO_2CF_3$ | H | O | O | $OCH_3$ | N | |
| $J_{34}$ | $CH_3$ | H | H | H | $SO_2N(CH_3)_2$ | H | O | O | $CH_3$ | CH | |
| $J_{34}$ | $CH_3$ | H | H | H | $SO_2N(CH_3)C_2H_5$ | H | O | O | $CH_3$ | CH | |
| $J_{34}$ | H | H | H | H | H | H | O | O | $CH_3$ | N | |
| $J_{34}$ | H | H | H | H | H | H | O | O | $OCH_3$ | N | |
| $J_{34}$ | H | H | H | H | H | H | O | O | $OC_2H_5$ | N | |
| $J_{34}$ | H | H | H | H | H | H | O | O | $OCF_2H$ | N | |

Table V Continued

| J | $R_{17}$ | $R_{18}$ | $R_{19}$ | $R_{20}$ | $R_{21}$ | R | W | Q | Y | Z | m.p. (°C) |
|---|---|---|---|---|---|---|---|---|---|---|---|
| $J_{34}$ | H | H | H | H | H | H | O | O | $SCF_2H$ | N | |
| $J_{34}$ | H | H | H | H | H | H | O | O | $OCH_2CF_3$ | N | |
| $J_{34}$ | H | H | H | H | H | H | O | O | $OCH_2CH_2F$ | N | |
| $J_{34}$ | H | H | H | H | H | H | O | O | $OCH_2CH=CH_2$ | N | |
| $J_{34}$ | H | H | H | H | H | H | O | O | $OCH_2C{\equiv}CH$ | N | |
| $J_{34}$ | $CH_3$ | H | H | H | $SO_2N(OCH_3)CH_3$ | H | O | O | $OCH_3$ | CH | |
| $J_{34}$ | H | H | H | H | H | H | O | O | $NHCH_3$ | N | |
| $J_{34}$ | H | H | H | H | H | H | O | O | $NH(CH_3)_2$ | N | |
| $J_{34}$ | H | H | H | H | H | H | O | O | $CH(OCH_3)_2$ | N | |
| $J_{34}$ | $CH_3$ | H | H | H | H | H | O | O | $OCH_3$ | N | |
| $J_{34}$ | $CH_3$ | H | H | H | H | H | O | O | $OCH_2H_5$ | N | |
| $J_{34}$ | $CH_3$ | H | H | H | H | H | O | O | $OCF_2H$ | N | |
| $J_{34}$ | H | $CH_3$ | H | H | H | H | O | O | $OCH_2CF_3$ | N | |
| $J_{34}$ | H | $CH_3$ | H | H | H | H | O | O | $OCH_2CH_2F$ | N | |
| $J_{34}$ | H | $CH_3$ | H | H | H | H | O | O | $OCH_2CH=CH_2$ | N | |
| $J_{34}$ | $CH_3$ | CH | H | H | H | H | O | O | $OCH_3$ | N | |
| $J_{34}$ | $CH_3$ | $CH_3$ | H | H | H | H | O | O | $OC_2H_5$ | N | |
| $J_{34}$ | $CH_3$ | H | $CH_3$ | H | H | H | O | O | $OCH_3$ | N | |
| $J_{34}$ | H | $CH_3$ | $CH_3$ | H | H | H | O | O | $OC_2H_5$ | N | |
| $J_{34}$ | $C_2H_5$ | H | H | H | H | H | O | $SO_2$ | $OCH_3$ | N | |
| $J_{34}$ | $CH(CH_3)_2$ | H | H | H | H | H | O | O | $OCH_3$ | N | |
| $J_{34}$ | *n*-propyl | H | H | H | H | H | O | O | $OCH_3$ | N | |
| $J_{34}$ | $CH_3$ | H | H | Cl | H | H | O | O | $OCH_3$ | N | |
| $J_{34}$ | H | H | H | Cl | H | H | O | O | $OC_2H_5$ | N | |
| $J_{34}$ | $CH_3$ | H | H | Br | H | H | O | O | $OCH_3$ | N | |
| $J_{34}$ | H | H | H | Cl | H | H | O | O | $OCH_3$ | N | |
| $J_{34}$ | $CH_3$ | H | H | $CH_3$ | H | H | O | O | $OCH_3$ | N | |
| $J_{34}$ | $CH_3$ | H | H | $OCH_3$ | H | H | O | O | $OCH_3$ | N | |
| $J_{34}$ | $CH_3$ | H | H | $CF_3$ | H | H | O | O | $OCH_3$ | N | |
| $J_{34}$ | H | H | H | H | H | H | O | $SO_2$ | $OCH_3$ | N | 234-236(d) |

Table V Continued

| J | $R_{17}$ | $R_{18}$ | $R_{19}$ | $R_{20}$ | $R_{21}$ | R | W | Q | Y | Z | m.p. (°C) |
|---|---|---|---|---|---|---|---|---|---|---|---|
| $J_{34}$ | H | H | H | H | H | H | O | $SO_2$ | $OC_2H_5$ | N | |
| $J_{34}$ | $CH_3$ | H | H | H | H | H | O | $SO_2$ | $CH_3$ | N | |
| $J_{34}$ | $CH_3$ | H | H | H | H | H | O | $SO_2$ | $OCH_3$ | N | 205-210(d) |
| $J_{34}$ | $CH_3$ | H | H | H | H | H | O | $SO_2$ | $OC_2H_5$ | N | |
| $J_{34}$ | $CH_3$ | H | H | H | H | H | O | $SO_2$ | $C_2H_5$ | N | |
| $J_{34}$ | $CH_3$ | H | H | H | H | H | O | $SO_2$ | $OCF_2H$ | N | |
| $J_{34}$ | $CH_3$ | H | H | H | H | H | O | $SO_2$ | $SCF_2H$ | N | |
| $J_{34}$ | H | $CH_3$ | H | H | H | H | O | $SO_2$ | $OCH_2CF_3$ | N | |
| $J_{34}$ | H | $CH_3$ | H | H | H | H | O | $SO_2$ | $OCH_2CH_2F$ | N | |
| $J_{34}$ | H | $CH_3$ | H | H | H | H | O | $SO_2$ | $OCH_2CH=CH_2$ | N | |
| $J_{34}$ | H | $CH_3$ | H | H | H | H | O | $SO_2$ | $OCH_2C\equiv CH$ | N | |
| $J_{34}$ | H | $CH_3$ | H | H | H | H | O | $SO_2$ | $NHCH_3$ | N | |
| $J_{34}$ | H | $CH_3$ | H | H | H | H | O | $SO_2$ | $N(CH_3)_2$ | N | |
| $J_{34}$ | $CH_3$ | $CH_3$ | H | H | H | H | O | $SO_2$ | $OCH_3$ | N | |
| $J_{34}$ | $C_2H_5$ | H | H | H | H | H | O | $SO_2$ | $OCH_3$ | N | |
| $J_{34}$ | *n*-$C_3H_7$ | H | H | H | H | H | O | $SO_2$ | $OCH_3$ | N | |
| $J_{34}$ | H | H | $CH_3$ | H | H | H | O | $SO_2$ | $OCH_3$ | N | |
| $J_{34}$ | H | H | H | H | H | H | O | S | $OCH_3$ | N | |
| $J_{34}$ | $CH_3$ | H | H | H | H | H | O | S | $OCH_3$ | N | |
| $J_{34}$ | $CH_3$ | $CH_3$ | H | H | H | H | O | S | $OCH_3$ | N | |
| $J_{34}$ | $C_2H_5$ | H | H | H | H | H | O | S | $OCH_3$ | N | |
| $J_{34}$ | *n*-$C_3H_7$ | H | H | H | H | H | O | S | $OCH_3$ | N | |
| $J_{34}$ | $CH_3$ | H | H | $CH_3$ | H | H | O | $SO_2$ | $OCH_3$ | N | |
| $J_{34}$ | $CH_3$ | H | H | H | H | $CH_3$ | O | $SO_2$ | $OCH_3$ | N | |
| $J_{34}$ | $CH_3$ | H | H | H | Cl | H | O | $SO_2$ | $OCH_3$ | N | |
| $J_{34}$ | $CH_3$ | H | H | H | $CH_3$ | H | O | $SO_2$ | $OCH_3$ | N | |
| $J_{34}$ | $CH_3$ | H | H | H | H | H | S | O | $OCH_3$ | N | |
| $J_{34}$ | $CH_3$ | H | H | H | Cl | H | O | $SO_2$ | $OC_2H_5$ | N | |
| $J_{34}$ | $CH_3$ | H | H | H | $CH_3$ | H | O | $SO_2$ | $OC_2H_5$ | N | |
| $J_{34}$ | $CH_3$ | H | H | H | H | H | O | SO | $OCH_3$ | CH | |
| $J_{34}$ | *n*-butyl | H | H | H | H | H | O | $SO_2$ | $OCH_3$ | N | |

Table V Continued

| J | $R_{17}$ | $R_{18}$ | $R_{19}$ | $R_{20}$ | $R_{21}$ | R | W | Q | Y | Z | m.p. (°C) |
|---|---|---|---|---|---|---|---|---|---|---|---|
| $J_{34}$ | H | $C_2H_5$ | H | H | H | H | O | O | $OCH_3$ | N | |
| $J_{34}$ | H | n-propyl | H | H | H | H | O | $SO_2$ | $OCH_3$ | N | |
| $J_{34}$ | H | $CH(CH_3)_2$ | H | H | H | H | O | $SO_2$ | $OCH_3$ | N | |
| $J_{34}$ | H | n-butyl | H | H | H | H | O | O | $OCH_3$ | N | |
| $J_{34}$ | $CH_3$ | H | H | F | H | H | O | O | $OCH_3$ | N | |
| $J_{34}$ | $CH_3$ | H | H | $SCH_3$ | H | H | O | O | $OCH_3$ | N | |
| $J_{34}$ | $CH_3$ | H | H | $OCF_2H$ | H | H | O | O | $OCH_3$ | N | |
| $J_{34}$ | $CH_3$ | H | H | H | F | H | O | O | $OCH_3$ | N | |
| $J_{34}$ | $CH_3$ | H | H | H | $CFH_3$ | H | O | O | $OCH_3$ | N | |
| $J_{34}$ | $CH_3$ | H | H | H | $SCH_3$ | H | O | O | $OCH_3$ | N | |
| $J_{34}$ | $CH_3$ | H | H | H | $OCF_2H$ | H | O | O | $OCH_3$ | N | |
| $J_{34}$ | $CH_3$ | H | H | H | H | H | O | O | $CF_3$ | N | |
| $J_{34}$ | H | H | H | Br | H | H | O | O | $OCH_3$ | N | |
| $J_{34}$ | $CH_3$ | H | H | H | Cl | H | O | $SO_2$ | $OCH_3$ | N | |

Table VI

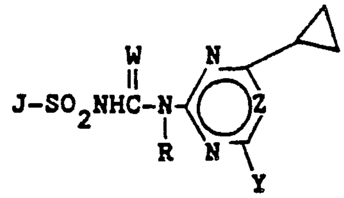

| J | $R_{17}$ | $R_{18}$ | $R_{19}$ | $R_{20}$ | $R_{21}$ | R | W | Q | Y | Z | m.p. (°C) |
|---|---|---|---|---|---|---|---|---|---|---|---|
| $J_{35}$ | H | H | H | H | H | H | O | O | $OCH_3$ | CH | |
| $J_{35}$ | H | H | H | H | H | H | O | O | $OCH_3$ | N | |
| $J_{35}$ | $CH_3$ | H | $CH_3$ | H | H | H | O | $SO_2$ | $OCH_3$ | CH | |
| $J_{35}$ | H | H | H | H | H | H | O | S | $OCH_3$ | CH | |
| $J_{35}$ | H | H | H | H | H | H | S | O | $OCH_3$ | CH | |
| $J_{35}$ | H | $CH_3$ | $CH_3$ | H | H | H | O | $SO_2$ | $OCH_3$ | N | |
| $J_{35}$ | H | H | H | H | $CH_3$ | H | O | O | $OCH_3$ | CH | |

| J | $R_{20}$ | $R_{21}$ | $R_{22}$ | $R_{23}$ | R | W | $Q_1$ | Y | Z | m.p. (°C) |
|---|---|---|---|---|---|---|---|---|---|---|
| $J_{36}$ | H | H | H | H | H | O | O | $OCH_3$ | CH | |
| $J_{36}$ | H | H | H | H | H | O | O | $OCH_3$ | N | |
| $J_{36}$ | H | H | $CH_3$ | H | H | O | O | $OCH_3$ | CH | |
| $J_{36}$ | H | H | $CH_3$ | H | H | O | O | $OCH_3$ | N | |
| $J_{36}$ | H | H | $CH_3$ | H | H | O | O | $OC_2H_5$ | N | |
| $J_{36}$ | H | H | $CH_3$ | $CH_3$ | H | O | $SO_2$ | $OCH_3$ | CH | |
| $J_{36}$ | H | H | $C_2H_5$ | H | H | O | O | $OCH_3$ | CH | |
| $J_{36}$ | H | H | $n-C_3H_7$ | H | H | O | O | $OC_2H_5$ | N | |
| $J_{36}$ | H | H | Cl | $CH_3$ | H | O | O | $OCH_3$ | CH | |
| $J_{36}$ | H | H | Br | $CH_3$ | H | O | O | $OCH_3$ | CH | |
| $J_{36}$ | H | H | $CH_3$ | Cl | H | O | O | $OCH_3$ | N | |
| $J_{36}$ | H | H | $CH_3$ | Br | H | O | O | $OCH_3$ | CH | |
| $J_{36}$ | H | H | $CH_3$ | $CH_3$ | H | O | S | $OCH_3$ | CH | |
| $J_{36}$ | Cl | H | $CH_3$ | H | H | O | O | $OCH_3$ | CH | |
| $J_{36}$ | $CH_3$ | H | $CH_3$ | H | H | O | O | $OCH_3$ | CH | |
| $J_{36}$ | H | Cl | $CH_3$ | H | H | O | O | $OCH_3$ | CH | |
| $J_{36}$ | H | H | n-butyl | H | H | O | O | $OCH_3$ | CH | |

Table VI (continued)

| J | $R_{21}$ | $R_{22}$ | $R_{23}$ | R | W | $Q_1$ | Y | Z | m.p. (°C) |
|---|---|---|---|---|---|---|---|---|---|
| $J_{37}$ | H | H | $CH_3$ | H | O | O | $OCH_3$ | CH | |
| $J_{37}$ | H | $CH_3$ | $CH_3$ | H | O | O | $OCH_3$ | CH | |
| $J_{37}$ | H | H | H | H | O | S | $OCH_3$ | CH | |
| $J_{37}$ | H | H | $CH_3$ | H | O | $SO_2$ | $OCH_3$ | CH | |
| $J_{37}$ | H | H | $CH_3$ | H | O | O | $OCH_3$ | N | |
| $J_{37}$ | H | H | $CH_3$ | H | O | $SO_2$ | $OCH_3$ | N | |

Formulations

Useful formulations of the compounds of Formula I can be prepared in conventional ways. They include dusts, granules, pellets, solutions, suspensions, emulsions, wettable powders, emulsifiable concentrates and the like. Many of these may be applied directly. Sprayable formulations can be extended in suitable media and used at spray volumes of from a few liters to several hundred liters per hectare. High strength compositions are primarily used as intermediates for further formulation. The formulations, broadly, contain about 0.1% to 99% by weight of active ingredient(s) and at least one of (a) about 0.1% to 20% surfactant(s) and (b) about 1% to 99.9% solid or liquid inert diluent(s). More specifically, they will contain these ingredients in the following approximate proportions:

Table VII

| | Active Ingredient | Weight Percent* Diluent(s) | Surfactant(s) |
|---|---|---|---|
| Wettable Powders | 20-90 | 0-74 | 1-10 |
| Oil Suspensions, Emulsions, Solutions, (including Emulsifiable Concentrates) | 3-50 | 40-95 | 0-15 |
| Aqueous Suspension | 10-50 | 40-84 | 1-20 |
| Dusts | 1-25 | 70-99 | 0-5 |
| Granules and Pellets | 0.1-95 | 5-99.9 | 0-15 |
| High Strength Compositions | 90-99 | 0-10 | 0-2 |

* Active ingredient plus at least one of a Surfactant or a Diluent equals 100 weight percent.

Lower or higher levels of active ingredient can, of course, be present depending on the intended use and the physical properties of the compound. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carriers", 2nd Ed., Dorland Books, Caldwell, New Jersey, but other solids, either mined or manufactured, may be used. The more absorptive diluents are preferred for wettable powders and the denser ones for dusts. Typical liquid diluents and solvents are described in Marsden, "Solvents Guide," 2nd Ed., Interscience, New York, 1950. Solubility under 0.1% is preferred for suspension concentrates: solution concentrates are preferably stable against phase separation at 0°C. "McCutcheon's Detergents and Emulsifiers Annual", MC Publishing Corp., Ridgewood, New Jersey, as well as Sisely and Wood, "Encyclopedia of Surface Active Agents", Chemical Publishing Co., Inc., New York, 1964, list surfactants

and recommended uses. All formulations can contain minor amounts of additives to reduce foaming, caking, corrosion, microbiological growth, etc.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", *Chemical Engineering*, December 4, 1967, pp. 147ff. and "Perry's Chemical Engineer's Handbook", 5th Ed., McGraw-Hill, New York, 1973, pp. 8—57ff.

For further information regarding the art of formulation, see for example:

H. M. Loux, U.S. Patent 3,235,361, February 15, 1966, Col. 6, line 16 through Col. 7, line 19 and Examples 10 through 41;

R. W. Luckenbaugh, U.S. Patent 3,309,192, March 14, 1967, Col. 5, line 43 through Col. 7, line 62 and Examples 8, 12, 15, 39, 41, 52, 53, 58, 132, 138—140, 162—164, 166, 167 and 169—182;

H. Gysin and E. Knusli, U.S. Patent 2,891,855, June 23, 1959, Col. 3, line 66 through Col. 5, line 17 and Examples 1—4;

G. C. Klingman, "Weed Control as a Science", John Wiley & Sons, Inc., New York, 1961, pp. 81—96; and

J. D. Fryer and S. A. Evans, "Weed Control Handbook", 5th Ed., Blackwell Scientific Publications, Oxford, 1968, pp. 101—103.

In the following examples, all parts are by weight unless otherwise indicated.

Example 2

Wettable Powder

| | |
|---|---|
| N-[(4-cyclopropyl-6-methoxy-1,3,5-triazin-2-yl)aminocarbonyl]-3,4-dihydro-2H-1-benzothiopyran-8-sulfonamide, 1,1-dioxide | 80% |
| sodium alkylnaphthalenesulfonate | 2% |
| sodium ligninsulfonate | 2% |
| synthetic amorphous silica | 3% |
| kaolinite | 13% |

The ingredients are blended, hammer-milled until all the solids are essentially under 50 microns, reblended, and packaged.

Example 3

Wettable Powder

| | |
|---|---|
| N-[(4-cyclopropyl-6-methoxy-1,3,5-triazin-2-yl)aminocarbonyl]-3,4-dihydro-2H-1-benzothiopyran-8-sulfonamide, 1,1-dioxide | 50% |
| sodium alkylnaphthalenesulfonate | 3% |
| low viscosity methyl cellulose | 2% |
| diatomaceous earth | 46% |

The ingredients are blended, coarsely hammer-milled and then air-milled to produce particles essentially all below 10 microns in diameter. The product is reblended before packaging.

Example 4

Granule

| | |
|---|---|
| Wettable Powder of Example 3 | 5% |
| attapulgite granules (U.S.S. 20—40 mesh; 0.84—0.42 mm) | 95% |

A slurry of wettable powder containing 25% solids is sprayed on the surface of attapulgite granules in a double-cone blender. The granules are dried and packages.

Example 5

Extruded Pellet

| | |
|---|---|
| N-[(4-cyclopropyl-6-methoxy-1,3,5-triazin-2-yl)aminocarbonyl]-3,4-dihydro-2H-1-benzothiopyran-8-sulfonamide, 1,1-dioxide | 25% |
| anhydrous sodium sulfate | 10% |
| crude calcium ligninsulfonate | 5% |
| sodium alkylnaphthalenesulfonate | 1% |
| calcium/magnesium bentonite | 59% |

The ingredients are blended, hammer-milled and then moistened with about 12% water. The mixture is extruded as cylinders about 3 mm diameter which are cut to produce pellets about 3 mm long. These may be used directly after drying, or the dried pellets may be crushed to pass a U.S.S. No. 20 sieve (0.84 mm openings). The granules held on a U.S.S. No. 40 shieve (0.42 mm openings) may be packaged for use and the fines recycled.

Example 6

Oil Suspension

| | |
|---|---|
| N-[(4-cyclopropyl-6-methoxy-1,3,5-triazin-2-yl)aminocarbonyl]-3,4-dihydro-2H-1-benzothiopyran-8-sulfonamide, 1,1-dioxide | 25% |
| polyoxyethylene sorbitol hexaoleate | 5% |
| highly aliphatic hydrocarbon oil | 70% |

The ingredients are ground together in a sand mill until the solid particles have been reduced to under about 5 microns. The resulting thick suspension may be applied directly, but preferably after being extended with oils or emulsified in water.

Example 7

Wettable Powder

| | |
|---|---|
| N-[(4-cyclopropyl-6-methoxy-1,3,5-triazin-2-yl)-aminocarbonyl]-2,3-dihydro-2-methyl-1,2-benzisothiazole-7-sulfonamide, 1,1-dioxide | 80% |
| sodium alkylnaphthalenesulfonate | 2% |
| sodium ligninsulfonate | 2% |
| synthetic amorphous silica | 3% |
| kaolinite | 13% |

The ingredients are blended, hammer-milled until all the solids are essentially under 50 microns, reblended, and packaged.

Example 8

Low Strength Granule

| | |
|---|---|
| N-[(4-cyclopropyl-6-methoxy-1,3,5-triazin-2-yl)-aminocarbonyl]-2,3-dihydro-2-methyl-1,2-benzisothiazole-7-sulfonamide, 1,1-dioxide | 1% |
| N,N-dimethylformamide | 9% |
| attapulgite granules (U.S.S. 20—40 sieve) | 90% |

The active ingredient is dissolved in the solvent and the solution is sprayed upon dedusted granules in a double cone blender. After spraying of the solution has been completed, the blender is allowed to run for a short period and then the granules are packaged.

Example 9

Aqueous Suspension

| | |
|---|---|
| N-[(4-cyclopropyl-6-methoxy-1,3,5-triazin-2-yl)-aminocarbonyl]-2,3-dihydro-2-methyl-1,2-benzisothiazole-7-sulfonamide, 1,1-dioxide | 40% |
| polyacrylic acid thickener | 0.3% |
| dodecylphenol polethylene gycol ether | 0.5% |
| disodium phosphate | 1% |
| monosodium phosphate | 0.5% |
| polyvinyl alcohol | 1.0% |
| water | 56.7% |

The ingredients are blended and ground together in a sand mill to produce particles essentially all under 5 microns in size.

Example 10

Solution

| | |
|---|---|
| N-[(4-cyclopropyl-6-methoxy-1,3,5-triazin-2-yl)aminocarbonyl]-2,3-dihydro-2-methyl-1,2-benzisothiazole-7-sulfonamide, 1,1-dioxide sodium salt | 5% |
| water | 95% |

The salt is added directly to the water with stirring to produce the solution, which may then be packaged for use.

Example 11

High Strength Concentrate

| | |
|---|---|
| N-[(4-cyclopropyl-6-methoxy-1,3,5-triazin-2-yl)aminocarbonyl]-2,3-dihydro-2-methyl-1,2-benzisothiazole-7-sulfonamide, 1,1-dioxide sodium salt | 99% |
| silica aerogel | 0.5% |
| synthetic amorphous silica | 0.5% |

The ingredients are blended and ground in a hammer-mill to produce a material essentially all passing a U.S.S. No. 50 screen (0.3 mm opening). The concentrate may be formulated further if necessary.

Example 12

Oil Suspension

| | |
|---|---|
| N-[(4-cyclopropyl-6-methylpyrimidin-2-yl)aminocarbonyl]2,3-dihydro-2-2-dimethyl-7-benzofuransulfonamide | 25% |
| polyoxyethylene sorbitol hexaoleate | 5% |
| highly aliphatic hydrocarbon oil | 70% |

The ingredients are ground together in a sand mill until the solid particles have been reduced to under about 5 microns. The resulting thick suspension may be applied directly, but preferably after being extended with oils or emulsified in water.

Example 13

| | |
|---|---|
| Low Strength Granule | |
| N-[(4-cyclopropyl-6-methoxy-1,3,5-triazin-2-yl)aminocarbonyl]-2,3-dihydro-7-benzothiophenesulfonamide | 0.1% |
| attapulgite granules (U.S.S. 20—40 mesh) | 99.9% |

The active ingredient is dissolved in a solvent and the solution is sprayed upon dedusted granules in a double-cone blender. After spraying of the solution has been completed, the material is warmed to evaporate the solvent. The material is allowed to cool and then packaged.

Example 14

| | |
|---|---|
| Granule | |
| N-[(4-cyclopropyl-6-methoxy-1,3,5-triazin-2-yl)aminocarbonyl]-2,3-dihydro-2,2-dimethyl-7-benzothiophenesulfonamide | 80% |
| wetting agent | 1% |
| crude ligninsulfonate salt (containing 5—20% of the natural sugars) | 10% |
| attapulgite clay | 9% |

The ingredients are blended and milled to pass through a 100 mesh screen. This material is then added to a fluid bed granulator, the air flow is adjusted to gently fluidize the material, and a fine spray of water is sprayed onto the fluidized material. The fluidization and spraying are continued until granules of the desired size range are made. The spraying is stopped, but fluidization is continued, optionally with heat, until the water content is reduced to the desired level, generally less than 1%. The material is then discharged, screened to the desired size range, generally 14—100 mesh (1410—149 microns), and packaged for use.

Example 15

| | |
|---|---|
| High Strength Concentrate | |
| N-[(4-cyclopropyl-6-methoxy-1,3,5-triazin-2-yl)aminocarbonyl]-2,3-dihydro-7-benzothiophenesulfonamide-1,1-dioxide | 99% |
| silica aerogel | 0.5% |
| synthetic amorphous silica | 0.5% |

The ingredients are blended and ground in a hammer-mill to produce a material essentially all passing a U.S.S. No. 50 screen (0.3 mm opening). The concentrate may be formulated further if necessary.

Utility

The compounds of the present invention are effective herbicides. They have utility for broad-spectrum pre- and/or post-emergence weed control in areas where complete control of all vegetation is desired, such as around fuel storage tanks, ammunition depots, industrial storage areas, parking lots, drive-in theaters, around billboards, highway and railroad structures, or on fallow land.

The rates of application for the compounds of the invention are determined by a number of factors, including the types of weeds to be controlled, weather and climate, formulations selected, mode of application, amount of foliage present, etc. In general terms, the subject compounds should be applied at levels of around 0.005 to 5 kg/ha, the lower rates being suggested for use on lighter soils and/or those having a low organic matter content, or for situations where only short-term persistence is required.

The compounds of the invention may be used in combination with any other commercial herbicide examples of which are those of the triazine, triazole, uracil, urea, amide, diphenylether, carbamate, acetanilide, bipyridylium, dinitroaniline and phenolic types.

The herbicidal properties of the subject compounds were observed in greenhouse tests. The test procedures follow.

Compounds

Compound 1

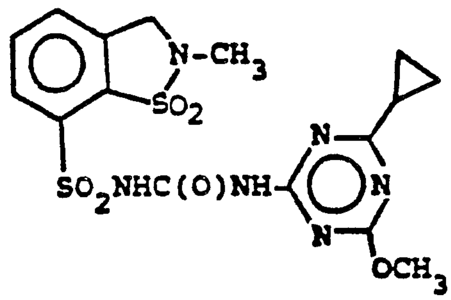

Compound 2

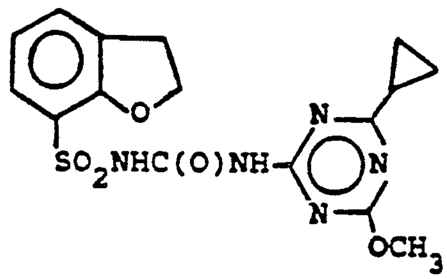

Compound 3

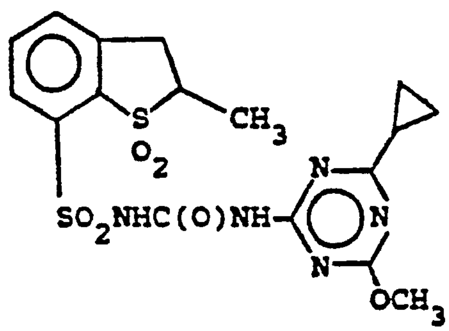

Compound 4

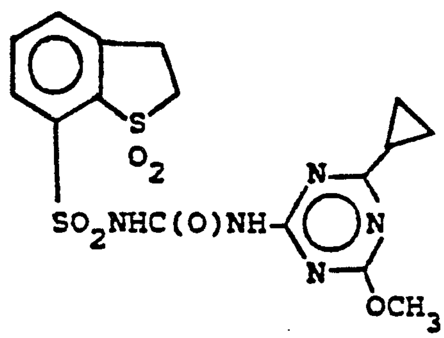

Compound 5

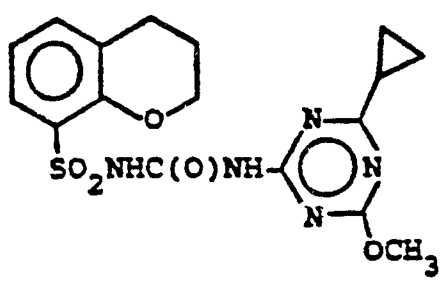

Test A

Seeds of crabgrass (*Digitaria* sp.). barnyardgrass (*Echinochloa crusgalli*), wild oats (*Avena fatua*), cheatgrass (*Bromus secalinus*), velvetleaf (*Abutilon theophrasti*), morningglory (*Ipomoea* sp.), cocklebur (*Xanthium* sp.), sorghum, corn, soybean, sugar beet, rice, wheat and nutsedge tubers (*Cyperus rotundus*) were planted in a growth medium and treated pre-emergence with chemicals dissolved in a non-phytotoxic solvent. At the same time, these crop and weed species, along with cotton and bush bean, were treated with a soil/foliage application. At the time of treatment, the plants ranged in height from 2 to 18 cm. Treated plants and controls were maintained in a greenhouse for sixteen days, after which all species were compared to controls and visually rated for response to treatment. The ratings are based on a numerical scale extending from 0 = no injury, to 10 = complete kill. The accompanying descriptive symbols have the following meanings:

C = chlorosis/necrosis;
E = emergence inhibitions;
G = growth retardation;
H = formative effects;
U = unusual pigmentation;
X = axillary stimulation; and
6Y = abscised buds or flowers.

TABLE A

| | Cmpd. 1 | Cmpd. 2 | Cmpd. 3 | Cmpd. 4 | Cmpd. 5 |
|---|---|---|---|---|---|
| Rate (kg/ha) | 0.05 | 0.05 | 0.05 | 0.05 | 0.05 |
| POST-EMERGENCE | | | | | |
| Morningglory | 3G | 10C | 10C | 5C, 9G | 3C, 8H |
| Cocklebur | 4C, 8H | 9C | 10C | 5C, 9H | 5C, 9G |
| Velvetleaf | 2C | 9C | 9C | 4C, 8G | 9C |
| Nutsedge | 0 | 3C, 5G | 5C, 9G | 3G | 3G |
| Crabgrass | 0 | 0 | 0 | 0 | 0 |
| Barnyardgrass | 0 | 3H | 0 | 0 | 5G |
| Cheatgrass | 0 | 5G | 0 | 0 | 0 |
| Wild Oats | 0 | 0 | 4C, 9H | 3G | 0 |
| Wheat | 0 | 0 | 0 | 0 | 0 |
| Corn | 0 | 3C, 9H | 9C | 4C, 9G | 3C, 9H |
| Soybean | 1H | 3C, 8H | 5C, 9G | 3H, 9G | 3C, 9H |
| Rice | 8G | 4C, 9G | 9C | 8G | 5C, 9G |
| Sorghum | 2C, 7H | 3C, 9H | 9C | 4C, 9G | 4C, 9H |
| Sugar Beets | 3C, 7G | 3C, 9H | 10C | 5C, 9H | 2C, 7G |
| Cotton | 0 | 3C, 9H | 5C, 9H | 9H | 5C, 9G |
| PRE-EMERGENCE | | | | | |
| Morningglory | 4G | 3C, 8H | 9G | 8H | 3C, 5H |
| Cocklebur | 0 | 3C, 5H | 9H | 0 | 2C, 5H |
| Velvetleaf | 0 | 8G | 3C, 6H | 0 | 5C, 9G |
| Nutsedge | 0 | 0 | 5G | 0 | 0 |
| Crabgrass | 0 | 0 | 0 | 0 | 0 |
| Barnyardgrass | 0 | 0 | 2H | 0 | 2C |
| Cheatgrass | 0 | 5G | 5G | 4G | 3G |
| Wild Oats | 0 | 0 | 5C, 9H | 3C, 7G | 3G |
| Wheat | 0 | 0 | 0 | 0 | 3G |
| Corn | 6G | 3C, 7H | 5C, 9G | 3C, 8H | 4C, 7G |
| Soybean | 0 | 3C, 3H | 3C, 6G | 2C, 4H | 0 |
| Rice | 7G | 3C, 9H | 10E | 3C, 7G | 8H |

TABLE A (Continued)

| | Cmpd. 1 | Cmpd. 2 | Cmpd. 3 | Cmpd. 4 | Cmpd. 5 |
|---|---|---|---|---|---|
| Rate (kg/ha) | 0.05 | 0.05 | 0.05 | 0.05 | 0.05 |
| PRE-EMERGENCE | | | | | |
| Sorghum | 8H | 3C, 8H | 5C, 9H | 4C, 9G | 3C, 8H |
| Sugar Beets | 7G | 4C, 9G | 9C | 4C, 9G | 4C, 9G |
| Cotton | 0 | 7G | 6G | 3G | 5G |

Test B

*Post-emergence*

Two round pans (25 cm diameter by 12.5 cm deep) were filled with Sassafras sandy loam soil. One pan was planted with blackgrass (*Alopecurus myosuroides*), sugar beets, nutsedge (*Cyperus rotundus*) tubers, crabrass (*Digitaria sanguinalis*), sicklepod (*Cassia obtusifolia*), teaweed (*Sida spinosa*), jimsonweed (*Datura stramonium*), velvetleaf (*Abutilon theophrasti*), giant foxtail (*Setaria faberii*) and rape (*Brassica napus*). The other pan was planted with wheat, cotton, rice, corn, soybean, wild oats (*Avena fatua*), cocklebur (*Xantium pensylvanicum*), morningglory (*Ipomoea hederacea*), johnsongrass (*Sorghum halepense*) and barnyardgrass (*Echinochloa crusgalli*). The plants were grown for approximately fourteen days, then sprayed post-emergence with the chemicals dissolved in a non-phytotoxic solvent.

*Pre-emergence*

Two round pans (25 cm diameter by 12.5 cm deep) were filled with Sassafras sandy loam soil. One pan was planted with blackgrass, sugar beets, nutsedge, crabgrass, sicklepod, teaweed, jimsonweed, velvetleaf, giant foxtail and rape. The other pan was planted with wheat, cotton, rice, corn, soybeans, wild oats, cocklebur, morningglory, johnsongrass, and barnyardgrass. The two pans were sprayed pre-emergence with the chemicals dissolved in a non-phytotoxic solvent.

Treated plants and controls were maintained in the greenhouse for 28 days, then all treated plants were compared to controls and visually rated for plant response utilizing the rating system as described for Test A.

The response is rated on a scale of 0—100 with 0 = no injury and 100 = complete control.

Response ratings are contained in Table B.

TABLE B
SASSAFRAS SANDY LOAM

| POST-EMERGENCE | Compound 2 | | | | Compound 3 | | | | Compound 4 | | |
|---|---|---|---|---|---|---|---|---|---|---|---|
| Rate g/ha | 250 | 62 | 16 | 4 | 250 | 62 | 16 | 4 | 62 | 16 | 4 |
| Corn | 100 | 100 | 30 | 0 | 100 | 100 | 100 | 90 | 100 | 90 | 20 |
| Wheat | 0 | 0 | 0 | 0 | 0 | 0 | 0 | 0 | 0 | 0 | 0 |
| Rice | 100 | 100 | 60 | 20 | 100 | 100 | 90 | 30 | 50 | 0 | 0 |
| Soybean | 100 | 70 | 20 | 0 | 100 | 100 | 90 | 50 | 100 | 70 | 20 |
| Cotton | 100 | 80 | 30 | 0 | 80 | 40 | 0 | 0 | 80 | 20 | 0 |
| Sugar beet | 100 | 100 | 90 | 70 | 100 | 100 | 100 | 90 | 100 | 100 | 50 |
| Rape | 100 | 100 | 90 | 50 | 100 | 100 | 100 | 50 | 100 | 90 | 40 |
| Crabgrass | 20 | 0 | 0 | 0 | 0 | 0 | 0 | 0 | 0 | 0 | 0 |
| Johnsongrass | 80 | 40 | 0 | 0 | 100 | 100 | 60 | 30 | 90 | 90 | 40 |
| Blackgrass | 100 | 70 | 30 | 0 | 100 | 100 | 100 | 40 | 50 | 20 | 0 |
| Barnyardgrass | 0 | 0 | 0 | 0 | 100 | 100 | 100 | 70 | 0 | 0 | 0 |
| Nutsedge | 40 | 0 | 0 | 0 | 100 | 100 | 40 | 0 | 0 | 0 | 0 |
| Giant Foxtail | 0 | 0 | 0 | 0 | 0 | 0 | 0 | 0 | 0 | 0 | 0 |
| Wild Oats | 20 | 0 | 0 | 0 | 100 | 70 | 30 | 0 | 0 | 0 | 0 |
| Cocklebur | 100 | 60 | 20 | 0 | 100 | 100 | 90 | 30 | 70 | 20 | 0 |
| Morningglory | 100 | 70 | 20 | 0 | 100 | 100 | 90 | 50 | 80 | 40 | 0 |
| Teaweed | 100 | 50 | 0 | 0 | 90 | 80 | 20 | 0 | 0 | 0 | 0 |
| Sicklepod | 40 | 0 | 0 | 0 | 100 | 90 | 40 | 0 | 40 | 0 | 0 |
| Jimsonweed | 90 | 70 | 30 | 0 | 100 | 100 | 70 | 0 | 70 | 50 | 20 |
| Velvetleaf | 100 | 100 | 20 | 0 | 100 | 100 | 70 | 30 | 70 | 30 | 0 |

TABLE B (Continued)
SASSAFRAS SANDY LOAM

| PRE-EMERGENCE | Compound 2 | | Compound 3 | | | Compound 4 | |
|---|---|---|---|---|---|---|---|
| Rate g/ha | 250 | 62 | 250 | 62 | 16 | 62 | 16 |
| Corn | 100 | 40 | 100 | 90 | 30 | 0 | 0 |
| Wheat | 20 | 0 | 0 | 0 | 0 | 0 | 0 |
| Rice | 100 | 60 | 100 | 100 | 90 | 70 | 0 |
| Soybean | 80 | 20 | 70 | 30 | 0 | 0 | 0 |
| Cotton | 60 | 0 | 0 | 0 | 0 | 0 | 0 |
| Sugar beet | 100 | 60 | 100 | 100 | 80 | 0 | 0 |
| Rape | 80 | 20 | 100 | 90 | 30 | 0 | 0 |
| Crabgrass | 40 | 0 | 90 | 40 | 0 | 20 | 0 |
| Johnsongrass | 50 | 40 | 100 | 100 | 100 | 80 | 0 |
| Blackgrass | 80 | 30 | 100 | 100 | 80 | 90 | 30 |
| Barnyardgrass | 0 | 0 | 100 | 40 | 0 | 0 | 0 |
| Nutsedge | 0 | 0 | 100 | 100 | 40 | 0 | 0 |
| Giant Foxtail | 30 | 0 | 0 | 0 | 0 | 0 | 0 |
| Wild Oats | 0 | 0 | 90 | 70 | 20 | 30 | 0 |
| Cocklebur | 50 | 0 | 70 | 30 | 0 | 20 | 0 |
| Morningglory | 50 | 0 | 100 | 50 | 30 | 20 | 0 |
| Teaweed | 60 | 20 | 100 | 20 | 0 | 0 | 0 |
| Sicklepod | 50 | 20 | 80 | 30 | 0 | 0 | 0 |
| Jimsonweed | 80 | 20 | 100 | 90 | 20 | 30 | 0 |
| Velvetleaf | 90 | 40 | 90 | 60 | 0 | 0 | 0 |

## Claims

1. A compound of the formula:

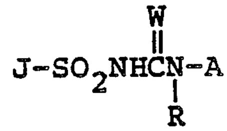

I

wherein

J is 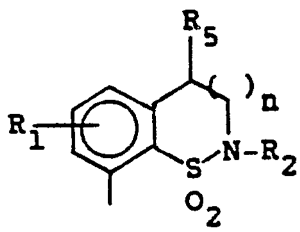 ,

J1

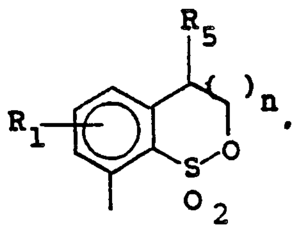 ,

J2

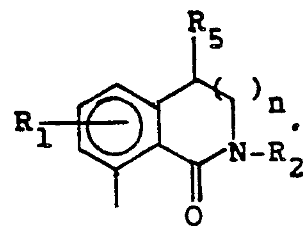 ,

J3

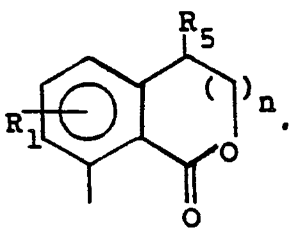 ,

J4

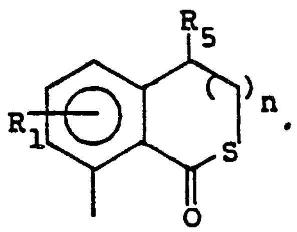 ,

J5

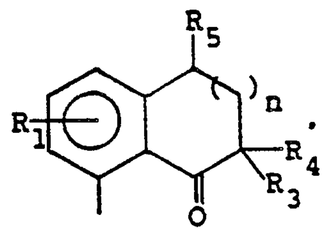 ,

J6

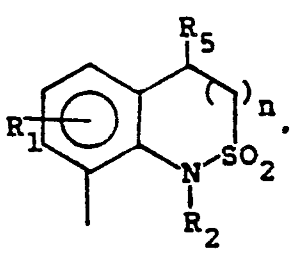 ,

J7

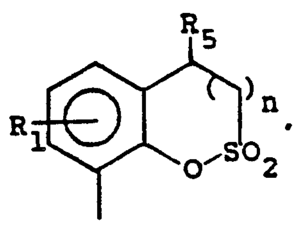 ,

J8

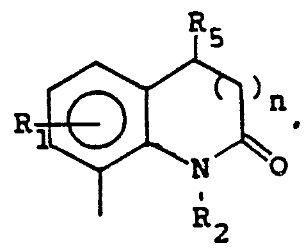 ,

J9

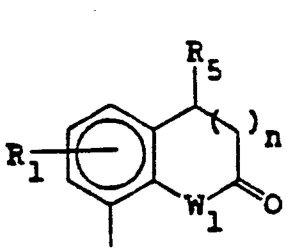 ,

J10

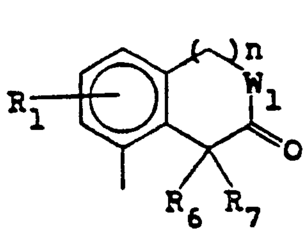 ,

J11

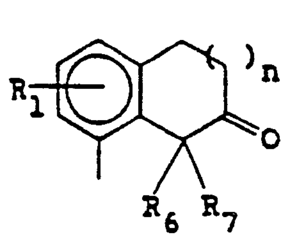 ,

J12

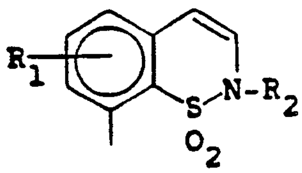 ,

$J_{13}$

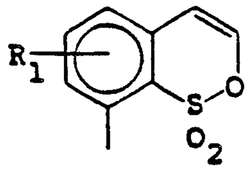 ,

$J_{14}$

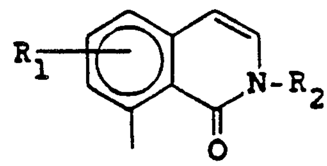 ,

$J_{15}$

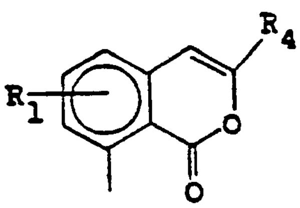 ,

$J_{16}$

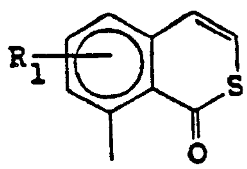 ,

$J_{17}$

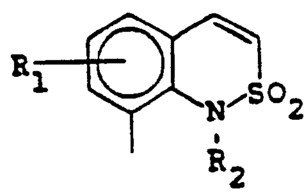 ,

$J_{18}$

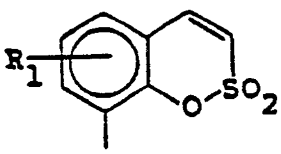 ,

$J_{19}$

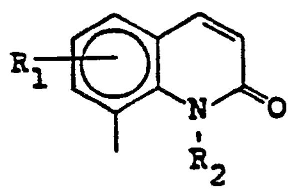 ,

$J_{20}$

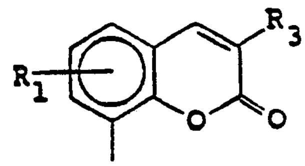 ,

$J_{21}$

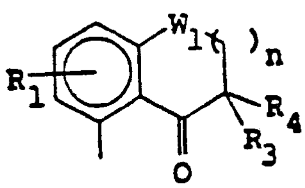 ,

$J_{22}$

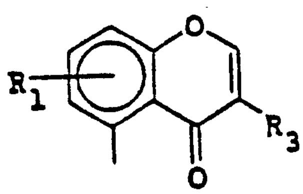 ,

$J_{23}$

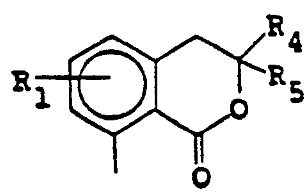 ,

$J_{24}$

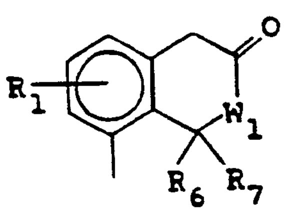

$J_{25}$

or

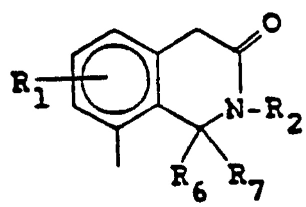 :

$J_{26}$

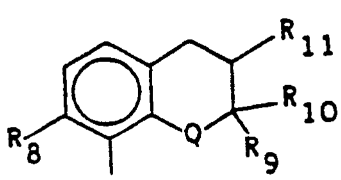 ,

$J_{27}$

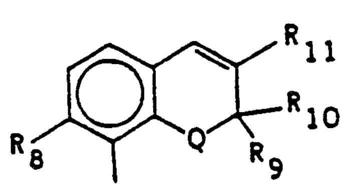 ,

$J_{28}$

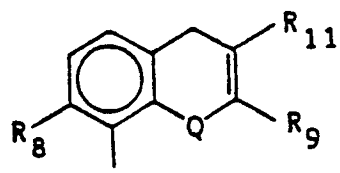 ,

$J_{29}$

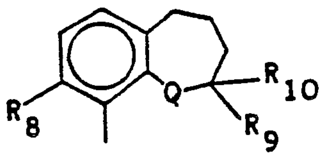,

J30

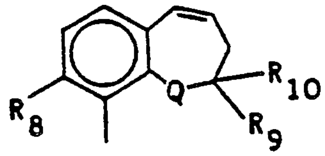,

J31

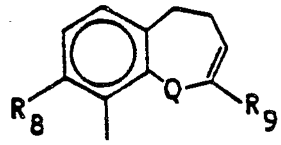,

J32

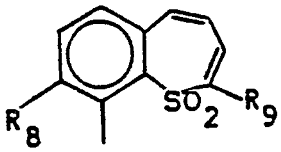,

J33

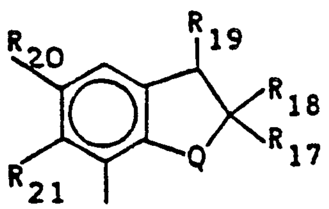,

J34

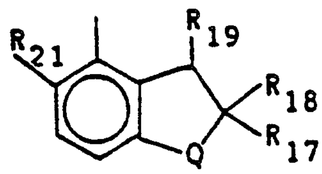,

J35

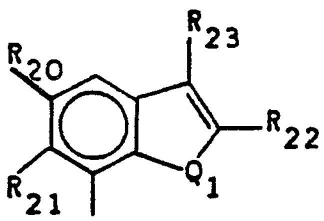,

J36

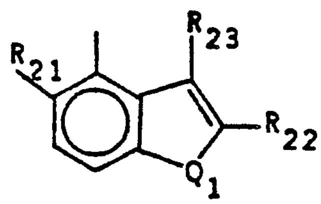;

J37

n is 0, 1 or 2;
W is O or S;
$W_1$ is O or S;
Q is O, S, SO or $SO_2$;
$Q_1$ is O, S or $SO_2$;
R is H or $CH_3$;
$R_1$ is H, F, Cl, Br, $CH_3$, $OCH_3$, $CF_3$, $SCH_3$ or $OCF_2H$;
$R_2$ is H or $C_1$—$C_4$ alkyl;
$R_3$ and $R_4$ are independently H, $C_1$—$C_4$ alkyl, Cl or Br;
$R_5$ is H or $CH_3$;
$R_6$ is H or $CH_3$;
$R_7$ is H or $CH_3$;
$R_8$ is H, $CH_3$, $OCH_3$, Cl, Br, $CO_2R_{12}$, $SO_2R_{13}$, $OSO_2R_{14}$ or $SO_2NR_{15}R_{16}$;
$R_9$ and $R_{10}$ are independently H or $C_1$—$C_3$ alkyl;
$R_{11}$ is H or $CH_3$;
$R_{12}$ is $C_1$—$C_3$ alkyl, $CH_2CH{=}CH_2$, $CH_2CH_2OCH_3$ or $CH_2CH_2Cl$;
$R_{13}$ is $C_1$—$C_3$ alkyl;
$R_{14}$ is $C_1$—$C_3$ alkyl or $CF_3$;
$R_{15}$ and $R_{16}$ are independently $C_1$—$C_2$ alkyl;
$R_{17}$ is H or $C_1$—$C_4$ alkyl;
$R_{18}$ is H or $C_1$—$C_4$ alkyl;
$R_{19}$ is H or $CH_3$;
$R_{20}$ is H, Cl, $CH_3$, $CF_3$, $OCH_3$, Br, F, $SCH_3$ or $OCF_2H$;
$R_{21}$ is H, $CH_3$, $OCH_3$, Cl, Br, $NO_2$, $CO_2R_{24}$, $SO_2R_{25}$, $OSO_2R_{26}$, $SO_2NR_{27}R_{28}$, F, $CF_3$, $SCH_3$, $OCF_2H$ or $SO_2N(OCH_3)CH_3$;
$R_{22}$ is H, Cl, Br or $C_1$—$C_4$ alkyl;
$R_{23}$ is H, $CH_3$, Cl or Br;
$R_{24}$ is $C_1$—$C_3$ alkyl, $CH_2CH{=}CH_2$, $CH_2CH_2OCH_3$ or $CH_2CH_2Cl$;
$R_{25}$ is $C_1$—$C_3$ alkyl;
$R_{26}$ is $C_1$—$C_3$ alkyl or $CF_3$;
$R_{27}$ and $R_{28}$ are independently $C_1$—$C_2$ alkyl;

A is 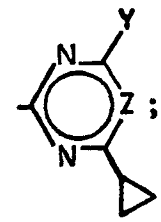 ;

Y is $C_1$—$C_2$ alkyl, $C_1$—$C_2$ alkoxy, $C_1$—$C_2$ alkoxymethyl, $OCF_2H$, $SCF_2H$, $OCH_2CH_2F$, $OCH_2CF_3$, $CF_3$, $OCH_2CH{=}CH_2$, $OCH_2C{\equiv}CH$, $NHCH_3$, $N(CH_3)_2$ or $CH(OCH_3)_2$; and

Z is CH or N;

and their agriculturally suitable salts; provided that

a) when W is S, then R is H;

b) the total number of carbon atoms in $R_3$ and $R_4$ is less than or equal to 4;

c) when $R_5$ is $CH_3$, then n is O;

d) when J is $J_{24}$, then $R_4$ and $R_5$ are not both H;

e) the total number of carbon atoms in $R_9$ and $R_{10}$ is less than or equal to 3;

f) in Formula $J_{28}$, when $R_9$ and $R_{10}$ are other than H, then $R_{11}$ is H;

g) in Formula $J_{29}$, when $R_9$ is other than H, then $R_{11}$ is H;

h) when Q is S, then $R_8$ is not $SO_2NR_{15}R_{16}$;

i) in Formulae $J_{29}$ and $J_{32}$, Q may not be O;

j) in Formula $J_{33}$, $R_8$ is not $CH_3$;

k) in Formulae $J_{36}$ and $J_{37}$, when $R_{21}$ is $NO_2$, then $R_{22}$ is $C_1$—$C_4$ alkyl and $R_{23}$ is $CH_3$;

l) in Formulae $J_{34}$ and $J_{35}$, when Q is SO, then W is O;

m) when $R_{20}$ is other than H, then $R_{21}$ is H;

n) $R_{17}$ and $R_{18}$ taken together are not more than four carbon atoms;

o) when J is $J_{27}$, $J_{28}$, $J_{29}$, $J_{30}$, $J_{31}$ or $J_{32}$, then Q is O, S or $SO_2$; and

p) when Y is $OCF_2H$ then Z is CH.

2. A compound of Claim 1 where W is O; $R_3$ and $R_4$ are independently H or $C_1$—$C_3$ alkyl; $R_{17}$ is H or $C_1$—$C_2$ alkyl; $R_{18}$ is H or $CH_3$; $R_{19}$ is H; $R_{22}$ is H or $C_1$—$C_2$ alkyl; $R_{23}$ is H; and $R_1$ is bonded to the *ortho* or *meta* position of the ring relative to the sulfonylurea moiety.

3. A compound of Claim 2 wherein R is H; $R_1$ is H, F, Cl, Br, $CH_3$, $OCH_3$, $CF_3$ or $SCH_3$; $R_8$ is H, $CH_3$, $OCH_3$ or Cl; $R_{20}$ is H, Cl, $CH_3$ or $OCH_3$; and $R_{21}$ is H, Cl, $CH_3$, $OCH_3$, $CO_2R_{24}$ or $SO_2R_{25}$.

4. A compound of Claim 3 wherein $R_1$ is H; $R_5$ is H; $R_8$ is H; $R_{20}$ is H; $R_{21}$ is H; and Y is $CH_3$, $OCH_3$ or $CH_2OCH_3$.

5. A compound of Claim 4 where J is $J_{27}$ or $J_{34}$.

6. A compound of Claim 1 wherein J is $J_{34}$, $J_{35}$, $J_{36}$ or $J_{37}$.

7. A compound of Claim 1 wherein J is selected from $J_1$—$J_{26}$.

8. A composition suitable for controlling the growth of undesired vegetation which comprises an effective amount of a compound of any of Claims 1 to 7 and at least one of the following: surfactant, solid or liquid diluent.

9. A method for controlling the growth of undesired vegetation which comprises applying to the locus to be protected an effective amount of a compound of any of claims 1 to 7.

10. A method for regulating the growth of plants which comprises applying to the locus of such plants an effective but substantially non-phytotoxic amount of a plant growth regulant as claimed in any of claims 1 to 7.

11. A process for the production of a compound of claim 1 which comprises:

(a) reacting a sulfonylisocyanate or sulfonylisothiocyanate of formula

$$J—SO_2NCW \qquad (II)$$

wherein J is as defined in claim 1 other than $J_6$, $J_{12}$, $J_{22}$ or $J_{23}$ and when Q is S or O, J is other than $J_{28}$, $J_{29}$, $J_{31}$ or $J_{32}$; and W is as defined in claim 1, with an appropriate heterocyclic amine of formula

HN—A (III)
|
R

wherein R and A are as defined in claim 1; or

(b) reacting a sulfonamide of formula

$$J—SO_2NH_2 \qquad (IV)$$

wherein J is as defined in claim 1, with a heterocyclic isothiocyanate of formula

SCN—A

wherein A is as defined in claim 1, to obtain a compound of claim 1 wherein W is S and R is H; or

(c) reacting a sulfonylcarbamate of formula

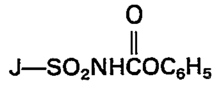

(VI)

wherein J is as defined in claim 1, with said heterocyclic amine (III) defined above; or

(d) reacting said sulfonamide (IV) defined above with a heterocyclic carbamate of formula

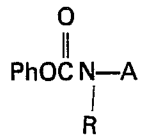

(VII)

wherein A and R are as defined in claim 1; or

(e) reacting said sulfonamide (IV) wherein J is as defined in claim 1 other than $J_4$, $J_5$, $J_{10}$, $J_{11}$, $J_{16}$, $J_{17}$, $J_{21}$, $J_{24}$ or $J_{25}$ and $R_8$ is other than $CO_2R_{12}$ and $R_{21}$ is other than $CO_2R_{24}$, with a methylcarbamate of formula

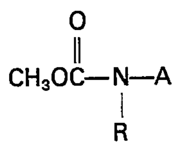

(VIII)

wherein A and R are as defined in claim 1.

## Patentansprüche

1. Verbindung der Formel:

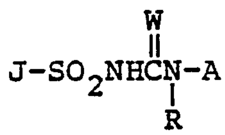

I

worin J

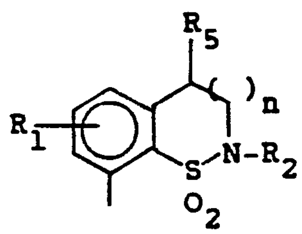,

$J_1$

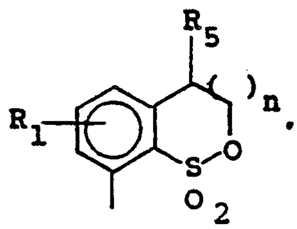,

$J_2$

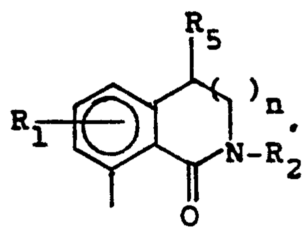,

$J_3$

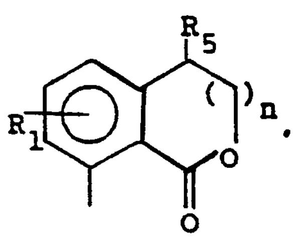,

$J_4$

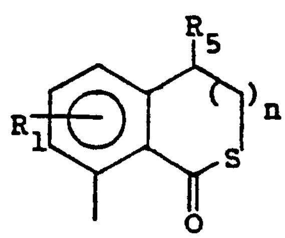,

$J_5$

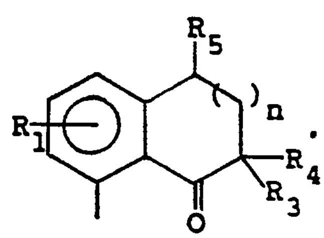,

$J_6$

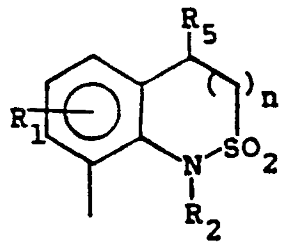,
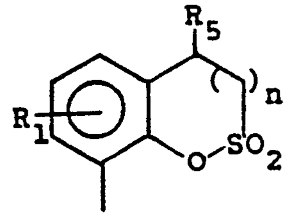,
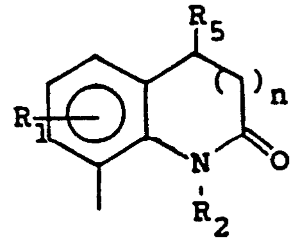,

$J_7$ $J_8$ $J_9$

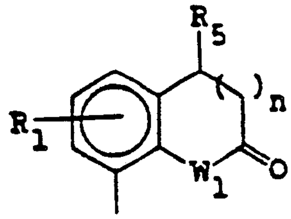,
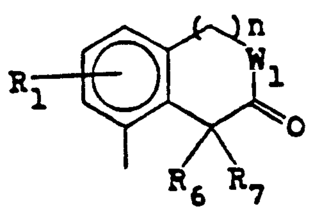,
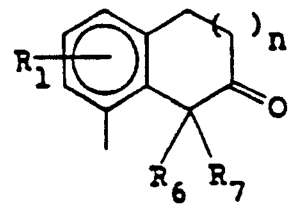,

$J_{10}$ $J_{11}$ $J_{12}$

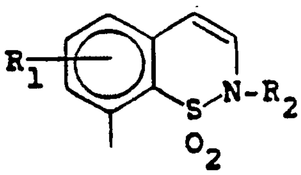,
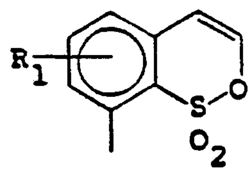,
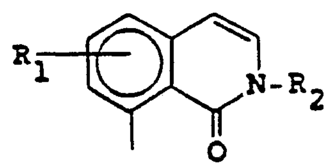,

$J_{13}$ $J_{14}$ $J_{15}$

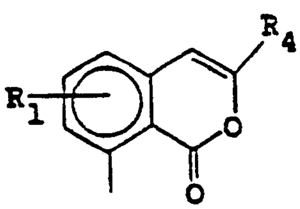,
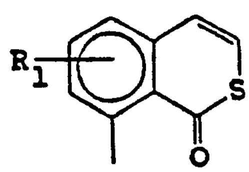,
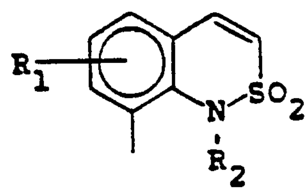,

$J_{16}$ $J_{17}$ $J_{18}$

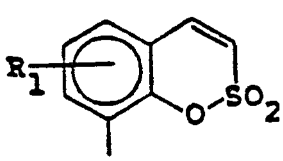,
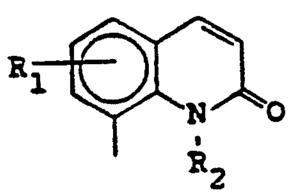,
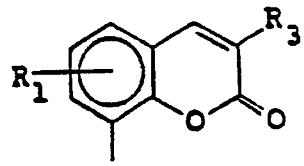,

$J_{19}$ $J_{20}$ $J_{21}$

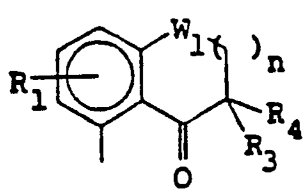,
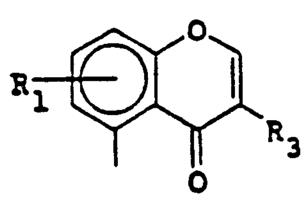,
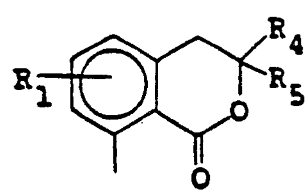,

$J_{22}$ $J_{23}$ $J_{24}$

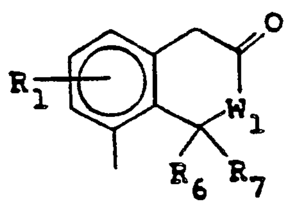 oder 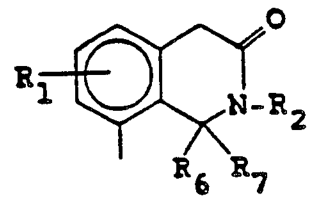 ;

$\underline{J_{25}}$ $\underline{J_{26}}$

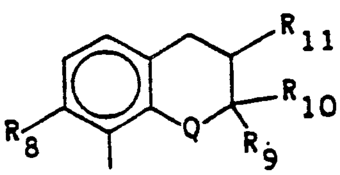 , 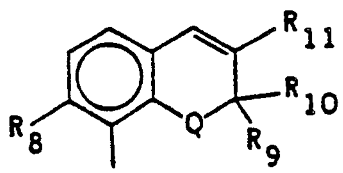 , 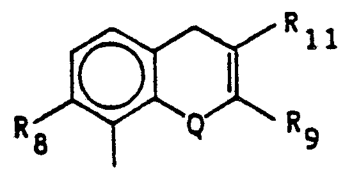 ,

$\underline{J_{27}}$ $\underline{J_{28}}$ $\underline{J_{29}}$

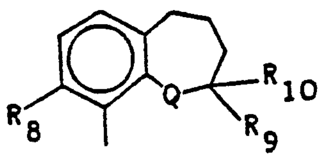 , 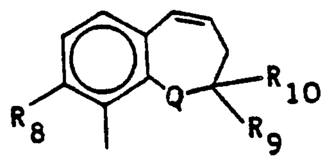 , 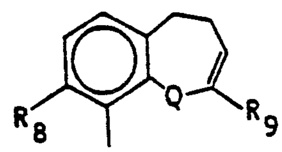 ,

$\underline{J_{30}}$ $\underline{J_{31}}$ $\underline{J_{32}}$

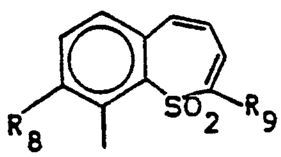 , 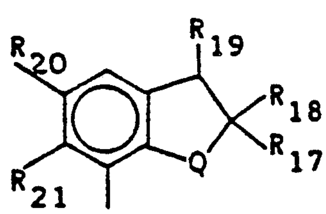 , 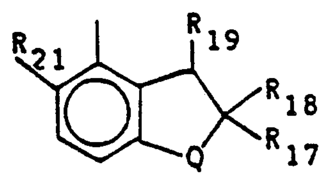 ,

$\underline{J_{33}}$ $\underline{J_{34}}$ $\underline{J_{35}}$

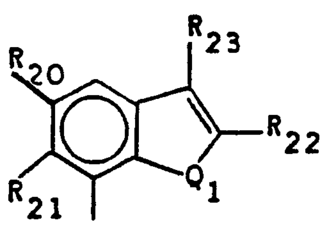 , 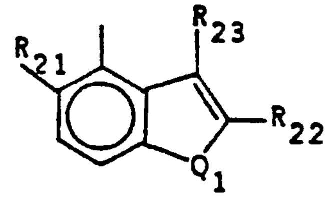 ist;

$\underline{J_{36}}$ $\underline{J_{37}}$

n 0, 1 oder 2 ist;
W O oder S ist;
$W_1$ O oder S ist;
Q O, S, SO oder $SO_2$ ist
$Q_1$ O, S oder $SO_2$ ist;
R H oder $CH_3$ ist;
$R_1$ H, F, Cl, Br, $CH_3$, $OCH_3$, $CF_3$, $SCH_3$ oder $OCF_2H$ ist;
$R_2$ H oder $C_1$—$C_4$-Alkyl ist;
$R_3$ und $R_4$ unabhängig H, $C_1$—$C_4$-Alkyl, Cl oder Br sind;
$R_5$ H oder $CH_3$ ist;
$R_6$ H oder $CH_3$ ist;

$R_7$ H oder $CH_3$ ist;
$R_8$ H, $CH_3$, $OCH_3$, Cl, Br, $CO_2R_{12}$, $SO_2R_{13}$, $OSO_2R_{14}$ oder $SO_2NR_{15}R_{16}$ ist;
$R_9$ und $R_{10}$ unabhängig H oder $C_1$—$C_3$-Alkyl sind;
$R_{11}$ oder $CH_3$ ist;
$R_{12}$ $C_1$—$C_3$-Alkyl, $CH_2CH=CH_2$, $CH_2CH_2OCH_3$ oder $CH_2CH_2Cl$ ist;
$R_{13}$ $C_1$—$C_3$-Alkyl ist;
$R_{14}$ $C_1$—$C_3$-Alkyl oder $CF_3$ ist;
$R_{15}$ und $R_{16}$ unabhängig $C_1$—$C_2$-Alkyl sind;
$R_{17}$ H oder $C_1$—$C_4$-Alkyl ist;
$R_{18}$ H oder $C_1$—$C_4$-Alkyl ist;
$R_{19}$ H oder $CH_3$ ist;
$R_{20}$ H, Cl, $CH_3$, $CF_3$, $OCH_3$, Br, F, $SCH_3$ oder $OCF_2H$ ist;
$R_{21}$ H, $CH_3$, $OCH_3$, Cl, Br, $NO_2$, $CO_2R_{24}$, $SO_2R_{25}$, $OSO_2R_{26}$, $SO_2NR_{27}R_{28}$, F, $CF_3$, $SCH_3$, $OCF_2H$ oder $SO_2N(OCH_3)CH_3$ ist;
$R_{22}$ H, Cl, Br oder $C_1$—$C_4$-Alkyl ist;
$R_{23}$ H, $CH_3$, Cl oder Br ist;
$R_{24}$ $C_1$—$C_3$-Alkyl, $CH_2CH=CH_2$, $CH_2CH_2OCH_3$ oder $CH_2CH_2Cl$ ist;
$R_{25}$ $C_1$—$C_3$-Alkyl ist;
$R_{26}$ $C_1$—$C_3$-Alkyl oder $CF_3$ ist;
$R_{27}$ und $R_{28}$ unabhängig $C_1$—$C_2$-Alkyl sind;

A 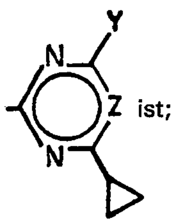 ist;

Y $C_1$—$C_2$-Alkyl, $C_1$—$C_2$-Alkoxy, $C_1$—$C_2$-Alkoxymethyl, $OCF_2H$, $SCF_2H$, $OCH_2CH_2F$, $OCH_2CF_3$, $CF_3$, $OCH_2CH=CH_2$, $OCH_2C{=}CH$, $NHCH_3$, $N(CH_3)_2$ oder $CH(OCH_3)_2$ ist; und
Z CH oder N ist;

und ihre landwirtschaftlich geeigneten Salze; mit der Maßgabe, daß
a) wenn W S ist, dann R H ist;
b) die Gesamtzahl an Kohlenstoffatomen in $R_3$ und $R_4$ weniger oder gleich vier ist;
c) wenn $R_5$ $CH_3$ ist, dann n 0 ist;
d) wenn J $J_{24}$ ist, dann $R_4$ und $R_5$ nicht beide H sind;
e) die Gesamtzahl oder Kohlenstoffatome in $R_9$ und $R_{10}$ weniger oder gleich 3 ist;
f) in Formel $J_{28}$, wenn $R_9$ und $R_{10}$ von H verschieden sind, dann $R_{11}$ H ist;
g) in Formel $J_{29}$, wenn $R_9$ von H verschieden ist, dann $R_{11}$ H ist;
h) wenn Q S ist, dann $R_8$ nicht $SO_2NR_{15}R_{16}$ ist;
i) in den Formeln $J_{29}$ und $J_{32}$ Q nicht O sein darf;
j) in Formel $J_{33}$ $R_8$ nicht $CH_3$ ist;
k) in den Formeln $J_{36}$ und $J_{37}$, wenn $R_{21}$ $NO_2$ ist, dann $R_{22}$ $C_1$—$C_4$-Alkyl und $R_{23}$ $CH_3$ ist;
l) in den Formeln $J_{34}$ und $J_{35}$, wenn Q SO ist, dann W O ist;
m) wenn $R_{20}$ von H verschieden ist, dann $R_{21}$ H ist;
n) $R_{17}$ und $R_{18}$ zusammengenommen nicht mehr als vier Kohlenstoffatome sind;
o) wenn J $J_{27}$, $J_{28}$, $J_{29}$, $J_{30}$, $J_{31}$ oder $J_{32}$ ist, dann Q O, S oder $SO_2$ ist; und
p) wenn Y $OCF_2H$ ist, dann Z CH ist.

2. Verbindung nach Anspruch 1, worin W O ist; $R_3$ und $R_4$ unabhängig H oder $C_1$—$C_3$-Alkyl sind; $R_{17}$ H oder $C_1$—$C_2$-Alkyl ist; $R_{18}$ H oder $CH_3$ ist; $R_{19}$ H ist; $R_{22}$ H oder $C_1$—$C_2$-Alkyl ist; $R_{23}$ H ist; und $R_1$ in der *ortho*- oder *meta*-Stellung des Ring relativ zur Sulfonylharnstoffeinheit gebunden ist.

3. Verbindung nach Anspruch 2, worin R H ist; $R_1$ H, F, Cl, Br, $CH_3$, $OCH_3$, $CF_3$ oder $SCH_3$ ist; $R_8$ H, $CH_3$, $OCH_3$ oder Cl ist; $R_{20}$ H, Cl, $CH_3$ oder $OCH_3$ ist; und $R_{21}$ H, Cl, $CH_3$, $OCH_3$, $CO_2R_{24}$ oider $SO_2R_{25}$ ist.

4. Verbindung nach Anspruch 3, worin $R_1$ H ist; $R_5$ H ist; $R_8$ H ist; $R_{20}$ H ist; $R_{21}$ H ist; und Y $CH_3$, $OCH_3$ oder $CH_2OCH_3$ ist.

5. Verbindung nach Anspruch 4, worin J $J_{27}$ oder $J_{34}$ ist.

6. Verbindung nach Anspruch 1, worin J $J_{34}$, $J_{35}$, $J_{36}$ oder $J_{37}$ ist.

7. Verbindung nach Anspruch 1, worin H aus $J_1$—$J_{26}$ ausgewählt.

8. Zur Bekämpfung des Wachstums unerwünschter Vegetation geeignete Zusammensetzung, die eine wirksame Menge einer Verbindung nach einem der Ansprüche 1 bis 7 und wenigstens eines der folgenden enthält: oberflächenaktives Mittel, festes oder flüssiges Verdünnungsmittel.

9. Verfahren zur Bekämpfung des Wachstums unerwünschter Vegetation, das die Anwendung einer wirksamen Menge einer Verbindung nach einem der Ansprüche 1 bis 7 auf den zu schützenden Ort umfaßt.

10. Verfahren zur Steuerung des Pflanzenwachstums, welches die Anwendung einer wirksamen, jedoch im wesentlichen nicht phytotoxischen Menge eines Pflanzenwachstumsregulans, wie in einem der Ansprüche 1 bis 7 beansprucht, auf den Ort solcher Pflanzen umfaßt.

11. Vefahren zur Herstellung einer Verbindung nach Anspruch 1, durch

(a) Reagieren eines Sulfonylisocyanats oder Sulfonylisothiocyanats der Formel

$$J—SO_2NCW \qquad (II)$$

worin J wie in Anspruch 1 definiert ist, von $J_6$, $J_{12}$, $J_{22}$ oder $J_{23}$ verschieden ist und, wenn Q S oder O ist, von $J_{28}$, $J_{29}$, $J_{31}$ oder $J_{32}$ verschieden ist; und W wie in Anspruch 1 definiert ist, mit einem geeigneten heterocyclischen Amin der Formel

HN—A (III)
|
R

worin R und A wie in Anspruch 1 definiert sind; oder

(b) Reagieren eines Sulfonamids der Formel

$$J—SO_2NH_2 \qquad (IV)$$

worin J wie in Anspruch 1 definiert ist, mit einem heterocyclischen Isothiocyanat der Formel

SCN—A

worin A wie in Anspruch 1 definiert ist, unter Erhalt einer Verbindung nach Anspruch 1, worin W S ist und R H ist; oder

(c) Reagieren eines Sulfonylcarbamats der Formel

O
||
$J—SO_2NHCOC_6H_5$ (VI)

worin J wie in Anspruch 1 definiert ist, mit dem oben definierten heterocyclischen Amin (III); oder

(d) Reagieren des oben definierten Sulfonamids (IV) mit einem heterocyclischen Carbamat der Formel

O
||
PhOCN—A (VII)
|
R

worin A und R wie in Anspruch 1 definiert sind; oder

(e) Reagieren des Sulfonamids (IV), worin J wie in Anspruch 1 definiert ist und von $J_4$,$J_5$, $J_{10}$, $J_{11}$, $J_{16}$, $J_{17}$, $J_{21}$, $J_{24}$ oder $J_{25}$ verschieden ist und $R_8$ von $CO_2R_{12}$ verschieden ist und $R_{21}$ von $CO_2R_{24}$ verschieden ist, mit einem Methylcarbamat der Formel

O
||
$CH_3OC—N—A$ (VIII)
|
R

worin A und R wie in Anspruch 1 definiert sind.

**Revendications**

1. Un composé de la formule:

W
||
$J-SO_2NHCN-A$ I
|
R

dans laquelle

J est 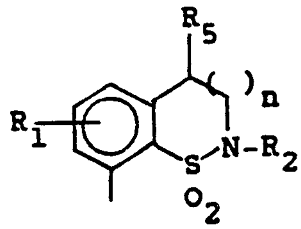, 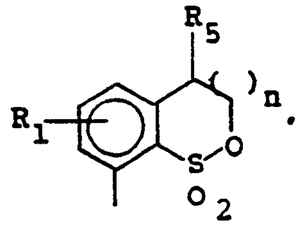, 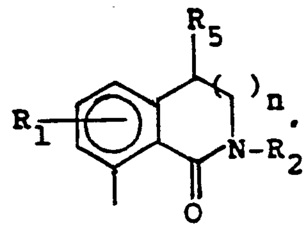,

$J_1$ $J_2$ $J_3$

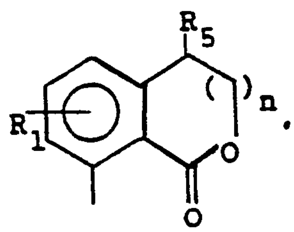, 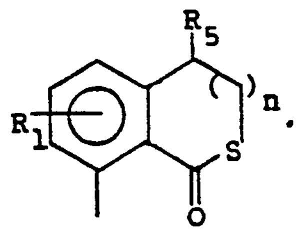, 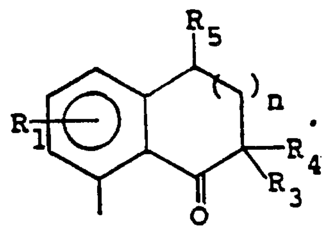,

$J_4$ $J_5$ $J_6$

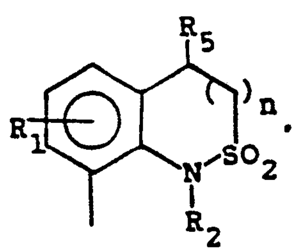, 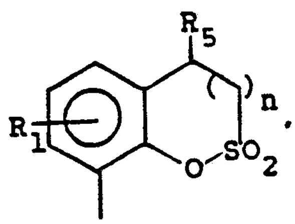, 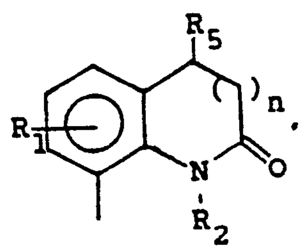,

$J_7$ $J_8$ $J_9$

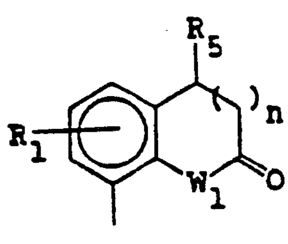, 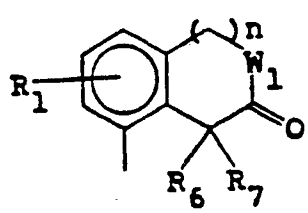, 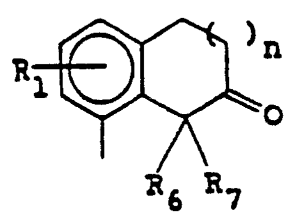,

$J_{10}$ $J_{11}$ $J_{12}$

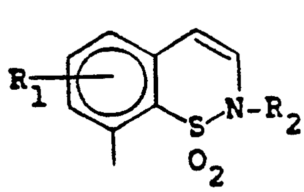, 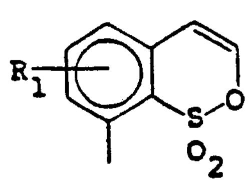, 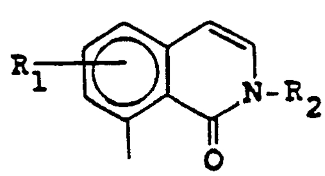,

$J_{13}$ $J_{14}$ $J_{15}$

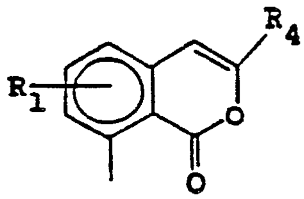 . 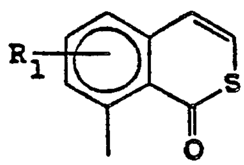 . 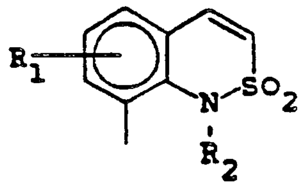 .

$J_{16}$ $J_{17}$ $J_{18}$

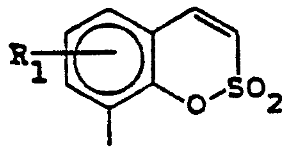 . 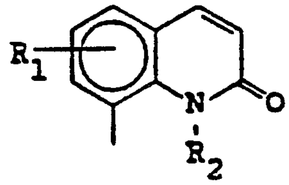 . 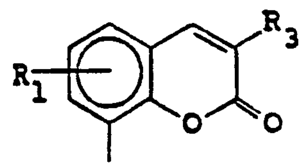 .

$J_{19}$ $J_{20}$ $J_{21}$

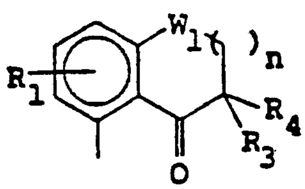 . 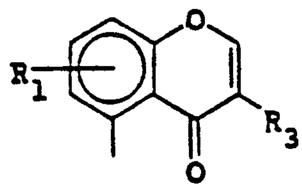 . 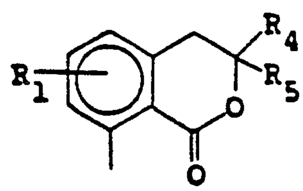 .

$J_{22}$ $J_{23}$ $J_{24}$

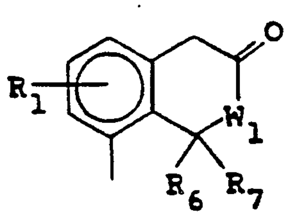 ou 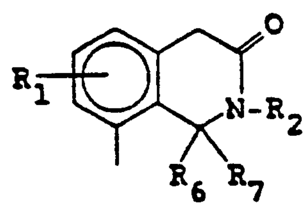 ;

$J_{25}$ $J_{26}$

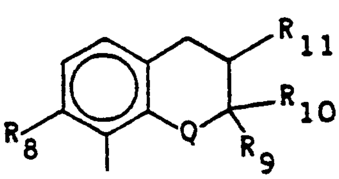 , 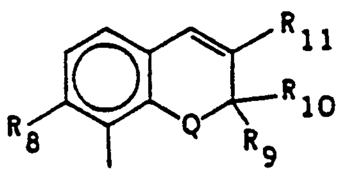 , 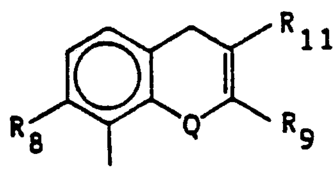 ,

$J_{27}$ $J_{28}$ $J_{29}$

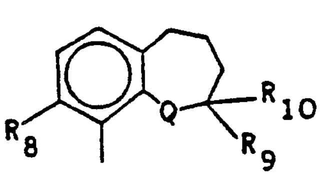 , 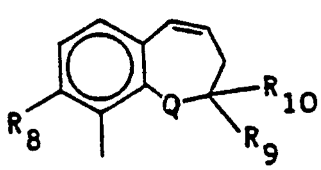 , 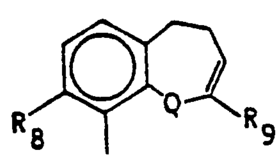 ,

$J_{30}$ $J_{31}$ $J_{32}$

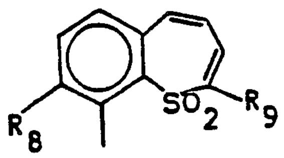 , 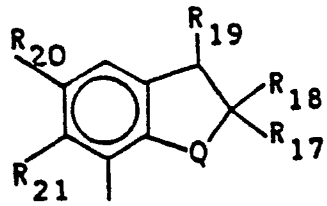 , 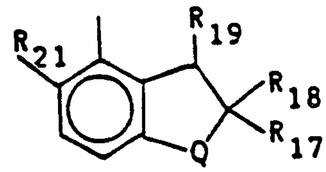 ,

J33 J34 J35

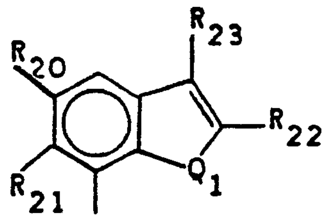 , 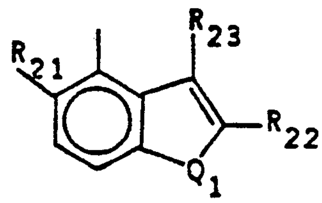 ;

J36 J37

n est O, 1 ou 2;
W est O ou S;
$W_1$ est O ou S;
Q est O, S, SO ou $SO_2$;
$Q_1$ est O, S ou $SO_2$;
R est H ou $CH_3$;
$R_1$ est H, F, Cl, Br, $CH_3$, $OCH_3$, $CF_3$, $SCH_3$ ou $OCF_2H$;
$R_2$ est H ou un groupe alkyle en $C_1$—$C_4$;
$R_3$ et $R_4$ sont indépendamment H, un groupe alkyle en $C_1$—$C_4$, Cl ou Br;
$R_5$ est H ou $CH_3$;
$R_6$ est H ou $CH_3$;
$R_7$ est H ou $CH_3$;
$R_8$ est H, $CH_3$, $OCH_3$, Cl, Br, $CO_2R_{12}$, $SO_2R_{13}$, $OSO_2R_{14}$ ou $SO_2NR_{15}R_{16}$;
$R_9$ et $R_{10}$ sont indépendamment H ou un groupe alkyle en $C_1$—$C_3$;
$R_{11}$ est H ou $CH_3$;
$R_{12}$ est un groupe alkyle en $C_1$—$C_3$, $CH_2CH=CH_2$, $CH_2CH_2OCH_3$ ou $CH_2CH_2Cl$;
$R_{13}$ est un groupe alkyle en $C_1$—$C_3$;
$R_{14}$ est un groupe alkyle en $C_1$—$C_3$ ou $CF_3$;
$R_{15}$ et $R_{15}$ et $R_{16}$ sont indépendamment un groupe alkyle en $C_1$—$C_2$;
$R_{17}$ est H ou un groupe alkyle en $C_1$—$C_4$;
$R_{18}$ est H ou un groupe alkyle en $C_1$—$C_4$;
$R_{19}$ est H ou $CH_3$;
$R_{20}$ est H, Cl, $CH_3$, $CF_3$, $OCH_3$, Br, F, $SCH_3$ ou $OCF_2H$;
$R_{21}$ est H, $CH_3$, $OCH_3$, Cl, Br, $NO_2$, $CO_2R_{24}$, $SO_2R_{25}$, $OSO_2R_{26}$, $SO_2NR_{27}R_{28}$, F, $CF_3$, $SCH_3$, $OCF_2H$ ou $SO_2N(OCH_3)CH_3$;
$R_{22}$ est H, Cl, Br ou un groupe alkyle en $C_1$—$C_4$;
$R_{23}$ est H, $CH_3$, Cl ou Br;
$R_{24}$ est un groupe alkyle en $C_1$—$C_3$, $CH_2CH=CH_2$, $CH_2CH_2OCH_3$ ou $CH_2CH_2Cl$;
$R_{25}$ est un groupe alkyle en $C_1$—$C_3$;
$R_{26}$ est un groupe alkyle en $C_1$—$C_3$ ou $CF_3$;
$R_{27}$ et $R_{28}$ sont indépendamment un groupe alkyle en $C_1$—$C_2$;

A est 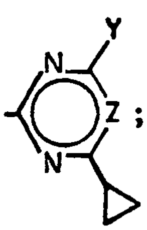 ;

Y est un groupe alkyle en $C_1$—$C_2$, alcoxy en $C_1$—$C_2$, (alcoxy en $C_1$—$C_2$)méthyle, $OCF_2H$, $SCF_2H$, $OCH_2CH_2F$, $OCH_2CF_3$, $CF_3$, $OCH_2CH=CH_2$, $OCH_2C\equiv CH$, $NHCH_3$, $N(CH_3)_2$ ou $CH(OCH_3)_2$; et
Z est CH ou N;

et leurs sels utilisables en agriculture;
avec les conditions que;

a) si W est S, R est alors H;
b) le nombre total d'atomes de carbone dans $R_3$ et $R_4$ est inférieur ou égal à 4;
c) si $R_5$ est $CH_3$, n est alors O;
d) si J est $J_{24}$, alors $R_4$ et $R_5$ ne sont pas tous deux H;
e) le nombre total d'atomes de carbone dans $R_9$ et $R_{10}$ est inférieur ou égal à 3;
f) dans la Formule $J_{28}$ si $R_9$ et $R_{10}$ sont autre chose que H, alors $R_{11}$ est H;
g) dans la Formule $J_{29}$, si $R_9$ est autre chose que H, alors $R_{11}$ est H;
h) si Q est S, alors $R_8$ n'est pas $SO_2NR_{15}R_{16}$;
i) dans les Formules $J_{29}$ et $J_{32}$, Q ne peut pas être O;
j) dans la Formule $J_{33}$, $R_8$ n'est pas $CH_3$;
k) dans les Formules $J_{36}$ et $J_{37}$, si $R_{21}$ est $NO_2$, alors $R_{22}$ est un groupe alkyle en $C_1$—$C_4$ et $R_{23}$ est $CH_3$;
l) dans les Formules $J_{34}$ et $J_{35}$, si Q est SO, alors W est O;
m) si $R_{20}$ est autre chose que H, alors $R_{21}$ est H;
n) $R_{17}$ et $R_{18}$, considérés ensemble, ne totalisent pas plus de quatre atomes de carbone;
o) si J est $J_{27}$, $J_{28}$, $J_{29}$, $J_{30}$, $J_{31}$ ou $J_{32}$, alors Q est O, S ou $SO_2$; et
p) si Y est $OCF_2H$, alors Z est CH.

2. Un composé selon la revendication 1, dans lequel W est O; $R_3$ et $R_4$ sont indépendamment H ou un groupe alkyle en $C_1$—$C_3$; $R_{17}$ est H ou un groupe alkyle en $C_1$—$C_2$; $R_{18}$ est H ou $CH_3$; $R_{19}$ est H; $R_{22}$ est H ou un groupe alkyle en $C_1$—$C_2$; $R_{23}$ est H; et $R_1$ est lié à la position *ortho* ou *méta* du noyau par rapport au fragment de sulfonylurée.

3. Un composé selon la revendication 2, dans lequel R est H; $R_1$ est H, Cl, Br, $CH_3$, $OCH_3$, $CF_3$ ou $SCH_3$; $R_8$ est H, $CH_3$, $OCH_3$ ou Cl; $R_{20}$ est H, Cl, $CH_3$ ou $OCH_3$; et $R_{21}$ est H, Cl, $CH_3$, $OCH_3$, $CO_2R_{24}$ ou $SO_2R_{25}$.

4. Un composé selon la revendication 3, dans lequel $R_1$ est H; $R_5$ est H; $R_8$ est H; $R_{20}$ est H; $R_{21}$ est H; et Y est $CH_3$, $OCH_3$ ou $CH_2OCH_3$.

5. Un composé selon la revendication 4, dans lequel J est $J_{27}$ ou $J_{34}$.

6. Un composé selon la revendication 1, dans lequel J est $J_{34}$, $J_{35}$, $J_{36}$ ou $J_{37}$.

7. Un composé selon la revendication 1, dans lequel J est choisi parmi $J_1$—$J_{26}$.

8. Une composition convenant pour lutter contre la croissance de végétation indésirable, qui comprend une quantité efficace d'un composé selon l'une quelconque des revendications 1 à 7 et au moins l'un des ingrédients suivants: agent tensio-actif, diluant solide ou liquide.

9. Un procédé pour lutter contre la croissance de végétation indésirable, qui consiste à appliquer au lieu à protéger une quantité efficace d'un composé selon l'une quelconque des des revendications 1 à 7.

10. Un procédé pour réguler la croissance des plantes, qui consiste à appliquer au lieu où se trouvent ces plantes une quantité efficace mais sensiblement non phytotoxique d'un régulateur de croissance des plantes tel que revendiqué dans l'une quelconque des revendications 1 à 7.

11. Un procédé pour la production d'un composé selon la revendication 1, qui consiste:

(a) à faire réagir un isocyanate de sulfonyle ou un isothiocyanate de sulfonyle de formule

$$J{-}SO_2NCW \qquad (II)$$

dans laquelle J est tel que défini dans la revendication 1 à l'exclusion de $J_6$, $H_{12}$, $J_{22}$ ou $J_{23}$, et si Q est S ou O, J est autre chose que $J_{28}$, $J_{29}$, $J_{31}$ ou $J_{32}$; et W est tel que défini dans la revendication 1, avec une amine hétérocyclique appropriée de formule

$$\begin{array}{c} HN{-}A \\ | \\ R \end{array} \qquad (III)$$

dans laquelle R et A sont tels que définis dans la revendication 1; ou

(b) à faire réagir un sulfonamide de formule

$$J{-}SO_2NH_2 \qquad (IV)$$

dans laquelle J est tel que défini dans la revendication 1, avec un isothiocyanate hétérocyclique de formule

$$SCN{-}A$$

dans laquelle A est tel que défini dans la revendication 1, pour obtenir un composé selon la revendication 1, dans lequel W est S et R est H; ou

(c) à faire réagir un sulfonylcarbamate de formule

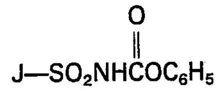

(VI)

dans laquelle J est tel que défini dans la revendication 1, avec ladite amine hétérocyclique (III) définie ci-dessus; ou

(d) à faire réagir ledit sulfonamide (IV) défini ci-dessus avec un carbamate hétérocyclique de formule

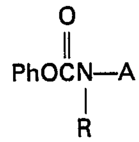

(VII)

dans laquelle A et R sont tels que définis dans la revendication 1; ou

(e) à faire réagir ledit sulfonamide (IV) dans lequel J est tel que défini dans la revendication 1, à l'exclusion de $J_4$, $J_5$, $J_{10}$, $J_{11}$, $J_{16}$, $J_{17}$, $J_{21}$, $J_{24}$ ou $J_{25}$, et $R_8$ est autre chose que $CO_2R_{12}$ et $R_{21}$ est autre chose que $CO_2R_{24}$, avec un méthylcarbamate de formule

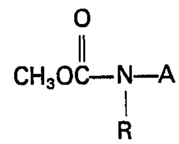

(VIII)

dans laquelle A et R sont tels que définis dans la revendication 1.